# EUROPEAN PATENT APPLICATION

(11) **EP 1 229 024 A1**
(43) Date of publication of application: **07.08.2002**
(21) Application number: 00964758.7
(22) Date of filing: 10.10.2000
(51) Int. Cl.: C07D 239/42, C07D 239/14, C07D 401/04, C07D 403/04, C07D 211/52, C07C 229/54, A61K 31/606, A61P 7/02, A61P 9/00, A61P 19/00, A61P 35/00

(54) **m-SUBSTITUTED BENZOIC ACID DERIVATIVES EXHIBITING INTEGRIN ALPHA-V BETA-3 ANTAGONISM**

(30) Priority: 08.10.1999 JP 28848799
(71) Applicant: Meiji Seika Kaisha, Ltd., Tokyo 104-8002 (JP)
(72) Inventor: AJITO, Keiichi, Pharma. Research Center, Meiji, Yokohama-shi, Kanagawa 222-8567 (JP); ISHIKAWA, Minoru, Pharma. Research Center, Meiji, Yokohama-shi, Kanagawa 222-8567 (JP); KUBOTA, Dai, Pharmaceutical Research Center, Meiji, Yokohama-shi, Kanagawa 222-8567 (JP); MURAKAMI, Shoichi, Pharma. Research Center, Meiji, Yokohama-shi, Kanagawa 222-8567 (JP); YAMAMOTO, Mikio, Pharma Research Center, Meiji, Yokohama-shi, Kanagawa 222-8567 (JP); YAHATA, Naokazu, Pharma Research Center, Meiji, Yokohama-shi, Kanagawa 222-8567 (JP); FUJISHIMA, Kazuyuki, Pharma Research Center, Meiji, Yokohama-shi, Kanagawa 222-8567 (JP); OYAMA, Makoto, Pharma Research Center, Meiji, Yokohama-shi, Kanagawa 222-8567 (JP)
(74) Representative: Hall, Marina
(86) International application number: JP0007031
(87) International publication number: WO0127090

(57) **Abstract**

An object of the present invention is to provide m-substituted benzoic acid derivatives having integrin αᵥβ₃ antagonistic activity. The derivatives according to the present invention are compounds represented by formula (I) or pharmaceutically acceptable salts or solvates thereof, which are useful for the treatment or prevention of cardiovascular diseases, angiogenesis-related diseases, cerebrovascular diseases, cancers and metastasis thereof, immunological diseases, osteopathy and other diseases: 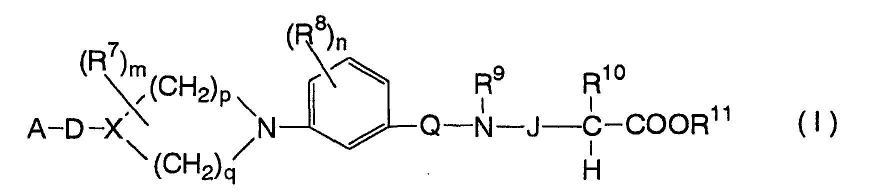 wherein A represents an optionally substituted heterocyclic group containing two nitrogen atoms, a bicylic group or the like; D represents a bond, >NR⁴, >CR⁵R⁶, O, S, or -NR⁴-CR⁵R⁶-; X represents CH or N; R⁷ and R⁸ represent hydroxyl, alkyl or the like; Q represents >C=O or the like; R⁹ represents hydrogen, alkyl or the like; J represents a bond or alkylene; R¹⁰ represents optionally substituted hydroxyl, amino or the like; R¹¹ represents hydrogen, alkyl or the like; m is 0 to 5; n is 0 to 4; and p and q are each 0 to 3.

## Description

### BACKGROUND OF THE INVENTION

### Field of the Invention

The present invention relates to m-substituted benzoic acid derivatives having integrin αᵥβ₃ antagonistic activity and pharmaceuticals comprising the same.

### Description of Related Art

A signal transmission system is very important to organisms from the viewpoints of physiological significance, the regulation of gene expression and the like. It has been clarified that integrins, i.e., glycoprotein receptors which are involved in cell adhesion and penetrate cell membranes, are related, for example, to wound healing and hemostasis, phagocytosis, biophylaxis, and the construction of cytoskeletons and, in addition, as such are signal transfer molecules (Cell, 69, 11, (1992)). For this reason, in recent years, organic chemistry associated with integrins has suddenly become drawn attention from the viewpoint of pharmacology, as well as from the viewpoints of molecular biology and cell biology.

It is being elucidated that, while the conformation of integrins undergoes a dynamic and complicate change, integrins bind to various ligands to transmit signal in both intracellular and extracellular directions (Junichi Takagi et al., The 50th Annual Meeting of the Japan Society for Cell Biology, S5-1, 1997). T. A. Springer of Harvard Medical School has recently predicted that a certain activated integrin has a β-propeller structure and binds to a ligand on the upper face of the β-propeller (Proc. Natl. Acad. Sci. USA, 94, 65, 1997). This hypothesis was also supported by researchers in Japan (Atsushi Irie et al., The 50th Annual Meeting of the Japan Society for Cell Biology, S5-2, 1997), and three-dimensional analysis on a molecular level associated with the activation of integrins as well as binding between integrins and ligands and the like has been initiated in real earnest. T. A. Springer et al. have recently substantiated a hypothesis regarding the β-propeller domain by experimentation, and have suggested that the β-propeller domain in integrin α-subunit has important interaction with integrin β-subunit (Proc. Natl. Acad. Sci. USA, 95, 4870, 1998).

Among others, integrin αᵥβ₃ binds to various extracellular matrixes, that is, ligands deeply involved, for example, in biodynamics or the crisis of diseases, such as vitronectin, fibrinogen, fibronectin, osteopontin, thrombospondin, von Willebrand factors, and collagen, to form complexes. Accordingly, integrin αᵥβ₃ is of special interest as a potential drug target (DN & P, 10, 456, 1997). In fact, αᵥβ₃ is expressed in a large amount in B cells, macrophages, monocytes, smooth muscle, activated endothelial cells and the like. Among others, biological functions of integrin αᵥβ₃ around blood vessels are important, and it has become clear that αᵥβ₃ is deeply involved in the growth, adhesion, and migration of vascular endothelial cells and vascular smooth muscle cells (T. V. Byzova et al., Thromb Haemost, 80, 726 (1998)). Further, αᵥβ₃ is known not to be strongly expressed in endothelial cells in a resting stage, but to be highly activated in the course of growth and infiltration, that is, in vascularization, wound healing, and inflamed sites. Further, the correlation between the frequency of expression of αᵥβ₃ and the increase in infiltration of cancer has been observed in various cancer cells. On the other hand, a group of researchers at Scripps Research Institute in the U.S. have clarified by advanced computer-assisted video imaging microscopy that microvascular expression of αᵥβ₃ is observed during experimental middle cerebral artery occlusion and reperfusion in a baboon as a model (Y. Okada et al., Am. J. Pathol., 149, 37, 1996).

As described above, relationship of cell species, which express integrin αᵥβ₃ in vivo, with αᵥβ₃ activation stage, biophylaxis mechanism and the like has led to an expectation of clinical application of molecules having integrin αᵥβ₃ antagonistic activity in various fields. In fact, compounds having integrin αᵥβ₃ antagonistic activity are intended to be used clinically, and the results of animal tests on compounds having αᵥβ₃ antagonistic activity in a wide range of diseases have been reported (S. S. Srivatsa et al., The 69th Annual Meeting of American Heart Association, 0231, 1996 (DuPont-Merc); J. F. Gourvest et al., The 18th Annual Meeting of The American Society for Bone and Mineral Research, p228, 1996 (Roussel-Hoechst); S. B. Rodan et al., The 18th Annual Meeting of The American Society for Bone and Mineral Research, M430, 1996 (Merck); T. L. Yue et al., The 70th Annual Meeting of American Heart Association, 3733, 1997 (SmithKline Beecham); A. L. Racanelli et al., The 70th Annual Meeting of American Heart Association, 3734, 1997 (DuPont-Merc); M. Friedlander et al., Conference of American IBC, September 11, 1997 (The Scripps Research Institute); W. S. Westlin, Conference of American IBC, February 23, 1998 (Searle); M. W. Lark et al., The 2nd Joint Conference of The American Society for Bone and Mineral Research and International Bone and Mineral Society, T064, 1998 (SmithKline Beecham); R. K. Keenan et al., Bioorg. Med. Chem. Lett., 8, 3171, 1998 (SmithKline Beecham); C. P. Carron et al., Cancer Res., 58, 1930, 1998 (Searle); W. H. Miller et al., Bioorg. Med. Chem. Lett., 9, 1807, 1999 (SmithKline Beecham); and S. A. Mousa et al., The 17th International Congress on Thrombosis and Hemostasis, 228, 1999 (DuPont Pharmaceuticals)).

From the viewpoint of chemical structure, compounds having integrin αᵥβ₃ antagonistic activity can be classified into antibodies, low-molecular peptide and compounds analogous thereto, and low-molecular organic compounds. All the antagonists are structurally related to the sequence of tripeptide RGD (arginine-glycine-aspartic acid) that is considered indispensable for recognition in the attachment of a ligand. Low-molecular peptides having antagonistic activity include disintegrins derived from venom of snakes and, in addition, cyclic peptides. One of them, GpenGRGDSPCA, has been reported to inhibit migration of smooth muscle and to block integrin αᵥβ₃, thereby to actually inhibit neointima formation in rabbits (E.T. Choi et al., J. Vasc. Surg., 19, 125, 1994). Further, RGD-containing cyclic peptide G4120 inhibited neointima formation in hamsters (Circulation, 90, 2203 (1994)). Further, Scripps Research Institute has recently reported that cyclic peptides having αᵥβ₃ antagonistic activity are promising novel therapeutic agents for rheumatic arthritis (C. M. Storgard et al., J. Clin. Invest., 103, 47 (1999)). On the other hand, cyclic peptides containing BTD designed by a β-turn mimic have been proved to strongly bind to αᵥβ₃ receptors (M. Goodman et al., Bioorg. Med. Chem. Lett., 7, 997, 1997).

Several methods are known for designing small molecules through the utilization of the amino acid sequence of interest (RGD being used here) as a clue (Gen Ojima et al., Journal of The Society of Synthetic Organic Chemistry, 52, 413 (1994); Toshio Furuya, Shin-Tanpakushitu Oyo Kogaku, Fujitec Corporation). A peptide mimesis for constructing a new molecule based on the backbone of a peptide chain is generally known in the art. The concept of a new de novo design focused on the chemical structure and spatial configuration of amino acid side chains has been introduced for the first time early in the 1990s (R. Hirschman et al., J. Am. Chem. Soc., 115, 12550 (1993)). An attempt to apply this approach to the design and synthesis of αᵥβ₃ antagonists has already been initiated (K.C. Nicolaou et al., Tetrahedron, 53, 8751, 1997).

Up to now, small molecules having αᵥβ₃ antagonistic activity are disclosed in WO 95/32710 (Merck); WO 96/37492 (Dupont-Merc); WO 97/01540 (SmithKline Beecham); WO 97/08145 (Searle); WO 97/23451 (Merck); WO 97/23480 (Dupont-Merc); WO 97/24119 (SKB); WO 97/26250 (Merck); WO 97/33887 (Dupont-Merc); WO 97/36858 (Searle); WO 97/36859 (Searle); WO 97/36860 (Searle); WO 97/36861 (Searle); WO 97/36862 (Searle); WO 97/24336 (SmithKline Beecham); WO 97/37655 (Merck); WO 98/08840 (Merck); WO 98/18460 (Merck); WO 98/25892 (Lilly); WO 98/30542 (SmithKline Beecham); WO 98/31359 (Merck); WO 98/35949 (Merck); WO 98/43962 (Dupont-Merc); WO 98/46220 (Merck); WO 99/05107 (SmithKline Beecham); WO 99/06049 (SmithKline Beecham); WO 99/11626 (SmithKline Beecham); WO 99/15170 (SmithKline Beecham); WO 99/15178 (SmithKline Beecham); WO 99/15506 (Hoechst Marion Roussel); WO 99/15507 (Hoechst Marion Roussel); WO 99/15508 (SmithKline Beecham); WO 99/30709 (Merck); WO 99/30713 (Merck); WO 99/31061 (Merck); WO 99/31099 (Merck); WO 99/32457 (Hoechst Marion Roussel); WO 99/33798 (Yamanouchi Pharmaceutical Co., Ltd.); WO 99/37621 (Hoechst Marion Roussel); US 5843906 (Searle); US 5852210 (Searle); EP 796855 (Hoechst); EP 820988 (Hoechst); EP 820991 (Hoechst); EP 853084 (Hoechst); EP 928790 (Hoffmann-La Roche Ltd.); EP 928793 (Hoffmann-La Roche Ltd.); GB 2326609 (Merck); GB 2327672 (Merck); R. M. Keenan et al., J. Med. Chem., 40, 2289 (1997); J. W. Corbett et al., Bioorg. Med. Chem. Lett., 7, 1371 (1997); K. C. Nicolaou et al., Bioorg. Med. Chem., 6, 1185 (1998); R. M. Keenan, et al., Bioorg. Med. Chem. Lett., 8, 3165 (1998); A. R. Rockwell et al. Bioorg. Med. Chem. Lett., 9, 937 (1999); R. M. Keenan et al., Bioorg. Med. Chem. Lett., 9, 1801 (1999); and S. A. Mousa et al., J. Cardiovasc. Pharmacol., 33, 641 (1999).

However, small molecules having potent αᵥβ₃ antagonistic activity, which have been known up to now, are not always usable in a wide amount range due to their limited solubility. When the administration of these small molecules by oral administration or by application of a liniment to the skin is contemplated, the antagonists are not particularly required to be soluble in water. When the administration of these small molecules, for example, through intraveneous injection, intraveneous drop infusion, or instillation is contemplated, however, the antagonists are preferably soluble in water. When the use of antagonists, which are expected to be used as therapeutic agents for diseases in acute phase, for example, acute myocardial infarction, restenosis after PTCA, unstable angina, cerebral infarction, peripheral infarction diseases, and diabetic retinopathy, is contemplated, highly water-soluble antagonists are desired.

Further, for example, chimeric antibody 7E3 is known to have αᵥβ₃ antagonistic activity and α_{IIb}β₃ antagonistic activity which are substantially identical to each other in activity level (S. H. Tam et al., Circulation, 98, 1085 (1998)). However, it is not easy to chemically modify or chemically convert this chimeric antibody to control the selectivity as desired. When small molecules having αᵥβ₃ antagonistic activity and α_{IIb}β₃ antagonistic activity are used as drugs, the optimal balance of the antagonistic activity against integrins varies depending upon diseases to which the drugs are to be applied. Specifically, in some cases, the optimal ratio of αᵥβ₃ antagonistic activity to α_{IIb}β₃ antagonistic activity is substantially 1 : 1, and, in other cases, the αᵥβ₃ antagonistic activity is preferably higher than the α_{IIb}β₃ antagonistic activity or vice versa. Therefore, when the selectivity to various integrins can be controlled as desired by chemically modifying or chemically converting an identical pharmacophore, this is very beneficial to the creation of drugs. Up to now, the balance between integrin αᵥβ₃ antagonistic activity and integrin α_{IIb}β₃ antagonistic activity has been discussed from the viewpoint of three-dimensional structure of molecules only in G. D. Hartman et al., Bioorg. Med. Chem. Lett., 9, 863 (1999), and M. A. Dechantsreiter et al., J. Med. Chem., 42, 3033 (1999). However, systematic methodology on the balance between integrin αᵥβ₃ antagonistic activity and integrin α_{IIb}β₃ antagonistic activity are not known in the art.

Meanwhile, a method for introducing a hetero ring, such as a piperazine ring or a piperidine ring, directly into the para- or ortho-position of benzoic acid through a hetero-atom is well known (WO 99/38849, Dai Kubota et al., The 19th Medicinal Chemistry Symposium/The 8th Annual Meeting of Medicinal Chemistry Section of The Pharmaceutical Society of Japan, 1P-01, 1999, WO99/52872, and Minoru Ishikawa et al., The 218th Meeting of Medicinal Chemistry Section of American Chemical Society, MEDI63, 1999). Conventional methods for introducing a hetero ring, such as a piperidine ring having a hydroxyl group at the 4-position, directly into the meta-position of benzoic acid through a nitrogen atom, however, involve problems. Specifically, in a classical method for introduction of the hetero ring through 1,5-dichloropentan-3-one (G. R. Owen et al., J. Chem. Soc. (C), 2401 (1970) and C. B. Reese et al., J. Chem. Soc. Perkin Trans., I, 2881 (1988)), a methodology for synthesizing an analogue of 1,5-dichloropentan-3-one is not generalized. Further, in a coupling reaction using advanced palladium, (J. P. Wolfe et al., Tetrahedron Lett., 38,6359 (1997) and Y. Guari et al., Tetrahedron Lett., 40,3789 (1999)), the use of palladium and phosphine ligand poses a problem of cost. In addition, in this literature, discussion has been not fully made on various purposes of this reaction wherein free hydroxyl groups are present.

### SUMMARY OF THE INVENTION

The present inventors have found that novel compounds have potent integrin αᵥβ₃ antagonistic activity, have improved αᵥβ₃ antagonistic activity in relationship to α_{IIb}β₃ antagonistic activity, and have improved solubility in water.

An object of the present invention is to provide novel compounds which have integrin αᵥβ₃ antagonistic activity, have improved αᵥβ₃ antagonistic activity in relationship to α_{IIb}β₃ antagonistic activity, and, at the same time, have excellent solubility in water.

According to the present invention, there is provided a compound represented by formula (I) or a pharmaceutically acceptable salt or solvate thereof: 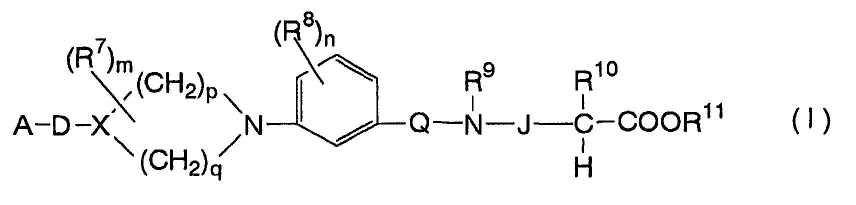 wherein
A represents a saturated or unsaturated five- to seven-membered heterocyclic group containing two nitrogen atoms, which is optionally condensed with another saturated or unsaturated five- to seven-membered carbocyclic ring or heterocyclic ring to form a bicyclic group, wherein the heterocyclic group and the bicyclic group are optionally substituted by C₁₋₆ alkyl optionally substituted by C₁₋₆ alkyl, C₁₋₆ alkoxy, C₁₋₆ alkoxycarbonyl, aralkyl, amino, or hydroxyl; a halogen atom; or amino optionally substituted by C₁₋₆ alkyl, C₁₋₆ alkoxy, C₁₋₆ alkoxycarbonyl, or aralkyl,
or a group represented by formula 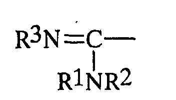 wherein R¹, R², and R³, which may be the same or different, represent a hydrogen atom, C₁₋₆ alkyl, C₂₋₆ alkenyl, or aralkyl, wherein the C₁₋₆ alkyl, C₂₋₆ alkenyl, and aralkyl groups are optionally substituted by C₁₋₆ alkyl, C₁₋₆ alkoxy, C₁₋₆ alkoxycarbonyl, aralkyl, amino, or hydroxyl;

D represents a bond; >NR⁴ wherein R⁴ represents a hydrogen atom or C₁₋₆ alkyl and this C₁₋₆ alkyl group is optionally substituted by C₁₋₆ alkyl, C₁₋₆ alkoxy, C₁₋₆ alkoxycarbonyl, aralkyl, amino, or hydroxyl; >CR⁵R⁶ wherein R⁵ and R⁶ each independently represent a hydrogen atom or C₁₋₆ alkyl and this C₁₋₆ alkyl group is optionally substituted by C₁₋₆ alkyl, C₁₋₆ alkoxy, C₁₋₆ alkoxycarbonyl, aralkyl, amino, or hydroxyl; -O-; -S-; or -NR⁴-CR⁵R⁶- wherein R⁴, R⁵, and R⁶ are as defined above;
X represents CH or N;
R⁷ represents C₁₋₆ alkyl, C₁₋₆ alkoxy, a halogen atom, amino, nitro, cyano, hydroxyl, thiol, or an oxygen atom, wherein the C₁₋₆ alkyl and C₁₋₆ alkoxy groups represented by R⁷ are optionally substituted by C₁₋₆ alkyl, C₁₋₆ alkoxy, C₁₋₆ alkoxycarbonyl, aralkyl, amino, hydroxyl, or a halogen atom, the amino group represented by R⁷ is optionally substituted by one or two C₁₋₆ alkyl groups, and the thiol group represented by R⁷ is optionally substituted by C₁₋₄ alkyl or phenyl;
R⁸ represents C₁₋₆ alkyl, C₁₋₆ alkoxy, a halogen atom, amino, nitro, cyano, hydroxyl, or thiol, wherein the C₁₋₆ alkyl and C₁₋₆ alkoxy groups represented by R⁸ are optionally substituted by C₁₋₆ alkyl, C₁₋₆ alkoxy, C₁₋₆ alkoxycarbonyl, aralkyl, amino, hydroxyl, or a halogen atom, the amino group represented by R⁸ is optionally substituted by one or two C₁₋₆ alkyl groups, and the thiol group represented by R⁸ is optionally substituted by C₁₋₄ alkyl or phenyl;
Q represents >C=O, >CHR¹³, or >CHOR¹³ wherein R¹³ represents a hydrogen atom or C₁₋₆ alkyl;
R⁹ represents a hydrogen atom, C₁₋₆ alkyl, C₂₋₆ alkenyl, or aralkyl and the C₁₋₆ alkyl, C₂₋₆ alkenyl, and aralkyl groups are optionally substituted by C₁₋₆ alkyl, C₁₋₆ alkoxy, C₁₋₆ alkoxycarbonyl, aralkyl, amino, or hydroxyl;
J represents a bond or an alkylene chain having 1 to 3 carbon atoms, wherein one or more hydrogen atoms on the alkylene chain are optionally substituted by the same or different substituent selected from C₁₋₆ alkyl, C₂₋₆ alkenyl, C₂₋₆ alkynyl, aralkyl, hydroxyl, or amino, the C₁₋₆ alkyl, C₂₋₆ alkenyl, C₂₋₆ alkynyl, and aralkyl groups are optionally substituted by a halogen atom, C₁₋₆ alkoxy, amino, or hydroxyl, and the hydroxyl and amino groups are optionally substituted by carboxyl; sulfonyl; C₁₋₆ alkyl; C₁₋₆ alkylcarbonyl; C₁₋₆ alkoxycarbonyl; C₁₋₆ alkylsulfonyl; -C(=O)-O-(CH₂)u-R¹⁴ wherein u is an integer of 0 to 4, R¹⁴ represents a saturated or unsaturated five- to seven-membered carbocyclic or heterocyclic group, and the carbocyclic group and the heterocyclic group are optionally substituted by C₁₋₆ alkyl, C₁₋₆ alkoxy, phenyl optionally condensed with the carbocyclic group or the heterocyclic group, carboxyl, hydroxyl, nitro, amino, C₁₋₆ alkylamino, or a halogen atom; -C(=O)-R¹⁴ wherein R¹⁴ is as defined above; or -S(=O)₂-(CH₂)v-R¹⁴ wherein v is an integer of 0 to 4 and R¹⁴ is as defined above;
R¹⁰ represents a hydrogen atom, hydroxyl, C₁₋₆ alkyl, C₂₋₆ alkenyl, C₂₋₆ alkynyl, aralkyl, or amino, the C₁₋₆ alkyl, C₂₋₆ alkenyl, C₂₋₆ alkynyl, and aralkyl groups are optionally substituted by a halogen atom, C₁₋₆ alkoxy, amino, or hydroxyl and and the hydroxyl and amino groups are optionally substituted by carboxyl; sulfonyl; C₁₋₆ alkyl; C₁₋₆ alkylcarbonyl; C₁₋₆ alkoxycarbonyl; C₁₋₆ alkylsulfonyl; -C(=O)-O-(CH₂)u-R¹⁴ wherein u is an integer of 0 to 4 and R¹⁴ represents a saturated or unsaturated five- to seven-membered carbocyclic or heterocyclic group, and the carbocyclic group and the heterocyclic group are optionally substituted by C₁₋₆ alkyl, C₁₋₆ alkoxy, phenyl optionally condensed with the carbocyclic group or the heterocyclic group, carboxyl, hydroxyl, nitro, amino, C₁₋₆ alkylamino, or a halogen atom; -C(=O)-R¹⁴ wherein R¹⁴ is as defined above; or -S(=O)₂-(CH₂)v-R¹⁴ wherein v is an integer of 0 to 4 and R¹⁴ is as defined above;
R¹¹ represents a hydrogen atom, aralkyl, or C₁₋₆ alkyl and this C₁₋₆ alkyl group is optionally substituted by C₁₋₆ alkyl, C₁₋₆ alkoxy, C₁₋₆ alkoxycarbonyl, aralkyl, amino, or hydroxyl;
m is an integer of 0 to 5;
n is an integer of 0 to 4;
p is an integer of 0 to 3; and
q is an integer of 0 to 3.

The compounds according to the present invention are useful for the treatment or prevention of integrin αᵥβ₃-mediated diseases, for example, cardiovascular diseases, angiogenesis-related diseases, cerebrovascular diseases, cancers and metastasis thereof, immunological diseases, and osteopathy.

The present inventors have found a production process which can produce a 3-(piperidin-1-yl)benzoic acid derivative, for example, ethyl 3-{3,4-(dihydroxy)piperidin-1-yl}benzoate, as an intermediate of the compounds according to the present invention, at low cost in a simple manner by efficiently combining reductive alkylation with intramolecular alkylation of naturally occurring saccharides or monosaccharides, which are inexpensive, especially 2-deoxy-D-ribosse.

Accordingly, an object of the present invention is to provide a production process which can produce an m-substituted benzoic acid derivative useful for the production of the compounds represented by formula (I), particularly a 3-(4-hydroxypiperidin-1-yl)benzoic acid derivative, at low cost in a simple manner.

According to the present invention, there is provided a process for producing a compound as an intermediate represented by formula (XX) 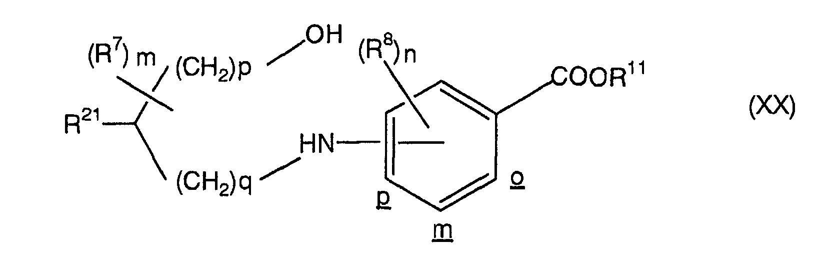 wherein R²¹ represents hydroxyl, azide, or optionally protected amino; R⁷, R⁸, R¹¹, m, n, p, and q are as defined in formula (I), provided that q is not 0 (zero) and the nitrogen atom is attached to the ortho-, meta-, or para-position of the phenyl group, said process comprising the step of reacting a compound represented by formula (XIX) 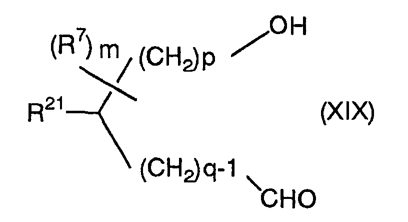 wherein R²¹ is as defined in formula (XX); and R⁷, m, p, and q are as defined in formula (I), provided that q is not 0 (zero),
with a compound represented by formula (XV) 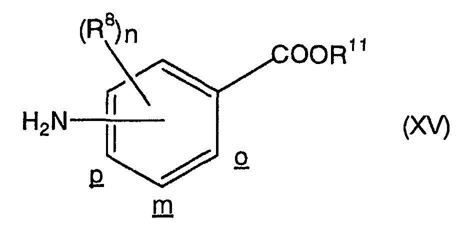 wherein R⁸, R¹¹, and n are as defined in formula (I); and the nitrogen atom is attached to the ortho-, meta-, or para-position of the phenyl group.

According to the present invention, there is provided another process for producing a compound as an intermediate represented by formula (XXII) 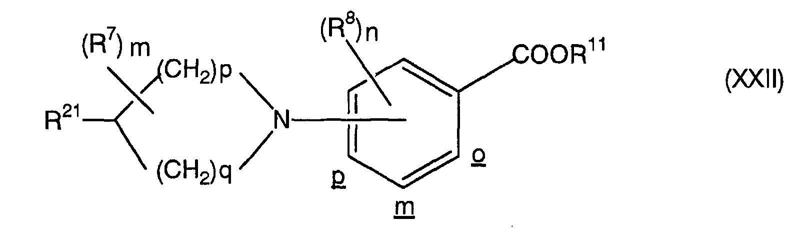 wherein R⁷, R⁸, R¹¹, m, n, p, and q are as defined in the definition formula (I), provided that q is not 0 (zero); R²¹ is as defined in formula (XX); and the nitrogen atom is attached to the ortho-, meta-, or para-position of the phenyl group, said process comprising the step of:
cyclizing a compound represented by formula (XXI) 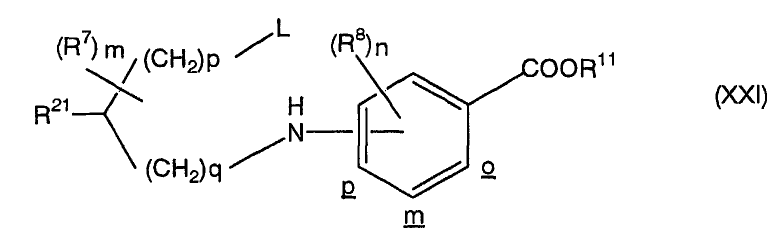 wherein R⁷, R⁸, R¹¹, m, n, p, and q are as defined in formula (I), provided that q is not 0 (zero); R²¹ is as defined in formula (XX); L represents a leaving group; and the nitrogen atom is attached to the ortho-, meta-, or para-position of the phenyl group by an intramolecular ring-closing reaction.

A further object of the present invention is to provide an intermediate useful for the production of the compounds represented by formula (I).

According to the present invention, there is provided an intermediate represented by formula (XXIII): 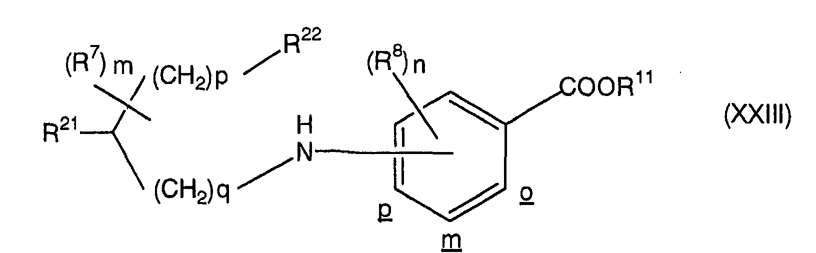 wherein R⁷, R⁸, R¹¹, m, n, p, and q are as defined in formula (I), provided that q is not 0 (zero); R²¹ is as defined in formula (XX); R²² represents hydroxyl or a leaving group; and the nitrogen atom is attached to the ortho-, meta-, or para-position, preferably meta-position, of the phenyl group.

Further, according to the present invention, there is provided an intermediate represented by formula (XXII): 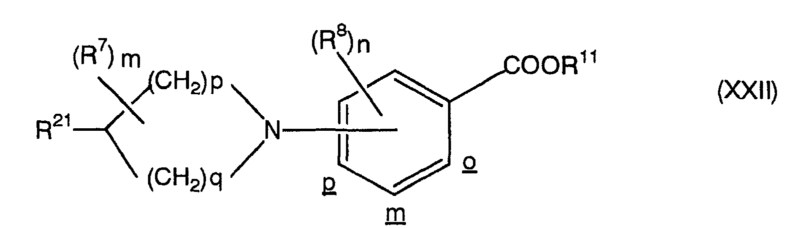 wherein R⁷, R⁸, R¹¹, m, n, p, and q are as defined in formula (I), provided that q is not 0 (zero); R²¹ is as defined in formula (XX); and the nitrogen atom is attached to the ortho-, meta-, or para-position, preferably meta-position, of the phenyl group.

### DETAILED DESCRIPTION OF THE INVENTION

### Compound

The terms "C₁₋₆ alkyl" and "C₁₋₆ alkoxy" as used herein as a group or a part of a group respectively mean straight chain, branched chain, or cyclic alkyl and alkoxy having 1 to 6, preferably 1 to 4 carbon atoms.

The terms "C₂₋₆ alkenyl" and "C₂₋₆ alkynyl" as used herein as a group or a part of a group respectively mean straight chain, branched chain, or cyclic alkenyl and alkynyl having 2 to 6, preferably 2 to 4 carbon atoms.

Examples of C₁₋₆ alkyl include methyl, ethyl, n-propyl, isopropyl, cyclopropyl, cyclopropylmethyl, n-butyl, i-butyl, s-butyl, t-butyl, n-pentyl, cyclopentyl, n-hexyl, and cyclohexyl.

Examples of C₁₋₆ alkoxy include methoxy, ethoxy, n-propoxy, i-propoxy, n-butoxy, i-butoxy, s-butoxy, and t-butoxy.

Examples of C₂₋₆ alkenyl include allyl.

Examples of C₂₋₆ alkynyl include 2-propynyl and ethynyl.

Examples of "saturated or unsaturated five- to seven-membered carbocyclic groups" include phenyl.

The term "saturated or unsaturated five- to seven-membered heterocyclic ring" as used herein means a five-to seven-membered heterocyclic ring containing at least one hetero-atom selected from oxygen, nitrogen, and sulfur atoms, preferably a five- to seven-membered heterocyclic ring containing one or two hetro-atoms, more preferably a five- or six-membered heterocyclic ring containing one or two hetro-atoms. The term "hetero-atom" used herein means an oxygen, nitrogen, or sulfur atom. Examples of saturated or unsaturated five-to seven-membered heterocyclic groups include pyridyl, pyrimidyl, 1,4,5,6-tetrahydropyrimidyl, imidazolyl, tetrahydro-[1,3]diazepinyl, imidazolidinyl, thiophenyl, and morpholyl.

The saturated or unsaturated heterocyclic group may be condensed with other saturated or unsaturated heterocyclic ring to form a bicyclic ring. Such condensed cyclic groups include benzimidazolyl, naphthyl, and azabenzimidazolyl, for example, imidazo[4,5-b]pyridyl.

The term "aralkyl" as used herein as a group or a part of a group means C₁₋₆ alkyl, preferably C₁₋₄ alkyl, substituted by a saturated or unsaturated five- to seven-membered carbocyclic group or heterocyclic group. Examples of aralkyl include benzyl and phenethyl.

The term "halogen atom" means a fluorine, chlorine, bromine, or iodine atom.

D preferably represents a bond, >NH, or -NH-CH₂-.

When D represents >NR⁴, X preferably represents CH.

When D represents -CR⁵R⁶- or -NR⁴-CR⁵R⁶-, X preferably represents CH.

When D represents a bond, X preferably represents N.

The "saturated or unsaturated five- to seven-membered heterocyclic group containing two nitrogen atoms" represented by A is preferably a saturated or unsaturated five- or six-membered heterocyclic group containing two nitrogen atoms.

The "bicyclic group" represented by A is preferably a nine- or ten-membered heterocyclic group, more preferably a nine- or ten-membered heterocyclic group containing two or three nitrogen atoms.

R² preferably represents a hydrogen atom.

A preferably represents a group of formula 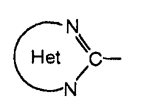 wherein
Het represents a saturated or unsaturated five- to seven-membered heterocyclic group containing two nitrogen atoms, which is optionally condensed with another saturated or unsaturated five- to seven-membered carbocyclic ring or heterocyclic ring to form a bicyclic group, wherein the heterocyclic group and the bicyclic group are optionally substituted by C₁₋₆ alkyl optionally substituted by C₁₋₆ alkyl, C₁₋₆ alkoxy, C₁₋₆ alkoxycarbonyl, aralkyl, amino, or hydroxyl; a halogen atom; or amino optionally substituted by C₁₋₆ alkyl, C₁₋₆ alkoxy, C₁₋₆ alkoxycarbonyl, or aralkyl.

More preferably, A represents a group of formula: 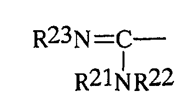 wherein
R²¹, R²², and R²³, which may be the same or different, represent a hydrogen atom, C₁₋₆ alkyl, C₂₋₆ alkenyl, or aralkyl and the C₁₋₆ alkyl, C₂₋₆ alkenyl, and aralkyl groups are optionally substituted by C₁₋₆ alkyl, C₁₋₆ alkoxy, C₁₋₆ alkoxycarbonyl, aralkyl, amino, or hydroxyl, or
R²¹ and R²³ may together form
group -(CH₂)₄-,
group -(CH₂)₃-,
group -CHR24CH2CH2- wherein R²⁴ represents C₁₋₆ alkyl, a halogen atom, or amino the amino group is optionally substituted by C₁₋₆ alkyl, C₁₋₆ alkoxy, C₁₋₆ alkoxycarbonyl, or aralkyl,
group -CH₂CHR²⁴CH₂- wherein R²⁴ is as defined above,
group -CH₂CH₂-,
group -CHR²⁴CH₂- wherein R²⁴ is as defined above,
group -CR²⁵=CR²⁶- wherein R²⁵ and R²⁶, which may be the same or different, represent a hydrogen atom or C₁₋₆ alkyl, or R²⁵ and R²⁶ may together form -CH=CH-CH=CH-, -CR²⁴=CH-CH=CH- wherein R²⁴ is as defined above, -CH=CR²⁴-CH=CH- wherein R²⁴ is as defined above, -N=CH-CH=CH-, or -CH=N-CH=CH-, or
R²¹ and R²³ may together form
=CH-CH=CH-,
-CHR²⁴CH₂CH₂- wherein R²⁴ is as defined above,
-CH₂CHR²⁴CH₂- wherein R²⁴ is as defined above,
=CH-CH=N-, or
=CH-N=CH-, and

R²² may represent a single bond between R²¹ and the nitrogen atom attached to R²¹.

R²² preferably represents a hydrogen atom.

In the compound represented by formula (I), one or more hydrogen atoms in the following portion may be substituted by R⁷.

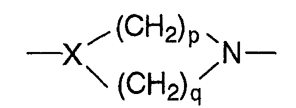

When m is zero (0), R⁷ is absent. When m is 1, one hydrogen atom in the above portion is substituted by R⁷. When m is 2 or more, two or more hydrogen atoms in the above portion are substituted by R⁷. In this case, the substituents may be the same or different. When R⁷ represents an oxygen atom, the bond between the R⁷ and the above portion is a double bond. m is preferably an integer of 0 to 2.

In the compound represented by formula (I), one or more hydrogen atoms in the phenylene portion may be substituted by R⁸.

When n is zero (0), R⁸ is absent. When n is 1, one hydrogen atom in the phenylene portion is substituted by R⁸. When n is 2 or more, two or more hydrogen atoms in the phenylene portion are substituted by R⁸. In this case, the substituents may be the same or different. n is preferably an integer of 0 to 2.

Q preferably represents >C=O or >CH₂.

R⁹ preferably represents a hydrogen atom, C₁₋₆ alkyl (preferably, methyl, propyl, cyclopropylmethyl), or aralkyl (preferably benzyl or phenethyl).

J preferably represents a methylene chain or an ethylene chain, more preferably a methylene chain.

On or more hydrogen atoms on the alkylene chain represented by J may be substituted. The substituent is preferably C₂₋₆ alkynyl or optionally substituted amino, more preferably C₂₋₆ alkynyl.

R¹⁰ preferably represents a hydrogen atom, C₂₋₆ alkynyl, hydroxyl, optionally substituted hydroxyl, or optionally substituted amino, more preferably, a hydrogen atom or optionally substituted amino.

Hydrogen atoms in the amino group represented by R¹⁰ may be substituted by two substituents which may be the same or different.

Preferred examples of -C(=O)-O-(CH₂)u-R¹⁴, which is a substituent for the amino group represented by R¹⁰, include groups wherein u is an integer of 0 to 3 (more preferably, 0 or 1) and R¹⁴ represents a five- to seven-membered carbocyclic group (more preferably, phenyl).

Preferred examples of -S(=O)₂-(CH₂)v-R¹⁴, which is a substituent for the amino group represented by R¹⁰, include groups wherein v is an integer of 0 to 3 (more preferably, 0 or 1) and R¹⁴ represents a five- to seven-membered carbocyclic group (more preferably, phenyl) or a five- to seven-membered heterocyclic group (more preferably, a five- or six-membered heterocyclic group containing one or two hetero-atoms, specifically morpholyl).

One or more hydrogen atoms in the carbocyclic group and the heterocyclic group represented by R¹⁴ are preferably optionally substituted by C₁₋₆ alkyl (more preferably methyl), C₁₋₆ alkoxy (more preferably methoxy), carboxyl, hydroxyl, nitro, amino, or a halogen atom.

The substituent of the amino group represented by R¹⁰ is preferably C₁₋₆ alkyl; C₁₋₆ alkylcarbonyl; C₁₋₆ alkoxycarbonyl; C₁₋₆ alkylsulfonyl; benzoyl or benzyloxycarbonyl wherein the phenyl portion of benzoyl and benzyloxycarbonyl is optionally substituted by C₁₋₆ alkyl, C₁₋₆ alkoxy, carboxyl, hydroxyl, nitro, amino, or a halogen atom; -C(=O)-O-(CH₂)u-R¹⁴ wherein u is an integer of 0 to 4 and R¹⁴ represents phenyl optionally substituted by C₁₋₆ alkyl, C₁₋₆ alkoxy, carboxyl, hydroxyl, nitro, amino, or a halogen atom, or a five- or six-membered heterocyclic group containing one or two hetero-atoms (preferably morpholyl); or -S(=O)₂-(CH₂)v-R¹⁴ wherein v is an integer of 0 to 4 and R¹⁴ represents phenyl optionally substituted by C₁₋₆ alkyl, C₁₋₆ alkoxy, carboxyl, hydroxyl, nitro, amino, or a halogen atom, or a five- or six-membered heterocyclic group containing one or two hetero-atoms (preferably morpholyl).

The C₁₋₆ alkyl group represented by R¹¹ is preferably methyl, ethyl, t-butyl, or diphenylmethyl.

p is preferably an integer of 0 to 2.

q is preferably 2 or 3.

The sum of p and q is preferably 2 to 4.

Preferred compounds represented by formula (I) are those wherein

A represents a group of formula 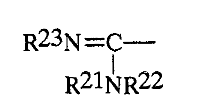 wherein
R²¹, R²², and R²³ are as defined above;
D represents a bond, >NH, or -NH-CH₂-;
X represents CH or N;
Q represents >C=O or >CH₂;
R⁹ represents a hydrogen atom, C₁₋₆ alkyl or aralkyl and the C₁₋₆ alkyl and aralkyl groups are optionally substituted by a halogen atom, C₁₋₆ alkoxy, amino, or hydroxyl;
J represents a methylene chain;
R¹⁰ represents a hydrogen atom, hydroxyl, or amino, the hydroxyl group is optionally substituted by C₁₋₆ alkyl, and the amino group is optionally substituted by C₁₋₆ alkyl; C₁₋₆ alkylcarbonyl; C₁₋₆ alkoxycarbonyl; C₁₋₆ alkylsulfonyl; benzoyl or benzyloxycarbonyl wherein the phenyl portion of benzoyl and benzyloxycarbonyl is optionally substituted by C₁₋₆ alkyl, C₁₋₆ alkoxy, carboxyl, hydroxyl, nitro, amino, or a halogen atom; -C(=O)-O-(CH₂)u-R¹⁴ wherein u is an integer of 0 to 4 and R¹⁴ represents phenyl optionally substituted by C₁₋₆ alkyl, C₁₋₆ ₆ alkoxy, carboxyl, hydroxyl, nitro, amino, or a halogen atom, or a five- or six-membered heterocyclic group containing one or two hetero-atoms (preferably morpholyl); or -S(=O)₂-(CH₂)v-R¹⁴ wherein v is an integer of 0 to 4 and R¹⁴ represents phenyl optionally substituted by C₁₋₆ alkyl, C₁₋₆ alkoxy, carboxyl, hydroxyl, nitro, amino, or a halogen atom, or a five- or six-membered heterocyclic group containing one or two hetero-atoms (preferably morpholyl);
R¹¹ represents a hydrogen atom or C₁₋₆ alkyl;
m and n are each an integer of 0 to 2;
p is 0 to 2; and
q is 2 or 3.

More preferred compounds represented by formula (I) are the following compounds:
1. t-butyl (2S)-benzenesulfonylamino-3-[3-{4-(pyrimidin-2-yl)piperazin-1-yl}benzoylamino]propionate;
2. (2S)-benzenesulfonylamino-3-[3-{4-(pyrimidin-2-yl)piperazin-1-yl}benzoylamino]propionic acid;
3. (2S)-benzenesulfonylamino-3-[3-{4-(1,4,5,6-tetrahydropyrimidin-2-yl)piperazin-1-yl}benzoylamino]-propionic acid;
4. t-butyl (2S)-benzenesulfonylamino-3-[3-{4-(pyrimidin-2-ylamino)piperidin-1-yl}benzoylamino]-propionate;
5. (2S)-benzenesulfonylamino-3-[3-{4-(pyrimidin-2-ylamino)piperidin-1-yl}benzoylamino]propionic acid;
6. (2S)-benzenesulfonylamino-3-[3-{4-(1,4,5,6-tetrahydropyrimidin-2-ylamino)piperidin-1-yl}benzoylamino]propionic acid;
7. t-butyl (2S)-benzenesulfonylamino-3-[4-fluoro-3-{4-(pyrimidin-2-ylamino)piperidin-1-yl}benzoylamino]-propionate;
8. (2S)-benzenesulfonylamino-3-[4-fluoro-3-{4-(pyrimidin-2-ylamino)piperidin-1-yl}benzoylamino]-propionic acid;
9. (2S)-benzenesulfonylamino-3-[4-fluoro-3-{4-(1,4,5,6-tetrahydropyrimidin-2-ylamino)piperidin-1-yl}benzoylamino]propionic acid;
10. t-butyl (2S)-benzenesulfonylamino-3-[3-{(3R)-hydroxy-(4R)-(pyrimidin-2-ylamino)piperidin-1-yl}benzoylamino]propionate;
11. (2S)-benzenesulfonylamino-3-[3-{(3R)-hydroxy(4R)-(pyrimidin-2-ylamino)piperidin-1-yl}benzoylamino]-propionic acid;
12. (2S)-benzenesulfonylamino-3-[3-{(3R)-hydroxy(4R)-(1,4,5,6-tetrahydropyrimidin-2-ylamino)piperidin-1-yl}benzoylamino]propionic acid;
13. t-butyl (2S)-benzenesulfonylamino-3-[{(3R)-methoxy-(4R)-(pyrimidin-2-ylamino)}piperidin-1-yl}benzoylamino]propionate;
14. (2S)-benzenesulfonylamino-3-[{(3R)-methoxy-(4R)-(pyrimidin-2-ylamino)piperidin-1-yl}benzoylamino]-propionic acid;
15. (2S)-benzenesulfonylamino-3-[3-{(3R)-methoxy-(4R)-(1,4,5,6-tetrahydropyrimidin-2-ylamino)piperidin-1-yl}benzoylamino]propionic acid;
16. t-butyl (2S)-benzenesulfonylamino-3-[5-fluoro-3-{4-(pyrimidin-2-ylamino)piperidin-1-yl}benzoylamino]-propionate;
17. (2S)-benzenesulfonylamino-3-[5-fluoro-3-{4-(pyrimidin-2-ylamino)piperidin-1-yl}benzoylamino]-propionic acid;
18. (2S)-benzenesulfonylamino-3-[5-fluoro-3-{4-(1,4,5,6-tetrahydropyrimidin-2-ylamino)piperidin-1-yl}benzoylamino]propionic acid;
19. t-butyl (2S)-benzenesulfonylamino-3-[6-fluoro-3-{4-(pyrimidin-2-ylamino)piperidin-1-yl}benzoylamino]-propionate;
20. (2S)-benzenesulfonylamino-3-[6-fluoro-3-{4-(pyrimidin-2-ylamino)piperidin-1-yl}benzoylamino]-propionic acid;
21. (2S)-benzenesulfonylamino-3-[6-fluoro-3-{4-(1,4,5,6-tetrahydropyrimidin-2-ylamino)piperidin-1-yl}benzoylamino]propionic acid;
22. t-butyl (2S)-benzenesulfonylamino-3-[2-fluoro-3-{4-(pyrimidin-2-ylamino)piperidin-1-yl}benzoylamino]-propionate;
23. (2S)-benzenesulfonylamino-3-[2-fluoro-3-{4-(pyrimidin-2-ylamino)piperidin-1-yl}benzoylamino]-propionic acid;
24. (2S)-benzenesulfonylamino-3-[2-fluoro-3-{4-(1,4,5,6-tetrahydropyrimidin-2-ylamino)piperidin-1-yl}benzoylamino]propionic acid;
25. t-butyl (2S)-benzenesulfonylamino-3-[3-{4-(pyrimidin-2-ylamino)piperidin-1-yl}-5-(trifluoromethyl)benzoylamino]propionate;
26. (2S)-benzenesulfonylamino-3-[3-{4-(pyrimidin-2-ylamino)piperidin-1-yl}-5-(trifluoromethyl)benzoylamino]propionic acid;
27. (2S)-benzenesulfonylamino-3-[3-{4-(1,4,5,6-tetrahydropyrimidin-2-ylamino)piperidin-1-yl}-5-(trifluoromethyl)benzoylamino]propionic acid;
28. t-butyl (2S)-(benzyloxycarbonyl)amino-3-[3-{4-(pyrimidin-2-ylamino)piperidin-1-yl}benzoylamino]-propionate;
29. t-butyl (2S)-amino-3-[3-{4-(pyrimidin-2-ylamino)piperidin-1-yl}benzoylamino]propionate;
30. t-butyl (2S)-acetamido-3-[3-{4-(pyrimidin-2-ylamino)piperidin-1-yl}benzoylamino]propionate;
31. (2S)-acetamido-3-[3-{4-(pyrimidin-2-ylamino)-piperidin-1-yl}benzoylamino]propionic acid;
32. (2S)-acetamido-3-[3-{4-(1,4,5,6-tetrahydropyrimidin-2-ylamino)piperidin-1-yl}benzoylamino]-propionic acid;
33. t-butyl (2S)-{2-(morpholin-4-yl-acetyl)amino}-3-[3-{4-(pyrimidin-2-ylamino)piperidin-1-yl}benzoylamino]propionate;
34. (2S)-{2-(morpholin-4-yl-acetyl)amino}-3-[3-{4-(pyrimidin-2-ylamino)piperidin-1-yl}benzoylamino]-propionic acid;
35. (2S)-{2-(morpholin-4-yl-acetyl)amino}-3-[3-{4-(1,4,5,6-tetrahydropyrimidin-2-ylamino)piperidin-1-yl}benzoylamino]propionic acid;
36. t-butyl 3-[3-{4-(pyrimidin-2-ylamino)piperidin-1-yl}benzoylamino]-(2S)-{(2,4,6-trimethylbenzenesulfonyl)amino}propionate;
37. 3-[3-{4-(pyrimidin-2-ylamino)piperidin-1-yl}benzoylamino]-(2S)-{(2,4,6-trimethylbenzenesulfonyl)-amino}propionic acid;
38. 3-[3-{4-(1,4,5,6-tetrahydropyrimidin-2-ylamino)piperidin-1-yl}benzoylamino]-(2S)-{(2,4,6-trimethylbenzenesulfonyl)amino}propionic acid;
39. t-butyl (2S)-{(4-methoxybenzenesulfonyl)amino}-3-[3-{4-(pyrimidin-2-ylamino)piperidin-1-yl}benzoylamino]propionate;
40. (2S)-{(4-methoxybenzenesulfonyl)amino}-3-[3-{4-(pyrimidin-2-ylamino)piperidin-1-yl}benzoylamino]-propionic acid;
41. (2S)-{(4-methoxybenzenesulfonyl)amino}-3-[3-{4-(1,4,5,6-tetrahydropyrimidin-2-ylamino)piperidin-1-yl}benzoylamino]propionic acid;
42. (2S)-{(4-hydroxybenzenesulfonyl)amino}-3-[3-{4-(pyrimidin-2-ylamino)piperidin-1-yl}benzoylamino]-propionic acid;
43. (2S)-{(4-hydroxybenzenesulfonyl)amino}-3-[3-{4-(1,4,5,6-tetrahydropyrimidin-2-ylamino)piperidin-1-yl}benzoylamino]propionic acid;
44. t-butyl (2S)-benzenesulfonylamino-3-[3-{(3S)-(pyrimidin-2-ylamino)piperidin-1-yl}benzoylamino]-propionate;
45. (2S)-benzenesulfonylamino-3-[3-{(3S)-(pyrimidin-2-ylamino)piperidin-1-yl}benzoylamino]-propionic acid;
46. (2S)-benzenesulfonylamino-3-[3-{(3S)-(1,4,5,6-tetrahydropyrimidin-2-ylamino)piperidin-1-yl}benzoylamino]propionic acid;
47. t-butyl (2S)-benzenesulfonylamino-3-[3-{(3R)-(pyrimidin-2-ylamino)piperidin-1-yl}benzoylamino]-propionate;
48. (2S)-benzenesulfonylamino-3-[3-{(3R)-(pyrimidin-2-ylamino)piperidin-1-yl}benzoylamino]-propionic acid;
49. (2S)-benzenesulfonylamino-3-[3-{(3R)-(1,4,5,6-tetrahydropyrimidin-2-ylamino)piperidin-1-yl}benzoylamino]propionic acid;
50. t-butyl (2S)-benzenesulfonylamino-3-[3-{4-(pyrimidin-2-ylaminomethyl)piperidin-1-yl}benzoylamino]propionate;
51. (2S)-benzenesulfonylamino-3-[3-{4-(pyrimidin-2-ylaminomethyl)piperidin-1-yl}benzoylamino]propionic acid;
52. (2S)-benzenesulfonylamino-3-[3-{4-(1,4,5,6-tetrahydropyrimidin-2-ylaminomethyl)piperidin-1-yl}benzoylamino]propionic acid;
53. t-butyl (2S)-benzenesulfonylamino-3-[3-{(3S)-hydroxy-(2S)-(pyrimidin-2-ylaminomethyl)pyrrolidin-1-yl}benzoylamino]propionate;
54. (2S)-benzenesulfonylamino-3-[3-{(3S)-hydroxy-(2S)-(pyrimidin-2-ylaminomethyl)pyrrolidin-1-yl}benzoylamino]propionic acid;
55. (2S)-benzenesulfonylamino-3-[3-{(3S)-hydroxy-(2S)-(1,4,5,6-tetrahydropyrimidin-2-ylaminomethyl)-pyrrolidin-1-yl}benzoylamino]propionic acid;
56. t-butyl (2S)-benzenesulfonylamino-3-[3-{(3S)-methoxy-(2S)-(pyrimidin-2-ylaminomethyl)pyrrolidin-1-yl}benzoylamino]propionate;
57. (2S)-benzenesulfonylamino-3-[3-{(3S)-methoxy-(2S)-(pyrimidin-2-ylaminomethyl)pyrrolidin-1-yl}benzoylamino]propionic acid; and
58. (2S)-benzenesulfonylamino-3-[3-{(3S)-methoxy-(2S)-(1,4,5,6-tetrahydropyrimidin-2-ylaminomethyl)-pyrrolidin-1-yl}benzoylamino]propionic acid.

The compounds according to the present invention may form pharmacologically acceptable salts thereof. Such salts include non-toxic salts. Preferred salts include: hydrohalogenic acid salts such as hydrochloride salts, hydrobromide salts, or hydroiodide salts; inorganic acid salts such as nitric acid salts, perchloric acid salts, sulfuric acid salts, or phosphoric acid salts; lower alkylsulfonic acid salts such as methanesulfonic acid salts, trifluoromethanesulfonic acid salts, or ethanesulfonic acid salts; arylsulfonic acid salts such as benzenesulfonic acid salts or p-toluenesulfonic acid salts; organic acid salts such as fumaric acid salts, succinic acid salts, citric acid salts, tartaric acid salts, oxalic acid salts, or maleic acid salts; amino acid salts such as glutamic acid salts or aspartic acid salts; alkali metal or alkaline earth metal salts such as sodium salts, potassium salts, or calcium salts; and organic alkali salts such as pyridine salts or triethylamine salts.

The compounds according to the present invention may form solvates, for example, hydrates; alcoholates, such as methanolates and ethanolates; and etherates, such as tetrahydrofuran.

### Production process of compounds

Compounds represented by formula (I) may be produced according to scheme 1. 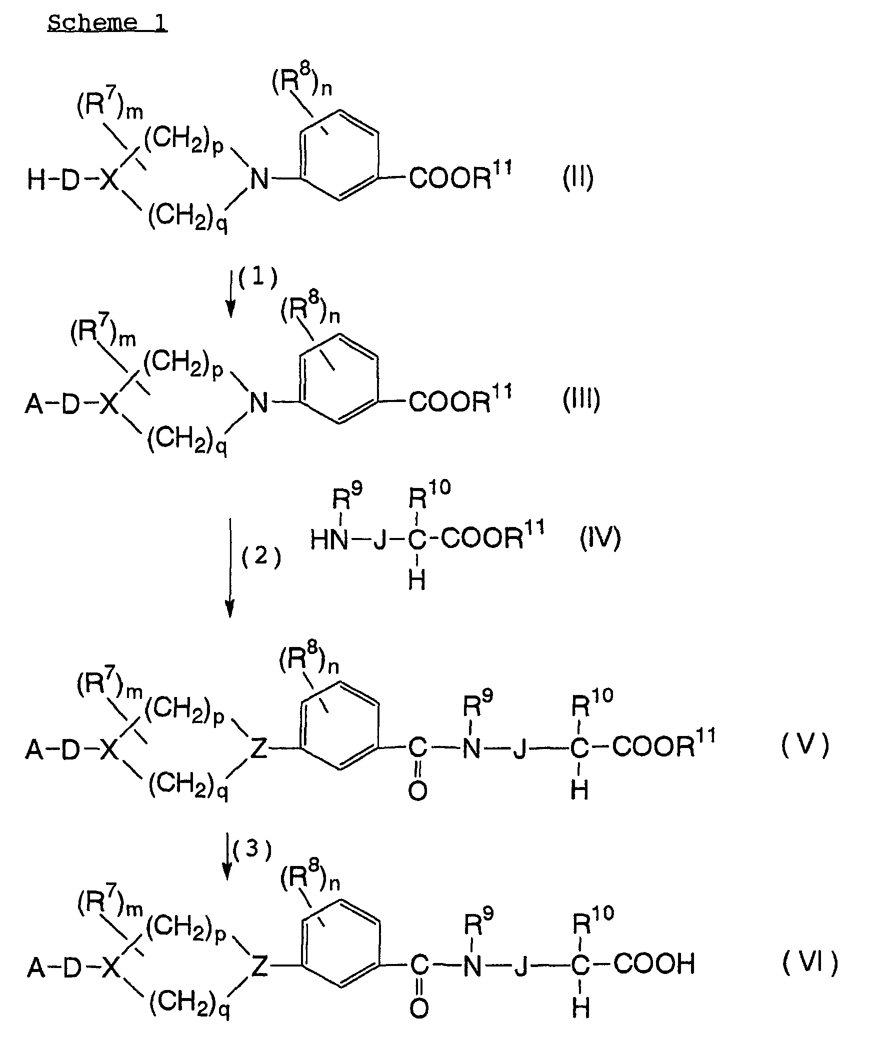 In the above scheme, A, D, X, J, R⁷, R⁸, R⁹, R¹⁰, R¹¹, m, n, p, and q are as defined in formula (I).

### Step (1)

A compound represented by formula (III) may be produced by introducing an atomic group corresponding to group A into the compound represented by formula (II).

The compound represented by formula (III) in scheme 1, wherein D represents >NR⁴ or -NR⁴CR⁵R⁶-, may be produced by introducing group A into the free primary amine in the compound represented by formula (IIa): 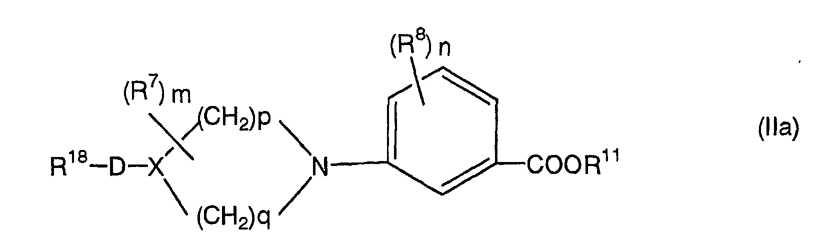 wherein D represents >NR⁴ or -NR⁴CR⁵R⁶-; R¹⁸ represents a substituent of a hydrogen atom or amino, for example, C₁₋₆ alkyl; and X, R⁷, R⁸, R¹¹, m, n, p, and q are as defined above. The N-C bond between the compound represented by formula (IIa) and group A may be formed by reacting the compound represented by formula (IIa) with a reagent, such as optionally modified or substituted 2-bromopyrimidine, modified or substituted 2-chlorobenzimidazole, or 2-methylthio-2-imidazoline, in the presence of a reaction solvent, such as dimethylformamide, dimethyl sulfoxide, sulfolane, pyridine, or methanol, preferably dimethylformamide, in the temperature range of 50 to 170°C, preferably in the temperature range of 60 to 140°C.

Reagents usable in this step are not limited to those recited herein, and any reagent may be used so far as a carbon atom attached to two nitrogen atoms finally combines with the nitrogen atom in the primary amine attached to a carbon atom in the piperidine derivative to form a single bond. Further, optimization of the kind of substrates used and reaction conditions permits the N-C bond to be formed by reacting palladium having a valency of 0 (zero), a phosphine ligand, and a base. Furthermore, the N-C bond may be formed in accordance with the method of Tetrahedron, 51(2), 353, 1995. The reaction may be carried out according to the method described, for example, in production examples of Intermediates 26, 27, 29, and 39 in WO 99/52872.

An organic base, such as diisopropylethylamine, N-methylmorpholine, dimethylaminopyridine, or triethylamine, is preferably added as an acid scavenger from the viewpoint of improving the yield. The addition of 2 to 10 equivalents of diisopropylethylamine is preferred.

The compound represented by formula (III) wherein R⁴ has been substituted may be prepared by conventional or reductive N-alkylation followed by the introduction of group A into the primary amino group in the compound represented by formula (IIa) or by the introduction of group A into the primary amino group in the compound represented by formula (IIa) followed by N-alkylation of the secondary amino group, if necessary. The reaction may be carried out according to the method described in Intermediate 30 in WO 99/52872.

The compound represented by formula (IIa) may be produced by reacting a compound represented by formula (IIb) 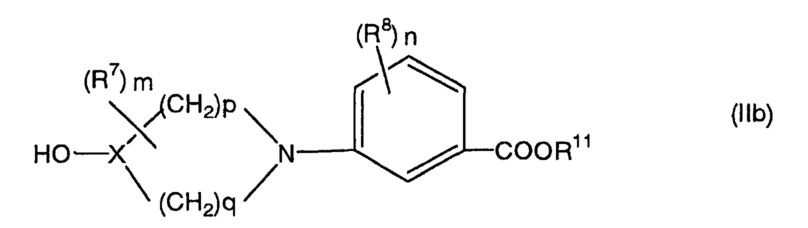 wherein X, R⁷, R⁸, R¹¹, m, n, p, and q are as defined above,
with phthalimide and an azo compound in a reaction solvent such as tetrahydrofuran, benzene, toluene, dioxane, or dimethylformamide, preferably tetrahydrofuran, in the presence of a trialkylphosphine, preferably tributylphosphine, at -40 to 100°C, preferably -10 to 40°C, followed by the removal of the phthaloyl group. Azo compounds include 1,1'-(azodicarbonyl)dipiperidine, diethyl azodicarboxylate, and 1,1'-azobis(N,N-dimethylformamide). Among them, 1,1'-(azodicarbonyl)dipiperidine is preferred.

Alternatively, the compound represented by formula (IIa) may be produced by converting the hydroxyl group in the compound represented by formula (IIb) to a leaving group, for example, a sulfonyloxy group such as a methanesulfonyloxy group, or a halogen atom such as a bromine atom, allowing sodium azide or a combination of hydrazoic acid with an azo compound to act on the leaving group to convert the leaving group to an azide group, and then reducing the azide group. The reaction may be carried out according to the method described, for example, in production examples of Intermediates 35, 36, 41, 42, 43, 47, 48, 49, and 58 in WO 99/52872.

The compound represented by formula (III) in scheme 1, wherein D represents >CR⁵R⁶, may be produced, for example, by reacting 2-(chloromethyl)benzimidazole with ethyl 4-(piperazin-1-yl)benzoate in dimethyl sulfoxide in the presence of potassium carbonate at room temperature. This reaction may be carried out according to the method described in Examples 89 and 90 in WO 99/52872.

The compound represented by formula (III) in scheme 1, wherein D represents -O-, may be produced by reacting the hydroxyl group in the compound represented by formula (IIb) with a basic atomic group having an alkylsulfonyl group, that is, a compound corresponding to group A. This reaction may be carried out in accordance with the method described, for example, in Japanese Patent Laid-Open No. 97818/1993 and EP 468766A1.

The compound represented by formula (III) in scheme 1, wherein D represents -S-, may be produced by halogenating the hydroxyl group in the compound represented by formula (IIb) and reacting the halogen atom with a basic atomic group having group -SH, that is, a compound corresponding to group A. The reaction of the halogen atom with group -SH may be carried out in accordance with the method described, for example, in Res. Lab., Kohjin Co., Ltd., Japan Chem. Pharm. Bull. (1977), 25(10), 2624-37.

The compound represented by formula (III) in scheme 1, wherein D represents a bond, may be produced by introducing group A into a free secondary amine of a compound represented by formula (IIc) 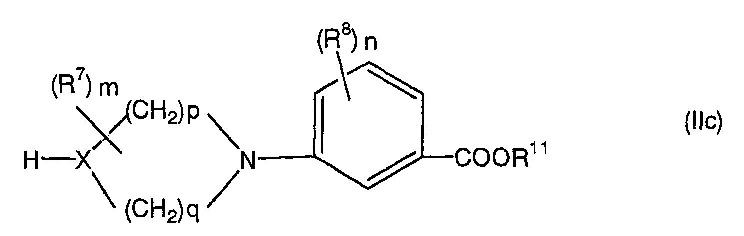 wherein X represents a nitrogen atom; and R⁷, R⁸, R¹¹, m, n, p, and q are as defined above.

The compound represented by formula (IIb) and the compound represented by formula (IIc) may be produced according to the method described, for example, in WO 99/52872 and WO 99/38849.

### Step (2)

A compound represented by formula (V) may be produced by hydrolyzing a carboxylic ester represented by formula (III), wherein R¹¹ is a group other than a hydrogen atom, to give a compound represented by formula (III), wherein R¹¹ represents a hydrogen atom, and then reacting this compound with a compound represented by formula (IV) to form an amide bond. More specifically, the free carboxyl group in the compound represented by formula (III), wherein R¹¹ represents a hydrogen atom, prepared by hydrolysis with an alkali according to a conventional method may be reacted with the amine represented by formula (IV) to perform condensation, thereby producing the compound represented by formula (V).

In the condensation reaction, a condensing agent, such as dicyclohexylcarbodiimide, diisopropylcarbodiimide, or 1-ethyl-3-(3-dimethylaminopropyl)carbodiimide hdyrochloride, may be used either solely or in combination with N-hydroxysuccinimide, 1-hydroxybenzotriazole or the like. Benzotriazol-1-yloxytri(dimethylamino)phosphonium hexafluorophosphate may be used solely in the presence of a base. The combination of these reagents permits the desired condensation reaction to proceed with high efficiency. Preferably, from the viewpoint of optimizing the yield, 1 to 3 equivalents of 1-ethyl-3-(3-dimethylaminopropyl)carbodiimide hydrochloride or its free base is used in combination with 1 to 2 equivalents of 1-hydroxybenzotriazole, or alternatively, 1 to 2 equivalents of benzotriazol-1-yloxytri(dimethylamino)-phosphonium hexafluorophosphate may be used.

Reaction solvents usable in the condensation reaction include dimethylformamide, dioxane, tetrahydrofuran, and methylene chloride. Preferred are dimethylformamide and a mixed solvent composed of dimethylformamide and methylene chloride. The reaction may be carried out in a range of 0 to 80°C, preferably in a range of 0 to 50°C.

In the condensation reaction, a tertiary amine, such as diisopropylethylamine, N-methylmorpholine, dimethylaminopyridine, or triethylamine, may be added as an organic base from the viewpoint of improving the yield. Preferably, 2 to 5 equivalents of N-methylmorpholine or diisopropylethylamine is added.

The reaction proceeds without the addition of these organic bases. The addition of the organic bases, however, is preferred from the viewpoint of the yield.

Compounds represented by formula (V), wherein A represents an optionally substituted pyrimidine ring, may be if necessary reduced to the corresponding tetrahydropyrimidine.

Compounds represented by formula (V), wherein >C=O bonded to the phenylene portion is >CH₂, may be produced by reductively converting the carboxylic ester represented by formula (III), wherein R¹¹ represents a group other than a hydrogen atom, to an aldehyde group and then reductively reacting the aldehyde compound with the amine represented by formula (IV). The reaction may be carried out according to the method described in Example 46 of WO 99/52872.

Compounds represented by formula (V) produced by the reductive amination, wherein R⁹ represents a group other than a hydrogen atom, can also be produced by a reaction process other than the method described herein. Specifically, the above aldehyde compound may be reductively reacted with an amine of formula

H₂N-J-CHR¹⁰COOR¹¹ (IV')

wherein R¹⁰, R¹¹, and J are as defined in formula (I),
to produce a compound represented by formula (V) wherein R⁹ represents a hydrogen atom. Thereafter, this compound may be reductively aminated to introduce alkyl, alkenyl, or aralkyl into R⁹. The introduction of alkyl, alkenyl, or aralkyl into R⁹ is not always carried out only for the compound represented by formula (V) in the scheme. That is, the introduction of alkyl, alkenyl, or aralkyl into R⁹ may be carried out for the compound represented by formula (VI) in the scheme. The reaction may be carried out according to the method described in Example 49 of WO 99/52872.

Further, in this reaction, R¹¹ in -COOR¹¹ corresponding to the carboxylic ester portion in the amine may represent a hydrogen atom.

The amine represented by formula (IV) and the amine represented by formula (IV') may be generally synthesized from a commercially available conventional compound in a single or two steps (for example, K. C. Nicolaou et al., Bioorg. Med. Chem., 6, 1185 (1998)).

When R¹⁰ represents an optionally substituted hydroxyl group, the corresponding carboxylic ester can be produced by treating ω-amino-α-hydoxycarboxylic acid with isobutene under proper reaction conditons, for example, in the presence of sulfuric acid. If necessary, the hydroxyl group may be protected. Specifically, for example, 3-amino-(2S)-hydroxypropionic acid (L-isoserine) or 4-amino-(2S)-hydroxybutyric acid (AHBA) may be used as ω-amino-α-hydroxycarboxylic acid. Protective groups of the carboxyl group include lower alkyl esters and aralkyl esters. For example, ethyl ester, t-butyl ester, and benzhydryl ester may be used.

In the esterification for the production of a t-butyl ester of 3-amino-(2S)-hydroxypropionic acid or 4-amino-(2S)-hydroxybutyric acid, a part of the hydroxyl group is sometimes t-butylated (etherified). In the condensation reaction in step (2), however, the hydroxyl group at the α-position may be protected or may not be protected.

In the t-butylation of 3-amino-(2S)-hydroxypropionic acid or 4-amino-(2S)-hydroxybutyric acid, when a conventional method described, for example, in WO 95/32710 as such is applied, the yield of the t-butylation can be improved, for example, by properly selecting the reaction solvent, the stirring efficiency, and the type and amount of the acid catalyst.

The esterification for the production of a benzhydryl ester of 3-amino-(2S)-hydroxypropionic acid or 4-amino-(2S)-hydroxybutyric acid proceeds without posing the problem of yield. In this case, when the amino group is previously converted to an acid salt, for example, a p-toluenesulfonic acid salt, the yield can be improved. The reaction solvent is not particularly limited. The reaction reagent is preferably diphenyldiazomethane.

Ester derivatives of 3-amino-(2S)-hydroxypropionic acid or 4-amino-(2S)-hydroxybutyric acid and hydroxyl-protected derivatives thereof, wherein R¹⁰ represents a hydrogen atom, may be used in the condensation reaction in step (2). Alternatively, the amino group may be further modified followed by the use of the modified amino group in the condensation reaction. An example of chemical modification of the ω-amino group is alkylation. The alkylation may be carried out according to the method described in WO 99/38849 or WO 99/52872.

### Step (3)

The compound represented by formula (VI) may be prepared by converting the carboxylic ester portion (-COOR¹¹) in the compound represented by formula (V) to a free carboxyl group, if necessary.

The carboxylic ester portion in the compound represented by formula (V) may be converted to the contemplated free carboxyl group by a conventional method, for example, by hydrolysis with an alkali, hydrolysis with an acid, or reaction with an acid. The deesterification reaction may be achieved by a novel method without any restriction or limitation.

The compound represented by formula (V) is orally administrable integrin αᵥβ₃ antagonist and/or GP IIb/IIIa antagonist. Therefore, the step of converting the carboxylic ester to the free carboxyl group is not always necessary.

Compounds represented by formula (VI), wherein A represents an optionally substituted pyrimidine ring, may be if necessary, reduced to the corresponding tetrahydropyrimidine. The reduction may be carried out by a conventional method. Examples of reduction methods usable herein include catalytic reduction in the presence of a catalyst, such as palladium-carbon, ruthenium-carbon, rhodium-carbon, palladium oxide, platinum oxide, ruthenium oxide, rhodium platinum oxide complex, rhodium aluminum oxide complex, Raney nickel, or palladium black, and a reaction, for example, with metallic sodium or metallic lithium in liquid ammonia. Preferably, the reduction is carried out in an acidic solvent, for example, in acetic acid acidified with hydrochloric acid, in the presence of palladium-carbon with hydrogen under normal or applied pressure. The conversion may be carried out before or after the formation of the amide bond.

In scheme 1, in producing the compound represented by formula (III), a benzoic ester is first bonded to the hetero ring, followed by the introduction of a basic atomic group, for example, an amidino or pyrimidinyl group. This step, however, is not limited to this method only Alternatively, the introduction of a basic atomic group into the hetero ring may be followed by attachment of the benzoic ester to the hetero ring.

The compound represented by formula (I) may also be produced according to scheme 2. 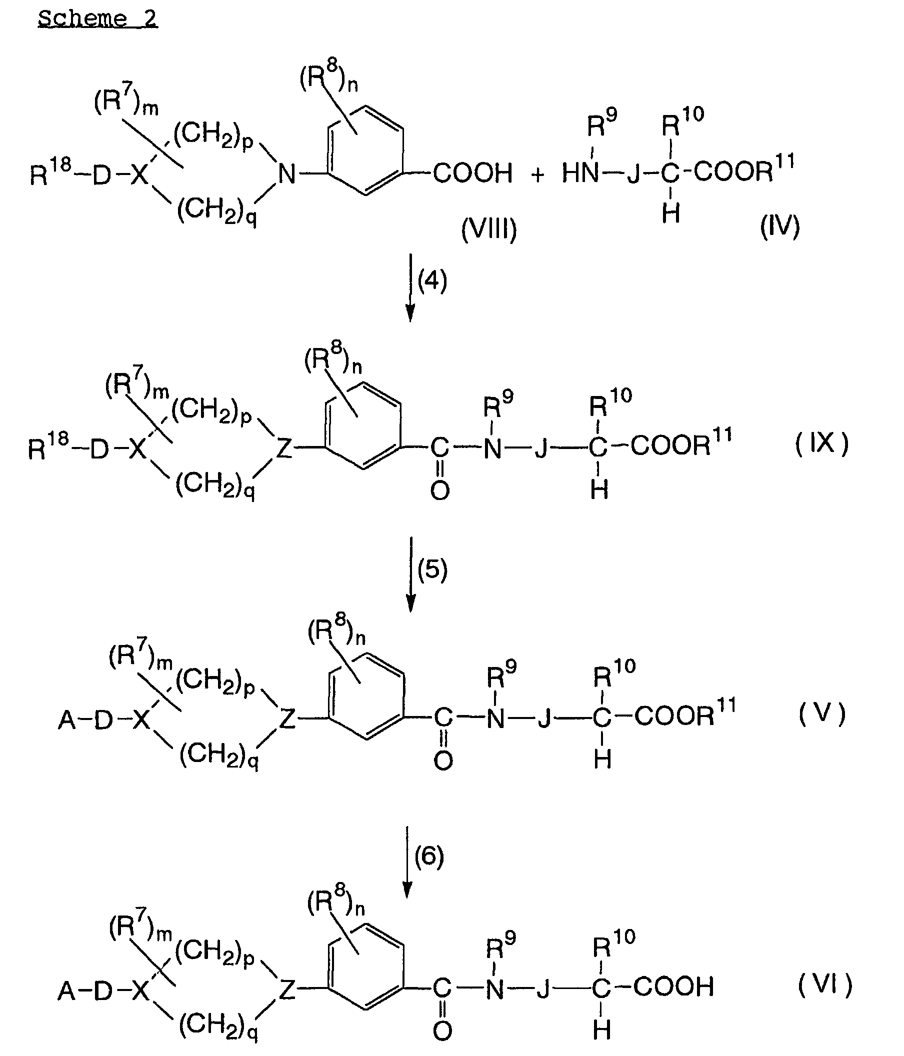 In the above scheme, R¹⁸ represents a protective group for amino; and A, D, X, J, R⁷, R⁸, R⁹, R¹⁰, R¹¹, m, n, p, and q are as defined in formula (I).

### Step (4)

A compound represented by formula (IX) may be produced by reacting a compound represented by formula (VIII) with an amine represented by formula (IV) to form an amide bond. More specifically, the compound represented by formula (IX) may be produced by reacting the amine represented by formula (IV) with the free carboxyl group in the compound represented by formula (VIII) to perform condensation. In the compound represented by formula (VIII), R¹⁸ represents a protective group of amino. Protective groups of amino include Fmoc (9-fluorenylmethoxycarbonyl), t-butyloxycarbonyl, benzyloxycarbonyl, and p-methoxybenzyloxycarbonyl. Preferred is t-butyloxycarbonyl.

The compound represented by formula (VIII) may be produced by hydrolyzing, with an alkali, the benzoic ester represented by formula (IIa) according to a conventional method.

### Steps (5) and (6)

The compound represented by formula (V) may be produced by removing the protective group in the piperidine derivative portion, introducing a basic atomic group corresponding to group A, for example, a pyrimidine, benzimidazole, or amidino group, into the deprotected primary amine, and then optionally converting the carboxylic ester portion to a free carboxyl group.

If necessary, the carboxylic ester portion (-COOR¹¹) in the compound represented by formula (V) may be converted to a free carboxyl group to produce the compound represented by formula (VI).

The pyrimidine ring represented by A may be converted to tetrahydropyrimidne by catalytic reduction. The conversion may be carried out before or after the formation of the amide bond.

In the compound represented by formula (V) and the compound represented by formula (VI) in scheme 1, atomic groups, which have already been constructed in the molecule, for example, R⁷, R⁸, R⁹, and R¹⁰, may be, if necessary, further converted.

The production of the compound represented by formula (II) will be described in conjunction with the following reverse synthesis analysis diagram.

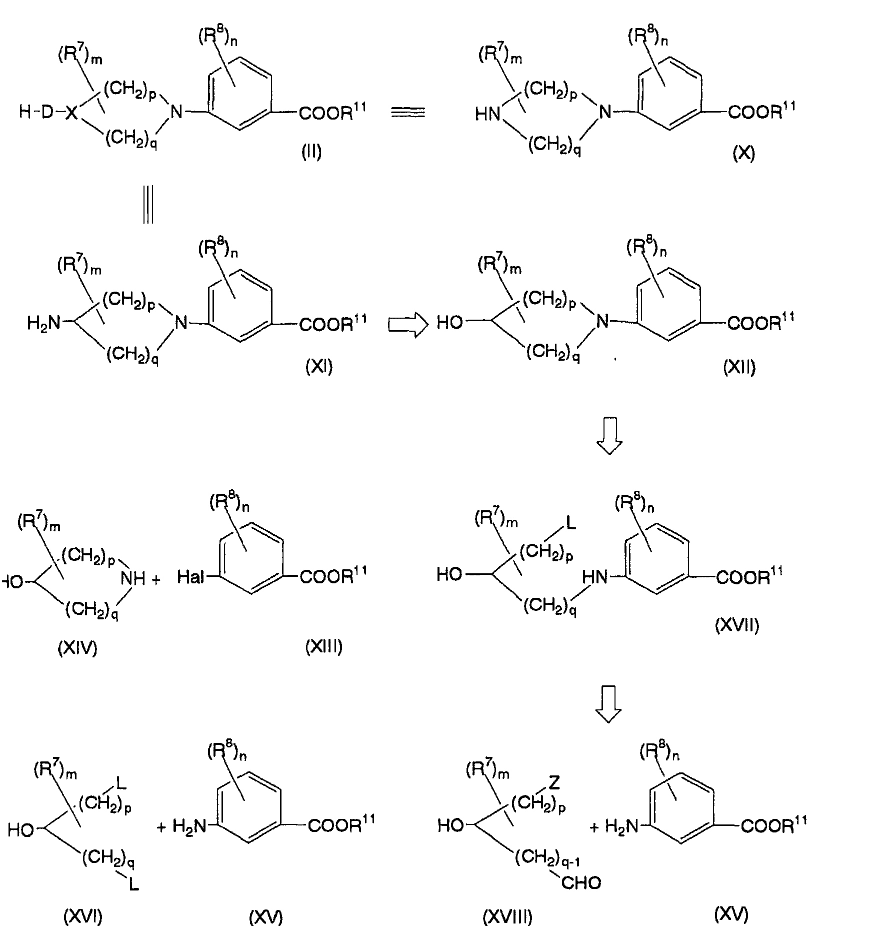

In the above scheme, L represents a leaving group; Z represents a functional group, which can be chemically converted to a leaving group, for example, a hydroxyl group; Hal represents a halogen atom, for example, bromine; D, X, J, R⁷, R⁸, R⁹, R¹⁰, R¹¹, m, n, p, and q are as defined in formula (I).

For the compounds represented by formula (II), phenylpiperazine derivatives represented by formula (X) or phenylpiperidine derivatives represented by formula (XI) are actually described in working examples.

Among them, the compound represented by formula (X) may be produced by a conventional method (see, for example, WO 98/54135).

On the other hand, the production of a compound represented by formula (XII), which is one of precursors of the compound represented by formula (XI), involves the following problems. In general, the compound represented by formula (XII) may be produced by reacting a halide represented by formula (XIII), for example, a bromide, with an amine represented by formula (XIV) in the presence of a palladium catalyst (J. P. Wolfe et al., Tetrahedron Lett., 38, 6359 (1997)). In this method, however, a catalyst, which is not inexpensive, and a phosphine ligand should be used. Further, this reaction does not always give a good yield when the substrate has a free hydroxyl group. In this respect, it can be said that, in the above production process, there is room for improvement.

On the other hand, one of other conventional methods for producing the compound represented by formula (XII) is to react aniline represented by formula (XV), for example, with a dihalide or a disulfonate represented by formula (XVI) (G. R. Owen et al., J. Chem. Soc. (C), 2401 (1970), and C. B. Reese et al., J. Chem. Soc. Perkin Trans., I, 2881 (1988)). This reaction per se involves no severe problem. When systematic synthesis of derivatives, in which various substituents have been introduced into the hetero ring, especially the piperidine ring, of the compounds according to the present invention is contemplated, however, intermediates represented by formula (XVI) should be prepared one by one. Therefore, it is difficult to say that this method is efficient.

Accordingly, the present inventors have studied on production processes which can efficiently produce compounds represented by formula (XII) and, as a result, have found that naturally occurring saccharides represented by formula (XVIII), especially monosaccharides, for example, 2-deoxy-D-ribose, are excellent starting compounds for preparing the compounds represented by formula (XII) and analogues thereof. More specifically, reducing sugar, for example, 2-deoxy-D-ribose, was reductively reacted with an amino group in a compound represented by formula (XV) in the presence of hydride reagent. Thereafter, only the primary hydroxyl group was selectively brominated to synthesize a compound represented by formula (XVII) which spontaneously caused an intramolecular cyclization to give the target compound represented by formula (XII). In this reaction, the substrate for the reaction is not limited to deoxyribose, and general saccharides, which widely exist in nature, especially monosaccharides, can be applied. Further, advantageously, a substituent can be stereo-specifically constructed in a piperidine ring which is newly constructed.

Thus, according to the present invention, there is provided a process for producing an intermediate (XXII) represented by scheme 3. 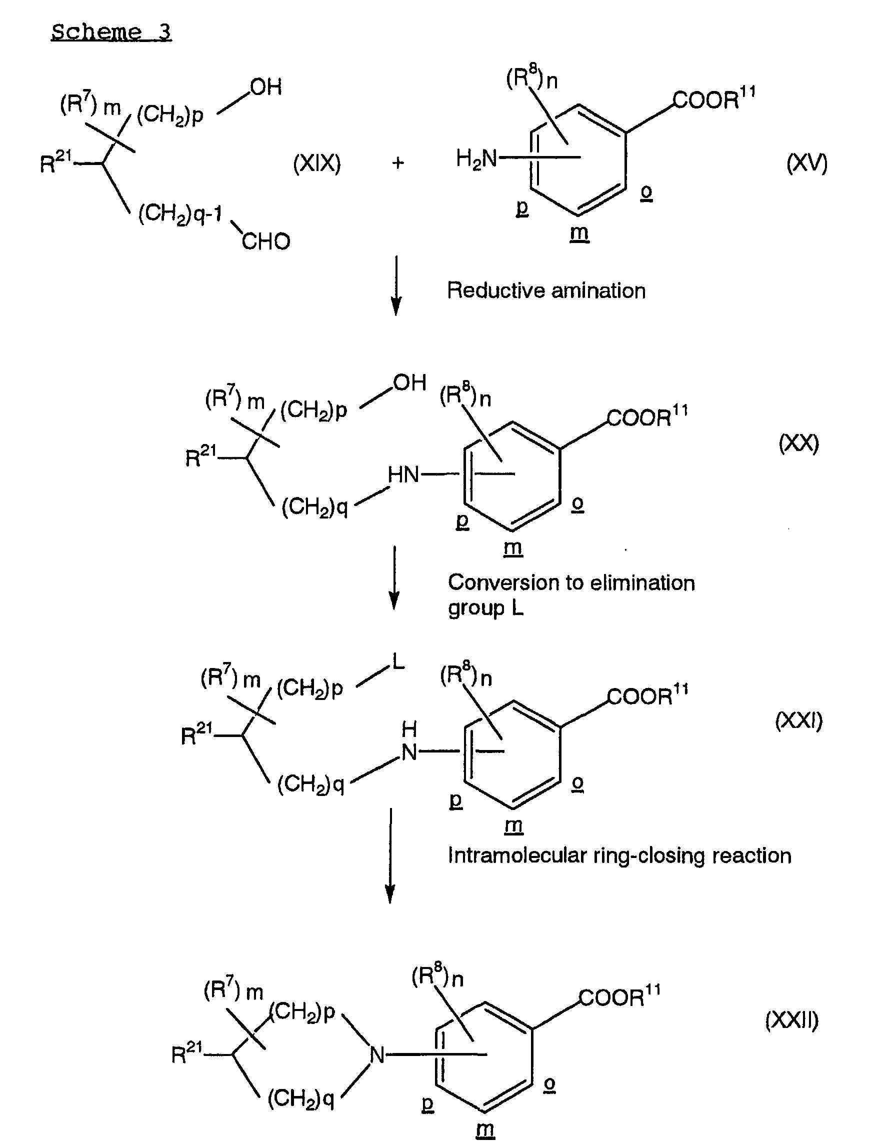 In the above scheme, R²¹ represents hydroxyl, azide, or optionally protected amino; L represents a leaving group; R⁷, R⁸, R¹¹, m, n, p, and q are as defined in formula (I), provided that q is not 0 (zero); and the nitrogen atom is attached to the ortho-position (o), meta-position (m), or para-position (p), preferably meta-position, of the phenyl group.

A compound represented by formula (XIX) is first reacted with a compound represented by formula (XV) to give a compound represented by formula (XX).

When naturally occurring monosaccharides are used in the production process according to the present invention, they are not required to be free aldehydes, that is, of ring opening type, and may be hemi-acetals of ring closing type.

The compound represented by formula (XIX) may be reacted with the compound represented by formula (XV) by reductive amination. The reductive amination can be carried out in the presence of a hydride reagent, for example, a reagent such as sodium boron cyanohydride, sodium borohydride, sodium triacetoxy borohydride, or lithium borohydride, in a reaction solvent such as methanol, methylene chloride, dimethylformamide, or dichloroethane, or a mixed solvent thereof, at 0°C to 50°C (provided that the temperature should not exceed the boiling point of the reaction solvent), preferably at room temperature.

For some structures of naturally occurring saccharides used in the reaction, a certain reaction time is required for the formation of an imine which is one of intermediates. The timing of addition of the hydride reagent can be determined according to the substrate used. More specifically, the hydride reagent may be added immediately after the initiation of the reaction, or alternatively a method may be adopted wherein two substrates are dissolved in the reaction solvent and, after the elapse of a certain period of time after the dissolution of the substrates in the reaction solvent, for example, 24 hr after the dissolution of the substrates in the reaction solvent, the hydride reagent is added. Further, in this case, certain organic acids, such as, acetic acid, citric acid, formic acid, methanesulfonic acid, monochloroacetic acid, and monofluoroacetic acid, or inorganic acids, such as, hydrochloric acid or sulfuric acid, may be added, for example, from the viewpoint of improving the selectivity of the hydride reagent in the reaction.

Next, the primary hydroxyl group in the compound represented by formula (XX) is converted to a leaving group L to give the compound represented by formula (XXI).

The conversion to the leaving group L can be carried out by a conventional method. For example, the primary hydroxyl group can be converted to a bromine atom by reacting the primary hydroxyl group with carbon tetrabromide and triphenylphosphine.

Leaving groups L include halogen atoms, for example, a chlorine atom, a bromine atom, an iodine atom, and sulfonates, for example, methanesulfonyloxy, p-toluenesulfonyloxy, benzenesulfonyloxy, and trifluoromethanesulfonyloxy.

The compound represented by formula (XXI), in which a leaving group L has been constructed, may be converted by an intramolecular ring-closing reaction to a compound represented by formula (XXII).

Upon the introduction of the leaving group L, the intramolecular ring-closing reaction proceeds spontaneously.

Bases usable in the intramolecular ring-closing reaction include tertiary organic bases, such as diisopropylethylamine, triethylamine, tribenzylamine, and inorganic bases, such as sodium carbonate and potassium carbonate. The use of organic bases is preferred from the viewpoint of the selectivity in reaction.

After the intramolecular ring-closing reaction, if necessary, the functional group represented by R²¹ may be further chemically converted. For example, the hydroxyl group may be converted to an azide group. Further, the azide group may be reductively converted to an amino group. The reduction reaction of the azide group may be carried out according to a conventional method.

The reaction solvent used in the step of conversion to a leaving group and the step of an intramolecular ring-closing reaction is not particularly limited.

The compound represented by formula (XXII) thus obtained may be treated according to the steps in scheme 1 or scheme 2 to give the compound represented by formula (I).

In formulae (XIX), (XX), (XXI), and (XXII) in scheme 3, preferably, p and q are 2, R⁷ and R²¹ represent hydroxyl, and m is 1.

In scheme 3, the compound represented by formula (XIX) may be selected from the group consisting of pentoses, hexoses, and heptoses and derivatives thereof. "Derivatives" as used herein include, for example, monosaccharides of which a hydroxyl group has been converted to other functional groups, such as C₁₋₄ alkyl (for example, methyl), C₁₋₄ alkoxy (for example, methoxy), azide, and amino.

More preferably, the compound represented by formula (XIX) can be 2-deoxy-D-ribose represented by formula (XIX') or derivatives thereof:

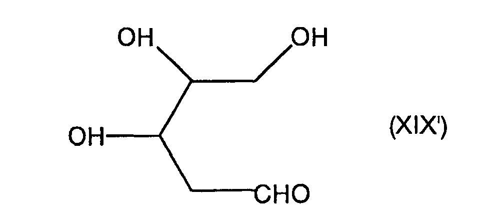

When the compound represented by formula (XIX) is 2-deoxy-D-ribose, the compounds represented by formulae (XX), (XXI), and (XXII) in scheme 3 can be respectively compounds represented by formulae (XX'), (XXI'), and (XXII'): 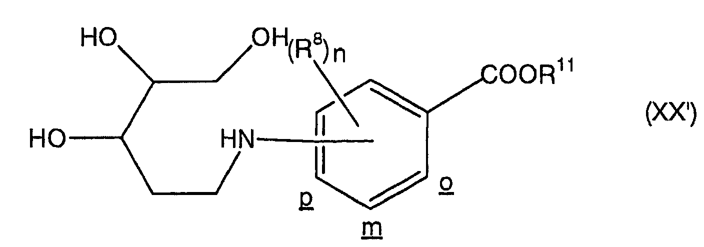 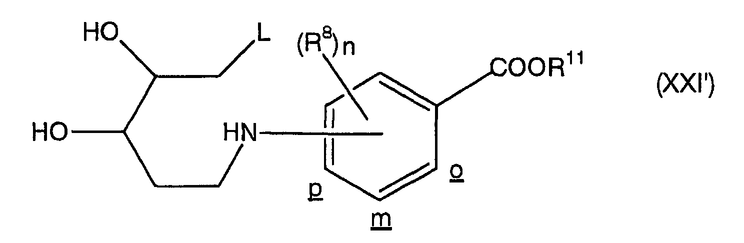 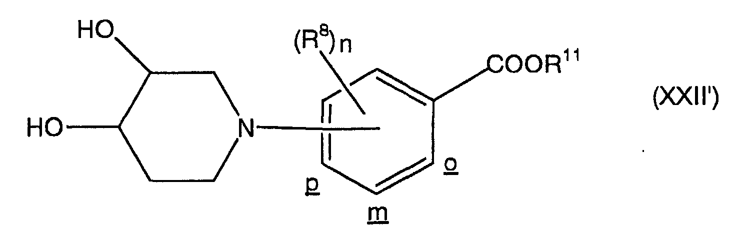 wherein R⁸, R¹¹, and n are as defined in formula (I); and the nitrogen atom is attached to the ortho-, meta-, or para-position, preferably meta-position, of the phenyl group.

### Use of compounds/pharmaceutical composition

The compounds according to the present invention have potent integrin αᵥβ₃ antagonistic activity, as demonstrated in Pharmacological Test Example 1. Accordingly, the compounds according to the present invention may be used in the treatment of integrin αᵥβ₃-mediated diseases. The integrin αᵥβ₃ mediates cardiovascular diseases such as acute myocardial infarction, neointima formation hypertrophy, restenosis after PTCA/stent operation, unstable angina, acute coronary syndrome, angina pectoris after PTCA/stent operation, arterial sclerosis, particularly atherosclerosis; angiogenesis-related diseases such as diabetic retinopathy, diabetic vascular complication, or vascular grafting; cerebrovascular diseases such as cerebral infarction; cancers such as solid tumors or metastasis thereof; immunological diseases such as arthritis, particularly rheumatic arthritis; and osteopathy such as osteoporosis, hypercalcemia, periodontitis, hyperparathyroidism, periarticular sore, or Paget's diseases (DN & P, 10 (8), 456 (1997)). The term "therapy" or "treatment" as used herein includes "prevention" or "prophylaxis."

The compounds according to the present invention have cell adhesion inhibitory activity, as demonstrated in Pharmacological Test Example 3. Accordingly, the compounds according to the present invention may be used in the treatment of diseases where the inhibition of cell adhesion is therapeutically effective. More specifically, diseases such as cardiovascular diseases, angiogenesis-related diseases, cerebrovascular diseases, cancers, and immunological diseases can be treated by inhibiting the adhesion between smooth muscle cells and cell adherent proteins, particularly vitronectin (DN & P, 10 (8), 456 (1997)). Further, cancers or metastasis thereof can be treated by inhibiting the adhesion between vascular endothelial cells and cell adherent proteins, particularly vitronectin. Furthermore, osteopathy can be treated by inhibiting the adhesion between osteoclasts and cell adherent proteins, particularly osteopontin.

The term "cell adhesion" as used herein means adhesion between vascular cells, specifically smooth muscle cells and endothelial cells, and cell adherent proteins, specifically vitronectin, osteopontin, and von Willebrand factors; adhesion between vascular cells and hemocyte cells, specifically leukocyte; and adhesion between hemocyte cells themselves, particularly adhesion between human vascular smooth muscle cells and human vitronectin.

As described in Pharmacological Test Example 2, the compounds according to the present invention have GP IIb/IIIa antagonistic activity and human platelet aggregation inhibitory activity. Therefore, the compounds according to the present invention can be used in the treatment of diseases where GP IIb/IIIa antagonism and the inhibition of human platelet aggregation are therapeutically effective. More specifically, the compounds according to the present invention can be used in the treatment of platelet thrombosis and thromboembolism during and after the treatment of thrombolysis and after angioplasty of coronary artery and other arteries and after bypassing of coronary artery, the improvement of peripheral circulating blood stream, and the inhibition of blood clotting during extracorporeal circulation. Furthermore, the compounds according to the present invention can be used in the treatment of thrombotic thrombocytopenic purpura and hemolytic uremic syndrome (Gendai Iryo, 29, (11), 2753 (1997)).

Not only compounds represented by formula (I) wherein R¹¹ represents alkyl, but also compounds represented by formula (I) wherein R¹¹ represents a hydrogen atom, had oral absorption in rats (data not shown). Therefore, any of the compounds, wherein R¹¹ represents alkyl or a hydrogen atom, can be used in the treatment of the above diseases.

The compounds according to the present invention and pharmacologically acceptable salts and solvates thereof can be administered orally or parenterally by administration routes, for example, inhalation administration, rhinenchysis, instillation, subcutaneous administration, intravenous injection, intravenous drip infusion, intramuscular injection, rectal administration, or percutaneous administration, and thus may be formed into appropriate various dosage forms depending on oral or parenteral administration routes and administered to human and non-human animals.

The compounds according to the present invention may be formulated into, for example, oral preparation, such as tablets, capsules, chewable preparations, granules, powders, pills, particulates, troches, syrups, or emulsions; liquids for external use such as inhalants, nasal drops, or eye drops; patches; injections such as intravenous injections or intramuscular injections and intravenous drip infusions; preparations for rectal administrations; oleaginous suppositories; water-soluble suppositories; and liniments such as ointments depending upon applications thereof. Further, liquid preparations, such as injections or drops, may be provided, for example, as a lyophilized powdery pharmaceutical composition, which may be dissolved or suspended in water or other suitable vehicle, for example, a physiological saline, a glucose infusion, or a buffer solution, before use.

These various preparations may be prepared by conventional methods with commonly used components, for example, excipients, extenders, binders, humidifiers, disintegrants, surface active agents, lubricants, dispersants, buffers, preservatives, dissolution aids, antiseptics, flavoring agents, analgesic agents, stabilizers and the like. Non-toxic additives usable herein include, for example, lactose, fructose, glucose, starch, gelatin, magnesium carbonate, synthetic magnesium silicate, talc, magnesium stearate, methylcellulose, carboxymethylcellulose or a salt thereof, gum arabic, olive oil, propylene glycol, polyethylene glycol, syrup, petrolatum, glycerin, ethanol, citric acid, sodium chloride, sodium sulfite, sodium phosphate, ascorbic acid, and cyclodextrins.

The dose of the compound according to the present invention in the medicament may vary depending on the dosage form. The dose is, however, generally 1.0 to 100% by weight, preferably 1.0 to 60% by weight, based on the whole composition.

Regarding the pharmaceuticals according to the present invention, the dose and the number of times of administration are not particularly limited, and may be appropriately determined depending on various conditions, for example, the purpose of treatment or prevention, the type of diseases, the age, weight, and severity of condition of patients. The dose for the treatment and prevention of coronary diseases and the like may be appropriately determined depending on, for example, the dosage route and the age, sex and severity of condition of patients, and the active ingredient may be administered usually in an amount of about 0.1 to 2,000 mg, preferably about 5 to 400 mg per day per adult. This dose may be administered at a time daily, divided doses of several times daily, or at a time every several days.

### EXAMPLES

The present invention will be described in more detail with reference to the following examples, though it is not limited to these examples only.

### Intermediate 1: Ethyl 3-{4-(pyrimidin-2-yl)piperazin-1-yl}benzoate

Dimethyl sulfoxide (11 ml) was added to 264 mg of ethyl 3-(piperazin-1-yl)benzoate, and 269 mg of 2-bromopyrimidine and 1.0 ml of diisopropylethylamine were then successively added thereto. The mixture was heated at 120°C for 12 hr. The temperature of the reaction mixture was returned to room temperature, and the reaction mixture was then added dropwise to 250 ml of water, followed by stirring at room temperature for one hr. The insolubles were collected by filtration, and were then washed twice with 20 ml of water. The solid was dried under the reduced pressure in the presence of diphosphorus pentaoxide at 50°C, and was then purified by column chromatography on silica gel (16 g, 2% methanol/methylene chloride) to give 317 mg of the title compound.
Physicochemical properties of intermediate 1
(1) Color and form: Colorless solid
(2) Molecular formula: C₁₇H₂₀N₄O₂
(3) Mass spectrum (EIMS): m/z 312 M⁺
(4) ¹H NMR spectrum (400 MHz, CDCl₃) δ (ppm): 1.40 (3H, t, Et), 3.30 (4H, m, piperazine), 4.00 (4H, m, piperazine), 4.38 (2H, q, Et), 6.53 (1H, t, pyrimidine), 7.15 (1H, br ddd, C₆H₄), 7.34 (1H, t, C₆H₄), 7.56 (1H, br ddd, C₆H₄), 7.64 (1H, br dd, C₆H₄), 8.34 (2H, d, pyrimidine)

### Intermediate 2: 3-{4-(Pyrimidin-2-yl)piperazin-1-yl}benzoic acid

Tetrahydrofuran (27 ml), 9.0 ml of methanol, and 9.0 ml of a 1 N aqueous sodium hydroxide solution were added in that order to 300 mg of intermediate 1. The reaction mixture was then heated at 45°C for 7 hr. The reaction solution was concentrated under the reduced pressure, and the residue was purified by column chromatography on silica gel (15 g, 10% methanol/methylene chloride) to give 264 mg of the title compound.
Physicochemical properties of intermediate 2
(1) Color and form: Colorless solid
(2) Molecular formula: C₁₅H₁₆N₄O₂
(3) Mass spectrum (TSP (thermospray) MS): m/z 285 (M+H)⁺
(4) ¹H NMR spectrum (400 MHz, CDCl₃ - CD₃OD = 1 : 1) δ (ppm): 3.32 (4H, m, piperazine), 3.99 (4H, m, piperazine), 6.61 (1H, t, pyrimidine), 7.22 (1H, br dd, C₆H₄), 7.36 (1H, t, C₆H₄), 7.57 (1H, br ddd, C₆H₄), 7.67 (1H, br dd, C₆H₄), 8.35 (2H, d, pyrimidine)

### Example 1: t-Butyl (2S)-benzenesulfonylamino-3-[3-{4-(pyrimidin-2-yl)piperazin-1-yl}benzoylamino]propionate

Dimethylformamide (6.5 ml) and 6.5 ml of methylene chloride were added to 256 mg of intermediate 2 and 597 mg of benzotriazol-1-yloxytri(dimethylamino)phosphonium hexafluorophosphate to prepare a solution. Diisopropylethylamine (0.24 ml) was added to the solution, and a reaction was allowed to proceed at room temperature for 2 hr. Separately, 6.5 ml of methylene chloride was added to 325 mg of t-butyl (2S)-N-benzenesulfonyl-2,3-diaminopropionate to prepare a solution. This solution was added to the above active ester solution at 0°C. Diisopropylethylamine (0.12 ml) was added thereto, and a reaction was allowed to proceed at room temperature for 16 hr. The reaction solution was concentrated under the reduced pressure, and the residue was extracted with 50 ml of ethyl acetate, followed by washing with a saturated aqueous sodium hydrogencarbonate solution and saturated brine in that order. The extract was then dried over anhydrous sodium sulfate. The ethyl acetate layer was concentrated under the reduced pressure, and the residue was purified by column chromatography on silica gel (30 g, 60% → 67% acetone/hexane) to give 482 mg of the title compound. Physicochemical properties of the compound prepared in Example 1
(1) Color and form: Colorless solid
(2) Molecular formula: C₂₈H₃₄N₆O₅S
(3) Mass spectrum (FABMS): m/z 567 (M+H)⁺
(4) Specific rotation: [α]_{D}²⁵ +45° (c 1.0, CHCl₃)
(5) ¹H NMR spectrum (400 MHz, CDCl₃) δ (ppm): 1.29 (9H, s, t-Bu), 3.33 (4H, m, piperazine), 3.57 (1H, ddd, CONHCH₂), 3.93 (2H, m, CONHCH₂CH), 3.99 (4H, m, piperazine), 6.53 (1H, t, pyrimidine), 7.10 (1H, br dd, C₆H₄), 7.22 (1H, br d, C₆H₄), 7.34 (1H, t, C₆H₄), 7.46 (1H, br dd, C₆H₄), 7.50 (2H, m, Ph), 7.58 (1H, m, Ph), 7.86 (2H, m, Ph), 8.34 (2H, d, pyrimidine)

### Example 2: (2S)-Benzenesulfonylamino-3-[3-{4-(pyrimidin-2-yl)piperazin-1-yl}benzoylamino]propionic acid

Methylene chloride (1.0 ml) and 0.05 ml of anisole were added to 85.1 mg of the compound prepared in Example 1 to prepare a solution, and the solution was cooled to 0°C. Trifluoroacetic acid (1.0 ml) was added thereto, and a reaction was allowed to proceed at room temperature for 16 hr. The reaction solution was concentrated under the reduced pressure, and the residue was subjected to azeotropic distillation twice with 2.0 ml of toluene. The product obtained by the azeotropic distillation was then washed twice with 2.0 ml of isopropyl ether. The residue was purified by column chromatography on silica gel (8.0 g, chloroform-methanol-concentrated aqueous ammonia = 9 : 2 : 0.1) to give 67.0 mg of the title compound.
Physicochemical properties of the compound prepared in Example 2
(1) Color and form: Colorless solid
(2) Molecular formula: C₂₄H₂₆N₆O₅S
(3) Mass spectrum (TSPMS): m/z 511 (M+H)⁺
(4) Specific rotation: [α]_{D}²⁵ +60° (c 1.0, MeOH)
(5) ¹H NMR spectrum (400 MHz, CD₃OD) δ (ppm): 3.30 (4H, m, piperazine), 3.54 (1H, dd, CONHCH₂), 3.71 (1H, dd, CONHCH₂), 3.83 (1H, dd, CONHCH₂CH), 3.96 (4H, m, piperazine), 6.60 (1H, t, pyrimidine), 7.18 (1H, br dd, C₆H₄), 7.26 (1H, br d, C₆H₄), 7.33 (1H, t, C₆H₄), 7.46 (3H, m, 1H of C₆H₄ and 2H of Ph), 7.52 (1H, m, Ph), 7.86 (2H, m, Ph), 8.34 (2H, d, pyrimidine)

### Example 3: (2S)-Benzenesulfonylamino-3-[3-{4-(1,4,5,6-tetrahydropyrimidin-2-yl)piperazin-1-yl}benzoylamino]-propionic acid

1,4-Dioxane (2.8 ml), 1.6 ml of acetic acid, 0.8 ml of water, and 0.8 ml of 1 N hydrochloric acid were successively added to 60.0 mg of the compound prepared in Example 2 to prepare a solution. To the solution was added 15 mg of 10% palladium-carbon, and the mixture was stirred in a hydrogen atmosphere at room temperature for 5 hr. The insolubles were filtered and were then washed twice with 2.0 ml of a solvent having the same composition as the mixed solvent used in the reaction, and the filtrate and the washings were combined followed by concentration under the reduced pressure. The residue was subjected to azeotropic distillation twice with 4.0 ml of toluene. The product obtained by the azeotropic distillation was first purified by preparative thin-layer chromatography on silica gel (development system: methylene chloride : ethanol : water : concentrated aqueous ammonia = 8 : 8 : 1 : 1) to give 45.7 mg of a solid, and was finally purified by Sephadex LH-20 (45 ml, 10% concentrated aqueous ammonia/methanol) to give 31.1 mg of the title compound.
Physicochemical properties of the compound prepared in Example 3
(1) Color and form: Colorless syrup
(2) Molecular formula: C₂₄H₃₀N₆O₅S
(3) Mass spectrum (TSPMS): m/z 515 (M+H)⁺
(4) Specific rotation: [α]_{D}²⁵ +69° (c 1.0, MeOH)
(5) ¹H NMR spectrum (400 MHz, CD₃OD) δ (ppm): 1.94 (2H, quintet, tetrahydropyrimidine), 3.28 (4H, m, piperazine), 3.38 (4H, t, tetrahydropyrimidine), 3.51 (4H, m, piperazine), 3.54 (1H, dd, CONHCH₂), 3.69 (1H, dd, CONHCH₂), 3.76 (1H, dd, CONHCH₂CH), 7.10 (1H, br d, C₆H₄), 7.30 (2H, m, C₆H₄), 7.41 (1H, br s, C₆H₄), 7.47 (2H, m, Ph), 7.53 (1H, m, Ph), 7.85 (2H, m, Ph)

### Intermediate 3: Ethyl 3-(4-hydroxypiperidin-1-yl)benzoate

Toluene (200 ml) was added to 5.00 g of ethyl 3-bromobenzoate to prepare a solution. The solution was added to 2.65 g of 4-hydroxypiperidine. Further, 9.96 g of anhydrous cesium carbonate, 73.5 mg of palladium(II) acetate, and 204 mg of (R)-(+)-2,2'-bis(diphenylphosphino)-1,1'-binaphthyl were added thereto in that order, and the mixture was stirred with heating at 90°C for 5 hr and then at 100°C for 2 hr. The temperature of the reaction mixture was returned to room temperature, and the reaction mixture was then added dropwise to 400 ml of a saturated aqueous ammonium chloride solution, followed by extraction with 200 ml of ethyl acetate. The ethyl acetate layer was dried over anhydrous sodium sulfate and was then concentrated under the reduced pressure. The residue was purified by column chromatography on silica gel (400 g, 67% → 75% ethyl acetate/hexane) to give 493 mg of the title compound.
Physicochemical properties of intermediate 3
(1) Color and form: Colorless oil
(2) Molecular formula: C₁₄H₁₉NO₃
(3) Mass spectrum (TSPMS): m/z 250 (M+H)⁺
(4) ¹H NMR spectrum (400 MHz, CDCl₃) δ (ppm): 1.39 (3H, t, Et), 1.70 (2H, m, piperidine), 2.02 (2H, m, piperidine), 2.97 (2H, ddd, piperidine), 3.61 (2H, m, piperidine), 3.87 (1H, m, piperidine), 4.37 (2H, q, Et), 7.12 (1H, br ddd, C₆H₄), 7.30 (1H, t, C₆H₄), 7.50 (1H, br ddd, C₆H₄), 7.61 (1H, br dd, C₆H₄)

### Intermediate 4: Ethyl 3-(4-azidopiperidin-1-yl)benzoate

Methylene chloride (21 ml) was added to 1.032 g of intermediate 3 to prepare a solution. Mesyl chloride (0.35 ml), 0.69 ml of triethylamine, and 25.3 mg of 4-dimethylaminopyridine were added in that order to the solution, and a reaction was allowed to proceed at room temperature for one hr. 1,3-Diaminopropane (40 mg) was added to stop the reaction, and 30 ml of methylene chloride was then added to dilute the reaction mixture. The methylene chloride layer was washed once with a 5% aqueous potassium hydrogensulfate solution, once with a saturated aqueous sodium hydrogencarbonate solution, and once with saturated brine in that order. The organic layer was dried over anhydrous sodium sulfate and was then concentrated under the reduced pressure to give 1.349 g of crude ethyl 3-{4-(methanesulfonyloxy)piperidin-1-yl}benzoate.
Dimethylformamide (27 ml) was added to this crude product to prepare a solution. Sodium azide (536 mg) was added to the solution, and the mixture was stirred with heating at 90°C for 8 hr. The temperature of the reaction mixture was returned to room temperature, and the reaction mixture was then extracted with 600 ml of ethyl acetate, followed by washing once with 600 ml of water. The aqueous layer was subjected to back extraction with 150 ml of ethyl acetate, and the extact was combined with the first ethyl acetate layer. The organic layer was washed once with a saturated aqueous sodium hydrogencarbonate solution and once with saturated brine in that order. The washed organic layer was dried over anhydrous sodium sulfate and was then concentrated under the reduced pressure. The residue was purified by column chromatography on silica gel (50 g, 1% acetone/chloroform) to give 996 mg of the title compound.
Physicochemical properties of intermediate 4
(1) Color and form: Colorless oil
(2) Molecular formula: C₁₄H₁₈N₄O₂
(3) Mass spectrum (EIMS): m/z 274 M⁺
(4) ¹H NMR spectrum (400 MHz, CDCl₃) δ (ppm): 1.39 (3H, t, Et), 1.79 (2H, m, piperidine), 2.04 (2H, m, piperidine), 3.01 (2H, ddd, piperidine), 3.59 (3H, m, piperidine), 4.37 (2H, q, Et), 7.11 (1H, br ddd, C₆H₄), 7.31 (1H, t, C₆H₄), 7.53 (1H, br ddd, C₆H₄), 7.60 (1H, br dd, C₆H₄)

### Intermediate 5: Ethyl 3-(4-aminopiperidin-1-yl)benzoate

1,4-Dioxane (70 ml), 20 ml of water, and 10 ml of acetic acid were added to 995 mg of intermediate 4 to prepare a solution. To the solution was added 250 mg of 10% palladium-carbon, and the mixture was stirred in a hydrogen atmosphere at room temperature for 16 hr. The insolubles were filtered and were washed twice with 4.0 ml of a solvent having the same composition as the mixed solvent used in the reaction. The filtrate and the washings were combined, followed by concentration under the reduced pressure. The residue was purified by column chromatography on silica gel (50 g, chloroform-methanol-concentrated aqueous ammonia = 10 : 1 : 0.1) to give 716 mg of the title compound.
Physicochemical properties of intermediate 5
(1) Color and form: Colorless syrup
(2) Molecular formula: C₁₄H₂₀N₂O₂
(3) Mass spectrum (FABMS): m/z 249 (M+H)⁺
(4) ¹H NMR spectrum (400 MHz, CDCl₃) δ (ppm): 1.39 (3H, t, Et), 1.50 (2H, m, piperidine), 1.94 and 2.04 (2H, each br d, piperidine), 2.83 (3H, m, piperidine), 3.71 (2H, m, piperidine), 4.36 (2H, q, Et), 7.12 (1H, br dd, C₆H₄), 7.29 (1H, t, C₆H₄), 7.49 (1H, br ddd, C₆H₄), 7.61 (1H, br dd, C₆H₄)

### Intermediate 6: Ethyl 3-{4-(pyrimidin-2-ylamino)-piperidin-1-yl}benzoate

Dimethyl sulfoxide (28 ml) was added to 716 mg of intermediate 5. Next, 839 mg of 2-bromopyrimidine and 3.2 ml of diisopropylethylamine were added thereto in that order, and the mixture was heated at 120°C for 12 hr. The temperature of the reaction mixture was returned to room temperature, and the reaction mixture was then added dropwise to 600 ml of water. The mixture was stirred at room temperature for one hr. The insolubles were collected by filtration and were then washed twice with 20 ml of water. The solid was then dried under the reduced pressure in the presence of diphosphorus pentaoxide at 60°C for 3 hr and was purified by column chromatography on silica gel (50 g, 7.5% acetone/chloroform) to give 531 mg of the title compound. Physicochemical properties of intermediate 6
(1) Color and form: Colorless platy crystal
(2) m.p.: 109 - 110°C
(3) Molecular formula: C₁₈H₂₂N₄O₂
(4) Mass spectrum (TSPMS): m/z 327 (M+H)⁺
(5) ¹H NMR spectrum (400 MHz, CDCl₃) δ (ppm): 1.39 (3H, t, Et), 1.66 (2H, br dq, piperidine), 2.19 (2H, br d, piperidine), 2.99 (2H, m, piperidine), 3.71 (2H, br d, piperidine), 4.02 (1H, m, piperidine), 4.37 (2H, q, Et), 6.54 (1H, t, pyrimidine), 7.13 (1H, br ddd, C₆H₄), 7.31 (1H, t, C₆H₄), 7.52 (1H, br ddd, C₆H₄), 7.63 (1H, br dd, C₆H₄), 8.28 (2H, d, pyrimidine)

### Intermediate 7: 3-{4-(Pyrimidin-2-ylamino)piperidin-1-yl}benzoic acid

Tetrahydrofuran (45 ml), 15 ml of methanol, and 15 ml of a 1 N aqueous sodium hydroxide solution were successively added to 528 mg of intermediate 6 to prepare a solution, and a reaction was allowed to proceed at 45°C for 16 hr. The temperature of the reaction solution was returned to room temperature, and the reaction solution was then concentrated to dryness. The residue was dissolved in 16 ml of water. The solution was adjusted to pH 3 by the addition of 2.5 ml of 5 N hydrochloric acid and 2.0 ml of 1 N hydrochloric acid. The precipitated insolubles were then collected by filtration and were washed twice with 6.0 ml of water. The solid was dried under the reduced pressure in the presence of diphosphorus pentaoxide at 60°C for 3 hr to give 467 mg of the title compound.
Physicochemical properties of intermediate 7
(1) Color and form: Colorless solid
(2) Molecular formula: C₁₆H₁₈N₄O₂
(3) Mass spectrum (TSPMS): m/z 299 (M+H)⁺
(4) ¹H NMR spectrum (400 MHz, DMSO-d₆) δ (ppm): 1.58 (2H, br dq, piperidine), 1.94 (2H, br d, piperidine), 2.85 (2H, br t, piperidine), 3.74 (2H, br d, piperidine), 3.90 (1H, m, piperidine), 6.55 (1H, t, pyrimidine), 7.21 (1H, dt, C₆H₄), 7.31 (1H, t, C₆H₄), 7.33 (1H, m, C₆H₄), 7.47 (1H, br s, C₆H₄), 8.27 (2H, d, pyrimidine)

### Example 4: t-Butyl (2S)-benzenesulfonylamino-3-[3-{4-(pyrimidin-2-ylamino)piperidin-1-yl}benzoylamino]propionate

Dimethylformamide (5.4 ml) and 5.4 ml of methylene chloride were added to 93.2 mg of intermediate 7 and 207 mg of benzotriazol-1-yloxytri(dimethylamino)phosphonium hexafluorophosphate to prepare a solution. Diisopropylethylamine (0.082 ml) was added to the solution, and a reaction was allowed to proceed at room temperature for 2 hr. Separately, 5.4 ml of methylene chloride was added to 113 mg of t-butyl (2S)-N-benzenesulfonyl-2,3-diaminopropionate to prepare a solution. Diisopropylethylamine (0.041 ml) was added to the solution. The above active ester solution was added to this mixture at 0°C, and a reaction was allowed to proceed at room temperature for 16 hr. The reaction solution was concentrated under the reduced pressure, and the residue was extracted with 40 ml of ethyl acetate, followed by washing with a saturated aqueous sodium hydrogencarbonate solution and saturated brine in that order. The extract was then dried over anhydrous sodium sulfate. The ethyl acetate layer was concentrated under the reduced pressure, and the residue was purified by column chromatography on silica gel (25 g, chloroform-methanol-concentrated aqueous ammonia = 30 : 1 : 0.03) to give 181 mg of the title compound.
Physicochemical properties of the compound prepared in Example 4
(1) Color and form: Colorless oil
(2) Molecular formula: C₂₉H₃₆N₆O₅S
(3) Mass spectrum (TSPMS): m/z 581 (M+H)⁺
(4) Specific rotation: [α]_{D}²⁵ +46° (c 0.7, CHCl₃)
(5) ¹H NMR spectrum (400 MHz, CDCl₃) δ (ppm): 1.29 (9H, s, t-Bu), 1.64 (2H, br q, piperidine), 2.17 (2H, m, piperidine), 2.99 (2H, br t, piperidine), 3.60 (1H, ddd, CONHCH₂), 3.73 (1H, br d, piperidine), 3.89 (1H, ddd, CONHCH₂), 3.93 - 4.05 (2H, m, CONHCH₂CH and piperidine), 6.53 (1H, t, pyrimidine), 7.07 (1H, br dd, C₆H₄), 7.15 (1H, br d, C₆H₄), 7.29 (1H, t, C₆H₄), 7.42 (1H, br dd, C₆H₄), 7.49 (2H, m, Ph), 7.57 (1H, m, Ph), 7.86 (2H, m, Ph), 8.29 (2H, d, pyrimidine)

### Example 5: (2S)-Benzenesulfonylamino-3-[3-{4-(pyrimidin-2-ylamino)piperidin-1-yl}benzoylamino]propionic acid

Methylene chloride (4.0 ml) and 0.20 ml of anisole were added to 174 mg of the compound prepared in Example 4 to prepare a solution, and the solution was cooled to 0°C. Trifluoroacetic acid (4.0 ml) was added thereto, and a reaction was allowed to proceed at room temperature for 8 hr. The reaction solution was concentrated under the reduced pressure, and the residue was subjected to azeotropic distillation twice with 4.0 ml of toluene. The product obtained by the azeotropic distillation was then washed twice with 4.0 ml of isopropyl ether, and the residue was purified by column chromatography on silica gel (20 g, chloroform-methanol-concentrated aqueous ammonia = 9 : 2 : 0.2) to give 157 mg of the title compound.
Physicochemical properties of the compound prepared in Example 5
(1) Color and form: Colorless solid
(2) Molecular formula: C₂₅H₂₈N₆O₅S
(3) Mass spectrum (TSPMS): m/z 525 (M+H)⁺
(4) Specific rotation: [α]_{D}²⁵ +60° (c 1.0, MeOH)
(5) ¹H NMR spectrum (400 MHz, CD₃OD) δ (ppm): 1.70 (2H, br q, piperidine), 2.10 (2H, br d, piperidine), 2.93 (2H, br t, piperidine), 3.54 (1H, br dd, CONHCH₂), 3.71 (1H, br dd, CONHCH₂), 3.75 - 3.85 (3H, m, piperidine and CONHCH₂CH), 3.94 (1H, m, piperidine), 6.58 (1H, t, pyrimidine), 7.15 (1H, br d, C₆H₄), 7.23 (1H, br d, C₆H₄), 7.30 (1H, t, C₆H₄), 7.45 (3H, m, 1H of C₆H₄ and 2H of Ph), 7.52 (1H, m, Ph), 7.85 (2H, br d, Ph), 8.26 (2H, d, pyrimidine)

### Example 6: (2S)-Benzenesulfonylamino-3-[3-{4-(1,4,5,6-tetrahydropyrimidin-2-ylamino)piperidin-1-yl}benzoylamino]propionic acid

1,4-Dioxane (10.5 ml), 3.0 ml of water, and 1.5 ml of 1 N hydrochloric acid were successively added to 157 mg of the compound prepared in Example 5 to prepare a solution. To the solution was added 40 mg of 10% palladium-carbon. The mixture was stirred in a hydrogen atmosphere at room temperature for 6 hr. The insolubles were filtered and were washed twice with 4.0 ml of a solvent having the same composition as the mixed solvent used in the reaction. The filtrate and the washings were combined, followed by concentration under the reduced pressure. The residue was purified by preparative thin-layer chromatography on silica gel (development system: chloroform : ethanol : water : concentrated aqueous ammonia = 8 : 8 : 1 : 1) to give the title compound as a crude compound. Finally, the crude compound was purified by Sephadex LH-20 (250 ml, 10% concentrated aqueous ammonia/methanol) to give 119 mg of the title compound.
Physicochemical properties of the compound prepared in Example 6
(1) Color and form: Colorless solid
(2) Molecular formula: C₂₅H₃₂NO₅S
(3) Mass spectrum (TSPMS): m/z 529 (M+H)⁺
(4) Specific rotation: [α]_{D}²⁵ +65° (c 1.0, 10% c. NH₄OH/MeOH)
(5) ¹H NMR spectrum (400 MHz, 10% c. ND₄OD/CD₃OD) δ (ppm): 1.63 (2H, m, piperidine), 1.94 (2H, quintet, tetrahydropyrimidine), 2.00 (2H, br d, piperidine), 2.90 (2H, m, piperidine), 3.35 (4H, t, tetrahydropyrimidine), 3.49 (1H, m, piperidine), 3.52 (1H, dd, CONHCH₂), 3.70 (1H, dd, CONHCH₂), 3.72 (2H, br d, piperidine), 3.78 (1H, dd, CONHCH₂CH), 7.13 (1H, br dd, C₆H₄), 7.24 (1H, br d, C₆H₄), 7.32 (1H, t, C₆H₄), 7.42 (1H, br s, C₆H₄), 7.48 (2H, m, Ph), 7.55 (1H, m, Ph), 7.85 (2H, m, Ph)

### Intermediate 8: Methyl 4-fluoro-3-(4-hydroxypiperidin-1-yl)benzoate

Anhydrous methanol (30 ml) was added to 3.00 g of 3-bromo-4-fluorobenzoic acid to prepare a solution. Concentrated sulfuric acid (3.0 ml) was slowly added to the solution, and the mixture was heated under reflux for 5 hr. The temperature of the reaction solution was returned to room temperature, and the reaction solution was then added dropwise to 600 ml of a saturated aqueous sodium hydrogencarbonate solution. The mixture was extracted once with 600 ml of diethyl ether and once with 120 ml of diethyl ether. The ether layers were combined, and the combined ether layers were dried over anhydrous sodium sulfate and were then concentrated and dried under the reduced pressure to give 3.47 g of crude methyl 3-bromo-4-fluorobenzoate. Toluene (110 ml) was added to a 2.83 g portion of the crude product to prepare a solution which was then added to 1.48 g of 4-hydroxypiperidine. Further, 5.55 g of anhydrous cesium carbonate, 273 mg of palladium(II) acetate, and 758 mg of (R)-(+)-2,2'-bis(diphenylphosphino)-1,1'-binaphthyl were added thereto in that order, and the mixture was stirred with heating at 100°C for 5 hr. The temperature of the reaction mixture was returned to room temperature, and the reaction solution was then added dropwise to 220 ml of a saturated aqueous sodium hydrogencarbonate solution. The mixture was extracted three times with 110 ml of ethyl acetate. The ethyl acetate layers were combined, and anhydrous sodium sulfate was added thereto to perform drying at room temperature for 3 days. The insolubles were filtered, and the filtrate was concentrated under the reduced pressure. The residue was purified by column chromatography on silica gel (120 g, 35% ethyl acetate/hexane) to give 424 mg of the title compound.
Physicochemical properties of intermediate 8
(1) Color and form: Colorless oil
(2) Molecular formula: C₁₃H₁₆NO₃F
(3) Mass spectrum (FABMS): m/z 254 (M+H)⁺
(4) ¹H NMR spectrum (400 MHz, CDCl₃) δ (ppm): 1.77 (2H, m, piperidine), 2.05 (2H, m, piperidine), 2.89 (2H, ddd, piperidine), 3.39 (2H, m, piperidine), 3.87 (1H, m, piperidine), 3.90 (3H, s, Me), 7.06 (1H, dd, C₆H₃), 7.65 (1H, ddd, C₆H₃), 7.67 (1H, dd, C₆H₃)

### Intermediate 9: Methyl 4-fluoro-3-{(4-methanesulfonyloxy)piperidin-1-yl}benzoate

Methylene chloride (9.7 ml) was added to 485 mg of intermediate 8 to prepare a solution. Mesyl chloride (0.16 ml), 0.32 ml of triethylamine, and 11.7 mg of 4-dimethylaminopyridine were added in that order to the solution, and a reaction was allowed to proceed at room temperature for one hr. Methylene chloride (40 ml) was added to dilute the reaction solution. The diluted solution was washed once with a 5% aqueous potassium hydrogensulfate solution, once with a saturated aqueous sodium hydrogencarbonate solution, and once with saturated brine in that order. The organic layer was dried over anhydrous sodium sulfate and was then concentrated under the reduced pressure to give 556 mg of the title compound.
Physicochemical properties of intermediate 9
(1) Color and form: Colorless solid
(2) Molecular formula: C₁₄H₁₈NO₅FS
(3) Mass spectrum (FABMS): m/z 332 (M+H)⁺
(4) ¹H NMR spectrum (400 MHz, CDCl₃) δ (ppm): 2.10 (2H, m, piperidine), 2.19 (2H, m, piperidine), 3.04 (2H, m, piperidine), 3.07 (3H, s, Ms), 3.35 (2H, m, piperidine), 3.90 (3H, s, Me ester), 4.93 (1H, m, piperidine), 7.07 (1H, dd, C₆H₃), 7.67 (2H, m, C₆H₃)

### Intermediate 10: Methyl 3-(4-azidopiperidin-1-yl)-4-fluorobenzoate

Dimethylformamide (11 ml) was added to 554 mg of intermediate 9 to prepare a solution. Sodium azide (217 mg) was added to the solution, and the mixture was stirred with heating at 90°C for 16 hr. The temperature of the reaction mixture was returned to room temperature, and the reaction mixture was then extracted with 240 ml of ethyl acetate, followed by washing once with 240 ml of water. The aqueous layer was subjected to back extraction with 60 ml of ethyl acetate, and the ethyl acetate layer was combined with the first ethyl acetate layer. The organic layer was washed once with a saturated aqueous sodium hydrogencarbonate solution and once with saturated brine in that order, was dried over anhydrous sodium sulfate, and was then concentrated under the reduced pressure. The residue was purified by column chromatography on silica gel (20 g, 1% → 1.5% acetone/chloroform) to give 449 mg of the title compound. Physicochemical properties of intermediate 10
(1) Color and form: Colorless oil
(2) Molecular formula: C₁₃H₁₅N₄O₂F
(3) Mass spectrum (EIMS): m/z 278 M⁺
(4) ¹H NMR spectrum (400 MHz, CDCl₃) δ (ppm): 1.85 (2H, m, piperidine), 2.07 (2H, m, piperidine), 2.92 (2H, ddd, piperidine), 3.38 (2H, m, piperidine), 3.59 (1H, m, piperidine), 3.90 (3H, s, Me), 7.07 (1H, dd, C₆H₃), 7.64 - 7.68 (2H, m, C₆H₃)

### Intermediate 11: Methyl 3-(4-aminopiperidin-1-yl)-4-fluorobenzoate

1,4-Dioxane (31.5 ml), 9.0 ml of water, and 4.5 ml of acetic acid were added to 449 mg of intermediate 10 to prepare a solution. To the solution was added 112 mg of 10% palladium-carbon. The mixture was stirred in a hydrogen atmosphere at room temperature for 4 hr. The insolubles were filtered and were then washed twice with 10 ml of a solvent having the same composition as the mixed solvent used in the reaction, and the filtrate and the washings were combined followed by concentration under the reduced pressure. The residue was subjected to azeotropic distillation twice with 10 ml of toluene and was then purified by column chromatography on silica gel (20 g, chloroform-methanol-concentrated aqueous ammonia = 15 : 1 : 0.07 → 10 : 1 : 0.1) to give 288 mg of the title compound.
Physicochemical properties of intermediate 11
(1) Color and form: Colorless syrup
(2) Molecular formula: C₁₃H₁₇N₂O₂F
(3) Mass spectrum (TSPMS): m/z 253 (M+H)⁺
(4) ¹H NMR spectrum (400 MHz, CDCl₃) δ (ppm): 1.57 (2H, m, piperidine), 1.95 (2H, br d, piperidine), 2.78 (2H, br t, piperidine), 2.83 (1H, m, piperidine), 3.45 (2H, br d, piperidine), 3.90 (3H, s, Me), 7.05 (1H, dd, C₆H₃), 7.63 (1H, ddd, C₆H₃), 7.66 (1H, dd, C₆H₃)

### Intermediate 12: Methyl 4-fluoro-3-{4-(pyrimidin-2-ylamino)piperidin-1-yl}-benzoate

Dimethyl sulfoxide (12 ml) was added to 288 mg of intermediate 11. 2-Bromopyrimidine (272 mg) and 1.0 ml of diisopropylethylamine were then added thereto in that order, and the mixture was heated at 120°C for 12 hr. The temperature of the reaction mixture was returned to room temperature, and the reaction mixture was then added dropwise to 240 ml of water. The mixture was stirred at 0°C for 15 min, followed by extraction with 240 ml of ethyl acetate. The organic layer was washed twice with 240 ml of water, was dried over anhydrous sodium sulfate, and was then concentrated under the reduced pressure. The residue was purified by column chromatography on silica gel (20 g, 10% acetone/chloroform) to give 253 mg of the title compound. Physicochemical properties of intermediate 12
(1) Color and form: Colorless solid
(2) Molecular formula: C₁₇H₁₉N₄O₂F
(3) Mass spectrum (TSPMS): m/z 331 (M+H)⁺
(4) ¹H NMR spectrum (400 MHz, CDCl₃) δ (ppm): 1.74 (2H, m, piperidine), 2.21 (2H, m, piperidine), 2.93 (2H, br t, piperidine), 3.47 (2H, br d, piperidine), 3.90 (3H, s, Me), 4.01 (1H, m, piperidine), 6.54 (1H, t, pyrimidine), 7.06 (1H, dd, C₆H₃), 7.65 (1H, ddd, C₆H₃), 7.68 (1H, dd, C₆H₃), 8.29 (2H, d, pyrimidine)

### Intermediate 13: 4-Fluoro-3-{4-(pyrimidin-2-ylamino)-piperidin-1-yl}benzoic acid

Tetrahydrofuran (15 ml), 5.0 ml of methanol, and 5.0 ml of a 1 N aqueous sodium hydroxide solution were added in that order to 253 mg of intermediate 12, and a reaction was allowed to proceed at 45°C for 12 hr. The temperature of the reaction solution was returned to room temperature, and the reaction solution was then concentrated to dryness. The residue was dissolved in 5.0 ml of water. The solution was adjusted to pH 3 by the addition of 0.8 ml of 5 N hydrochloric acid and 1.1 ml of 1 N hydrochloric acid. The precipitated insolubles were then collected by filtration and were washed twice with 2.0 ml of water. The solid was dried under the reduced pressure in the presence of diphosphorus pentaoxide at 50°C for 2 hr to give 227 mg of the title compound.
Physicochemical properties of intermediate 13
(1) Color and form: Colorless solid
(2) Molecular formula: C₁₆H₁₇N₄O₂F
(3) Mass spectrum (TSPMS): m/z 317 (M+H)⁺
(4) ¹H NMR spectrum (400 MHz, DMSO-d₆) δ (ppm): 1.67 (2H, br dq, piperidine), 1.98 (2H, br d, piperidine), 2.81 (2H, br t, piperidine), 3.39 (2H, br d, piperidine), 3.88 (1H, m, piperidine), 6.56 (1H, t, pyrimidine), 7.24 (1H, dd, C₆H₃), 7.56 (1H, ddd, C₆H₃), 7.59 (1H, dd, C₆H₃), 8.28 (2H, d, pyrimidine)

### Example 7: t-Butyl (2S)-benzenesulfonylamino-3-[4-fluoro-3-{4-(pyrimidin-2-ylamino)piperidin-1-yl}benzoylamino]propionate

Dimethylformamide (1.3 ml) and 1.3 ml of methylene chloride were added to 50.0 mg of intermediate 13 and 105 mg of benzotriazol-1-yloxytri(dimethylamino)phosphonium hexafluorophosphate to prepare a solution. Diisopropylethylamine (0.041 ml) was added to the solution, and a reaction was allowed to proceed at room temperature for 2 hr. Separately, 1.3 ml of methylene chloride was added to 57.0 mg of t-butyl (2S)-N-benzenesulfonyl-2,3-diaminopropionate to prepare a solution, and 0.021 ml of diisopropylethylamine was added to the solution. The above active ester solution was added to this mixture at 0°C, and a reaction was allowed to proceed at room temperature for 2 hr. The reaction solution was concentrated under the reduced pressure, and the residue was extracted with 20 ml of ethyl acetate, followed by washing with a saturated aqueous sodium hydrogencarbonate solution and saturated brine in that order. The extract was then dried over anhydrous sodium sulfate. The ethyl acetate layer was concentrated under the reduced pressure, and the residue was purified by preparative thin-layer chromatography on silica gel (development system: chloroform : methanol : concentrated aqueous ammonia = 20 : 1 : 0.05) to give 93.7 mg of the title compound.
Physicochemical properties of the compound prepared in Example 7
(1) Color and form: Colorless oil
(2) Molecular formula: C₂₉H₃₅N₆O₅FS
(3) Mass spectrum (FABMS): m/z 599 (M+H)⁺
(4) Specific rotation: [α]_{D}²⁵ +50° (c 1.0, CHCl₃)
(5) ¹H NMR spectrum (400 MHz, CDCl₃) δ (ppm): 1.28 (9H, s, t-Bu), 1.71 (2H, m, piperidine), 2.17 (2H, m, piperidine), 2.91 (2H, br t, piperidine), 3.46 (1H, br d, piperidine), 3.59 (1H, ddd, CONHCH₂), 3.89 (1H, ddd, CONHCH₂), 3.97 (2H, m, CONHCH₂CH and piperidine), 6.53 (1H, t, pyrimidine), 7.03 (1H, dd, C₆H₃), 7.32 (1H, ddd, C₆H₃), 7.49 (3H, m, 2H of Ph and 1H of C₆H₃), 7.57 (1H, m, Ph), 7.85 (2H, m, Ph), 8.29 (2H, d, pyrimidine)

### Example 8: (2S)-Benzenesulfonylamino-3-[4-fluoro-3-{4-(pyrimidin-2-ylamino)piperidin-1-yl}benzoylamino]propionic acid

Methylene chloride (2.0 ml) and 0.10 ml of anisole were added to 93.7 mg of the compound prepared in Example 7 to prepare a solution, and the solution was cooled to 0°C. Trifluoroacetic acid (2.0 ml) was added thereto, and a reaction was allowed to proceed at 0°C for 16 hr. The reaction solution was concentrated under the reduced pressure. The residue was subjected to azeotropic distillation twice with 2.0 ml of toluene. The product obtained by the azeotropic distillation was then washed twice with 2.0 ml of isopropyl ether, and the residue was purified by column chromatography on silica gel (5.0 g, chloroform-methanol-concentrated aqueous ammonia = 9 : 2 : 0.1) to give 84.9 mg of the title compound.
Physicochemical properties of the compound prepared in Example 8
(1) Color and form: Colorless solid
(2) Molecular formula: C₂₅H₂₇N₆O₅FS
(3) Mass spectrum (FABMS): m/z 543 (M+H)⁺
(4) Specific rotation: [α]_{D}²⁵ +54° (c 1.2, MeOH)
(5) ¹H NMR spectrum (400 MHz, CD₃OD) δ (ppm): 1.75 (2H, br dq, piperidine), 2.11 (2H, br d, piperidine), 2.89 (2H, br t, piperidine), 3.49 (2H, br d, piperidine), 3.52 (1H, dd, CONHCH₂), 3.72 (1H, dd, CONHCH₂), 3.84 (1H, dd, CONHCH₂CH), 3.93 (1H, m, piperidine), 6.58 (1H, t, pyrimidine), 7.09 (1H, dd, C₆H₃), 7.41 (1H, ddd, C₆H₃), 7.44 (2H, m, Ph), 7.51 (1H, m, Ph), 7.53 (1H, dd, C₆H₃), 7.85 (2H, m, Ph), 8.27 (2H, d, pyrimidine)

### Example 9: (2S)-Benzenesulfonylamino-3-[4-fluoro-3-{4-(1,4,5,6-tetrahydropyrimidin-2-ylamino)piperidin-1-yl}benzoylamino]propionic acid

1,4-Dioxane (5.6 ml), 1.6 ml of water, and 0.8 ml of 1 N hydrochloric acid were successively added to 79.4 mg of the compound prepared in Example 8 to prepare a solution. To the solution was added 20 mg of 10% palladium-carbon. The mixture was stirred in a hydrogen atmosphere at room temperature for 5 hr. The insolubles were filtered and were then washed twice with 2.0 ml of a solvent having the same composition as the mixed solvent used in the reaction, and the filtrate and the washings were combined. 10% palladium-carbon (32 mg) was newly added thereto, and the mixture was stirred in a hydrogen atmosphere at room temperature for additional 8 hr. The insolubles were filtered and were then washed twice with 2.0 ml of a solvent having the same composition as the mixed solvent used in the reaction, and the filtrate and the washings were combined, followed by concentration under the reduced pressure. The residue was purified by preparative thin-layer chromatography on silica gel (development system: chloroform : ethanol : water : concentrated aqueous ammonia = 8 : 8 : 1 : 1) to give the title compound as a crude compound and was finally purified by Sephadex LH-20 (60 ml, 10% concentrated aqueous ammonia/methanol) to give 51.6 mg of the title compound.
Physicochemical properties of the compound prepared in Example 9
(1) Color and form: Colorless solid
(2) Molecular formula: C₂₅H₃₁N₆O₅FS
(3) Mass spectrum (FABMS): m/z 547 (M+H)⁺
(4) Specific rotation: [α]_{D}²⁵ +63° (c 1.0, 10% c. NH₄OH/MeOH)
(5) ¹H NMR spectrum (400 MHz, 10% c. ND₄OD/CD₃OD) δ (ppm): 1.64 (2H, m, piperidine), 1.89 (2H, quintet, tetrahydropyrimidine), 1.97 (2H, br d, piperidine), 2.81 (2H, br t, piperidine), 3.30 (4H, t, tetrahydropyrimidine), 3.38 (2H, br d, piperidine), 3.46 (1H, dd, CONHCH₂), 3.67 (1H, dd, CONHCH₂), 3.75 (1H, dd, CONHCH₂CH), 7.07 (1H, dd, C₆H₃), 7.38 (1H, ddd, C₆H₃), 7.42 (2H, m, Ph), 7.48 (2H, m, Ph and C₆H₃), 7.80 (2H, m, Ph)

### Intermediate 14: Ethyl 3-{(3S,4R)-3,4,5-(trihydroxy)pentan-1-ylamino}benzoate

Methanol (50 ml) was added to 1.34 g of 2-deoxy-D-ribose to prepare a solution. Separately, 50 ml of methylene chloride was added to 1.60 g of ethyl 3-aminobenzoate to prepare a solution which was then added to the above methanol solution. A reaction was allowed to proceed at room temperature for 16 hr. Acetic acid (1.0 ml) and 500 mg of sodium boron cyanohydride were then added thereto in that order, and a reaction was allowed to proceed at room temperature for 4 hr. The reaction solution was concentrated under the reduced pressure, and the residue was extracted with 300 ml of chloroform. The organic layer was washed with 200 ml of a saturated aqueous sodium hydrogencarbonate solution containing a minor amount of sodium chloride. The aqueous layer was subjected to back extraction with 100 ml of chloroform. The chloroform layers were combined and were then dried over anhydrous sodium sulfate, followed by concentration under the reduced pressure. The residue was purified by column chromatography on silica gel (100 g, chloroform-methanol-concentrated aqueous ammonia = 10 : 1 : 0.1 → 10 : 1.3 : 0.1) to give 2.23 g of the title compound.
Physicochemical properties of intermediate 14
(1) Color and form: Colorless solid
(2) Molecular formula: C₁₄H₂₁NO₅
(3) Mass spectrum (TSPMS): m/z 284 (M+H)⁺
(4) Specific rotation: [α]_{D}²⁵ -17° (c 1.0, CHCl₃)
(5) ¹H NMR spectrum (400 MHz, CDCl₃) δ (ppm): 1.36 (3H, t, Et), 1.80 (2H, m, NHCH₂CH₂), 3.32 (2H, m, NHCH₂), 3.62 (1H, br s, CHOH), 3.77 (2H, br s, CH₂OH), 3.89 (1H, br s, CHOH), 4.33 (2H, q, Et), 6.78 (1H, br dd, C₆H₄), 7.20 (1H, t, C₆H₄), 7.29 (1H, br s, C₆H₄), 7.37 (1H, br d, C₆H₄)

### Intermediate 15: Ethyl 3-{(3R,4S)-3,4-(dihydroxy)piperidin-1-yl}benzoate

Tetrahydrofuran (15 ml) was added to 372 mg of intermediate 14 to prepare a solution. Carbon tetrabromide (653 mg) was added to the solution. The mixture was cooled to 0°C, and 689 mg of triphenylphosphine was then added thereto. The temperature of the mixture was gradually raised to room temperature over a period of one hr. The reaction solution was concentrated under the reduced pressure, and the residue was purified by column chromatography on silica gel (40 g, chloroform-methanol-concentrated aqueous ammonia = 20 : 1 : 0.05) to give the title compound as a crude compound. The crude compound was purified by preparative thin-layer chromatography on silica gel (development system: chloroform : methanol : benzene : ethyl acetate = 9 : 1 : 6 : 4) to give 157 mg of the title compound.
Physicochemical properties of intermediate 15
(1) Color and form: Colorless syrup
(2) Molecular formula: C₁₄H₁₉NO₄
(3) Mass spectrum (FABMS): m/z 266 (M+H)⁺
(4) Specific rotation: [α]_{D}²⁵ +3° (c 1.0, CHCl₃)
(5) ¹H NMR spectrum (400 MHz, CDCl₃) δ (ppm): 1.39 (3H, t, Et), 1.94 (2H, m, NCH₂CH₂), 2.99 (1H, m, NCH₂CH₂), 3.16 (1H, dd, NCH₂CHOH), 3.42 (1H, m, NCH₂CH₂), 3.51 (1H, ddd, NCH₂CHOH), 3.84 (1H, br s, CHOH), 3.95 (1H, br s, CHOH), 4.37 (2H, q, Et), 7.14 (1H, br ddd, C₆H₄), 7.32 (1H, t, C₆H₄), 7.56 (1H, br ddd, C₆H₄), 7.63 (1H, br dd, C₆H₄)

### Intermediate 16: Ethyl 3-{(3R)-acetoxy-(4S)-hydroxypiperidin-1-yl}benzoate

### Intermediate 17: Ethyl 3-{(4S)-acetoxy-(3R)-hydroxypiperidin-1-yl}benzoate

Trimethyl orthoacetate (0.50 ml) was added to intermediate 15 (134 mg, 0.51 mmol) to prepare a suspension. p-Toluenesulfonic acid monohydrate (15.4 mg) was added to the suspension at room temperature, and a reaction was allowed to proceed for 3 hr. The reaction solution was concentrated under the reduced pressure. Acetic acid (1.0 ml) was then added to the residue at room temperature, and a reaction was allowed to proceed for 45 min. Water (100 ml) was then added thereto. The mixture was extracted twice with 100 ml of ethyl acetate. The organic layers were combined, and the combined organic layers were washed with 100 ml of saturated brine, were dried over anhydrous magnesium sulfate, and were then concentrated under the reduced pressure. The residue was purified by column chromatography on silica gel (development system: methylene chloride : methanol : benzene : ethyl acetate = 9 : 1 : 6 : 4) to give intermediate 16 (47 mg, 30%) and intermediate 17 (86 mg, 55%).
Physicochemical properties of intermediate 16
(1) Color and form: Colorless syrup
(2) Molecular formula: C₁₆H₂₁NO₅
(3) Mass spectrum (EIMS): m/z 307 (M)⁺
(4) Specific rotation: [α]_{D}²⁵ -25° (c 1.1, CH₂Cl₂)
(5) ¹H NMR spectrum (400 MHz, CDCl₃) δ (ppm): 1.39 (3H, t, Et), 1.90 - 2.04 (2H, m, piperidine), 2.10 (3H, s, acetyl), 3.20 - 3.28 (1H, m, piperidine), 3.88 (1H, dd, piperidine), 3.44 (1H, ddd, piperidine), 3.52 (1H, dd, piperidine), 4.07 (1H, dddd, piperidine), 4.37 (2H, q, Et), 5.04 (1H, ddd, piperidine), 7.12 (1H, ddd, C₆H₄), 7.30 (1H, dd, C₆H₄), 7.51 (1H, ddd, C₆H₄), 7.60 (1H, dd, C₆H₄)
Physicochemical properties of intermediate 17
(1) Color and form: Colorless syrup
(2) Molecular formula: C₁₆H₂₁NO₅
(3) Mass spectrum (EIMS): m/z 307 (M)⁺
(4) Specific rotation: [α]_{D}²⁵ +4.9° (c 1.1, CH₂Cl₂)
(5) ¹H NMR spectrum (400 MHz, CDCl₃) δ (ppm): 1.39 (3H, t, Et), 1.94 - 1.99 (1H, m, piperidine), 2.07 - 2.16 (1H, m, piperidine), 2.15 (3H, s, acetyl), 3.07 (1H, ddd, piperidine), 3.23 (1H, dd, piperidine), 3.43 (1H, m, piperidine), 3.50 (1H, ddd, piperidine), 4.08 (1H, br s, piperidine), 4.38 (2H, q, Et), 5.00 (1H, ddd, piperidine), 7.16 (1H, dd, C₆H₄), 7.33 (1H, dd, C₆H₄), 7.58 (1H, m, C₆H₄), 7.64 (1H, m, C₆H₄)

### Intermediate 18: Ethyl 3-{(3R)-acetoxy-(4S)-methanesulfonyloxypiperidin-1-yl}benzoate

Methylene chloride (3.0 ml) was added to intermediate 16 (47 mg, 0.15 mmol) to prepare a solution. Triethylamine (45 µl, 0.32 mmol) and methanesulfonyl chloride (15 µl, 0.20 mmol) were added to the solution at room temperature, and a reaction was allowed to proceed for 5 min. Water (100 ml) was added thereto, and the mixture was extracted twice with 50 ml of methylene chloride. The combined organic layers were dried over anhydrous magnesium sulfate and were concentrated under the reduced pressure to give the title compound (40 mg, 67%).
Physicochemical properties of intermediate 18
(1) Color and form: Light yellow syrup
(2) Molecular formula: C₁₇H₂₃NO₇S
(3) Mass spectrum (EIMS): m/z 385 (M)⁺
(4) ¹H NMR spectrum (400 MHz, CDCl₃) δ (ppm): 1.40 (3H, t, Et), 2.10 (3H, s, acetyl), 2.08 - 2.16 (1H, m, piperidine), 2.20 - 2.30 (1H, m, piperidine), 3.09 (3H, s, Ms), 3.30 - 3.46 (3H, m, piperidine), 3.50 - 3.56 (1H, m, piperidine), 4.38 (2H, q, Et), 5.08 - 5.15 (2H, m, piperidine), 7.13 (1H, dd, C₆H₄), 7.33 (1H, dd, C₆H₄), 7.56 (1H, br d, C₆H₄), 7.60 - 7.62 (1H, m, C₆H₄)

### Intermediate 19: Ethyl 3-{(3R)-acetoxy-(4R)-azidopiperidin-1-yl}benzoate

Dimethylformamide (2.0 ml) was added to intermediate 18 (39 mg, 0.10 mmol) to prepare a solution. Sodium azide (15 mg, 0.23 mmol) was added to the solution, and a reaction was allowed to proceed at 90°C for 10 hr. The reaction mixture was returned to room temperature, 100 ml of water was then added thereto, and the mixture was extracted twice with 70 ml of ethyl acetate. The combined organic layers were washed twice with 100 ml of water and once with 100 ml of saturated brine, were then dried over anhydrous magnesium sulfate, and were concentrated under the reduced pressure. The residue was then purified by column chromatography on silica gel (development system: hexane : ethyl acetate = 1 : 1) to give the title compound (34 mg, 100%).
Physicochemical properties of intermediate 19
(1) Color and form: Colorless syrup
(2) Molecular formula: C₁₆H₂₀N₄O₄
(3) Mass spectrum (EIMS): m/z 332 (M)⁺
(4) Specific rotation: [α]_{D}²⁵ -10° (c 1.7, CH₂Cl₂)
(5) ¹H NMR spectrum (400 MHz, CDCl₃) δ (ppm): 1.39 (3H, t, Et), 1.76 (1H, dddd, piperidine), 2.08 - 2.13 (1H, m, piperidine), 2.14 (3H, s, acetyl), 2.80 (1H, dd, piperidine), 2.93 (1H, ddd, piperidine), 3.60 (1H, ddd, piperidine), 3.69 (1H, dddd, piperidine), 3.89 (1H, ddd, piperidine), 4.37 (2H, q, Et), 4.90 (1H, ddd, piperidine), 7.12 (1H, dd, C₆H₄), 7.32 (1H, dd, C₆H₄), 7.55 (1H, br d, C₆H₄), 7.57 - 7.60 (1H, m, C₆H₄)

### Intermediate 20: Ethyl 3-{(4R)-azido-(3R)-hydroxy}piperidin-1-yl}benzoate

Tetrahydrofuran (11 ml) was added to intermediate 19 (390 mg, 1.2 mmol) to prepare a solution. Sodium ethoxide (99 mg, 1.4 mmol) was added to the solution, and a reaction was allowed to proceed at 30°C for 3.5 hr. The reaction solution was adjusted to pH 4 by the addition of 1.0 M hydrochloric acid, and 100 ml of water was added thereto. The mixture was extracted twice with 150 ml of ethyl acetate. The combined organic layers were then washed with 150 ml of saturated brine, were dried over anhydrous magnesium sulfate, and were concentrated under the reduced pressure to give the title compound (346 mg, 100%).
Physicochemical properties of intermediate 20
(1) Color and form: Light yellow syrup
(2) Molecular formula: C₁₄H₁₈N₄O₃
(3) Mass spectrum (EIMS): m/z 290 (M)⁺
(4) ¹H NMR spectrum (400 MHz, CDCl₃) δ (ppm): 1.39 (3H, t, Et), 1.79 (1H, dddd, piperidine), 2.15 (1H, dddd, piperidine), 2.84 (1H, ddd, piperidine), 2.94 (1H, ddd, piperidine), 3.45 (1H, ddd, piperidine), 3.60 (1H, dddd, piperidine), 3.72 (1H, dd, piperidine), 3.76 (1H, ddd, piperidine), 4.37 (2H, q, Et), 7.11 (1H, dd, C₆H₄), 7.32 (1H, dd, C₆H₄), 7.56 (1H, ddd, C₆H₄), 7.60 (1H, dd, C₆H₄)

### Intermediate 21: Ethyl 3-{(4R)-amino-(3R)-hydroxy-piperidin-1-yl}benzoate

1,4-Dioxane (1.0 ml) and 0.5 ml of water were successively added to intermediate 20 (11 mg, 0.039 mmol) to prepare a solution. 10% palladium-carbon (3.0 mg) was added to the solution, and the mixture was stirred in a hydrogen atmosphere at room temperature for 3 hr. The insolubles were filtered and were washed with 20 ml of a solvent having the same composition as the mixed solvent used in the reaction. The filtrate and the washings were combined, followed by concentration under the reduced pressure to give the title compound (11 mg, 100%).
Physicochemical properties of intermediate 21
(1) Color and form: Colorless syrup
(2) Molecular formula: C₁₄H₂₀N₂O₃
(3) Mass spectrum (FABMS): m/z 265 (M+H)⁺
(4) ¹H NMR spectrum (400 MHz, CDCl₃) δ (ppm): 1.39 (3H, t, Et), 1.50 - 1.62 (1H, m, piperidine), 1.93 - 2.01 (1H, m, piperidine), 2.57 - 2.70 (2H, m, piperidine), 2.83 (1H, ddd, piperidine), 3.44 (1H, ddd, piperidine), 3.65 - 3.72 (1H, m, piperidine), 3.86 - 3.93 (1H, m, piperidine), 4.37 (2H, q, Et), 7.11 (1H, dd, C₆H₄), 7.32 (1H, dd, C₆H₄), 7.51 (1H, ddd, C₆H₄), 7.60 (1H, dd, C₆H₄)

### Intermediate 22: Ethyl 3-{(3R)-hydroxy-(4R)-(pyrimidin-2-yl)piperidin-1-ylamino}benzoate

Dimethyl sulfoxide (3.0 ml) was added to intermediate 21 (87 mg, 0.33 mmol) to prepare a solution. 2-Bromopyrimidine (55 mg, 0.33 mmol) and diisopropylethylamine (320 µl, 1.85 mmol) were successively added to the solution, and a reaction was allowed to proceed at 120°C for 14 hr. The reaction mixture was returned to room temperature, and 500 ml of water was added thereto, followed by extraction three times with 250 ml of ethyl acetate. The combined organic layers were washed twice with 200 ml of water and twice with 200 ml of saturated brine, were then dried over anhydrous magnesium sulfate, and were concentrated under the reduced pressure. The residue was purified by column chromatography on silica gel (development system: benzene : ethyl acetate = 1 : 4) to give the title compound (51 mg, 45%).
Physicochemical properties of intermediate 22
(1) Color and form: Colorless solid
(2) Molecular formula: C₁₈H₂₂N₄O₃
(3) Mass spectrum (TSPMS): m/z 343 (M+H)⁺
(4) ¹H NMR spectrum (400 MHz, CDCl₃) δ (ppm): 1.39 (3H, t, Et), 1.80 (1H, dddd, piperidine), 2.14 (1H, dddd, piperidine), 2.74 (1H, dd, piperidine), 2.89 (1H, ddd, piperidine), 3.72 - 3.86 (3H, m, piperidine), 3.97 (1H, ddd, piperidine), 4.37 (2H, q, Et), 6.64 (1H, t, pyrimidine), 7.14 (1H, dd, C₆H₄), 7.32 (1H, dd, C₆H₄), 7.53 (1H, ddd, C₆H₄), 7.63 (1H, dd, C₆H₄), 8.29 (2H, d, pyrimidine)

### Example 10: t-Butyl (2S)-benzenesulfonylamino-3-[3-{(3R)-hydroxy-(4R)-(pyrimidin-2-ylamino)piperidin-1-yl}benzoylamino]propionate

Tetrahydrofuran (1.8 ml) and 0.60 ml of methanol were successively added to intermediate 22 (49 mg, 0.14 mmol) to prepare a solution, and a 1.0 M aqueous sodium hydroxide solution (0.60 ml) was added to the solution. A reaction was allowed to proceed at 50°C for one hr. The temperature of the reaction mixture was then returned to room temperature, and the reaction mixture was adjusted to pH 4 by the addition of 1.0 M hydrochloric acid and was concentrated under the reduced pressure to give 3-{(3R)-hydroxy-(4R)-(pyrimidin-2-ylamino)piperidin-1-yl}benzoic acid. Dimethylformamide (7.0 ml) was added to this product to prepare a solution. Benzotriazol-1-yloxytri(dimethylamino)phosphonium hexafluorophosphate (98 mg, 0.21 mmol) and diisopropylethylamine (40 µl, 0.22 mmol) were added to the solution, and a reaction was allowed to proceed at room temperature for 30 min. Further, t-butyl (2S)-N-benzenesulfonyl-2,3-diaminopropionate (55 mg, 0.18 mmol) was added to the above active ester solution at room temperature, and a reaction was allowed to proceed at room temperature for one hr. A saturated aqueous sodium hydrogencarbonate solution (20 ml) was added thereto, followed by extraction twice with 100 ml of ethyl acetate. The combined organic layers were washed with 100 ml of saturated brine, were dried over anhydrous magnesium sulfate, and were then concentrated under the reduced pressure. The residue was purified by column chromatography on silica gel (development system: methylene chloride : methanol = 20 : 1) to give the title compound (18 mg, 21%).
Physicochemical properties of the compound prepared in Example 10
(1) Color and form: Light yellow solid
(2) Molecular formula: C₂₉H₃₆N₆O₆S
(3) Mass spectrum (TSPMS): m/z 597 (M+H)⁺
(4) Specific rotation: [α]_{D}²⁵ +75° (c 0.48, CH₂Cl₂)
(5) ¹H NMR spectrum (400 MHz, CDCl₃) δ (ppm): 1.29 (9H, s, t-Bu), 1.72 - 1.83 (1H, m, piperidine), 2.09 - 2.15 (1H, m, piperidine), 2.75 (1H, dd, piperidine), 2.90 (1H, ddd, piperidine), 3.54 - 3.63 (1H, m, piperidine), 3.70 - 3.83 (3H, m, piperidine and CONHCH₂CH), 3.86 - 3.95 (2H, m, piperidine and CONHCH₂), 3.98 (1H, ddd, piperidine), 6.62 (1H, t, pyrimidine), 7.10 (1H, dd, C₆H₄), 7.18 (1H, br d, C₆H₄), 7.31 (1H, dd, C₆H₄), 7.43 (1H, dd, C₆H₄), 7.47 - 7.60 (3H, m, C₆H₅), 7.84 - 7.88 (2H, m, C₆H₅), 8.29 (2H, d, pyrimidine)

### Example 11: (2S)-Benzenesulfonylamino-3-[3-{(3R)-hydroxy-(4R)-(pyrimidin-2-ylamino)piperidin-1-yl}benzoylamino]propionic acid

Methylene chloride (2.0 ml) was added to the compound (16 mg, 0.027 mmol) prepared in Example 10 to prepare a solution, and 2.0 ml of trifluoroacetic acid was added to the solution at room temperature. A reaction was allowed to proceed for 7 hr, and the reaction solution was then concentrated under the reduced pressure to give the title compound (18 mg, 75% (as tritrifluoroacetate)).
Physicochemical properties of the compound prepared in Example 11 (as tritrifluoroacetate)
(1) Color and form: Light yellow solid
(2) Molecular formula: C₂₅H₂₈N₆O₆S
(3) Mass spectrum (TSPMS): m/z 541 (M+H)⁺
(4) Specific rotation: [α]_{D}²⁵ +26° (c 0.50, CH₃OH)
(5) ¹H NMR spectrum (400 MHz, CD₃OD) δ (ppm): 1.76 (1H, dddd, piperidine), 2.15 - 2.22 (1H, m, piperidine), 2.76 (1H, dd, piperidine), 2.91 (1H, ddd, piperidine), 3.50 (1H, dd, CONHCH₂), 3.73 (1H, dd, CONHCH₂), 3.72 - 3.83 (2H, m, piperidine), 3.86 - 3.96 (2H, m, piperidine), 4.21 (1H, dd, CONHCH₂CH), 6.77 (1H, t, pyrimidine), 7.18 (1H, dd, C₆H₄), 7.21 (1H, d, C₆H₄), 7.32 (1H, dd, C₆H₄), 7.40 - 7.47 (3H, m, C₆H₄ and C₆H₅), 7.48 - 7.54 (1H, m, C₆H₅), 7.81 - 7.86 (2H, m, C₆H₅), 8.39 (2H, d, pyrimidine)

### Example 12: (2S)-Benzenesulfonylamino-3-[3-{(3R)-hydroxy-(4R)-(1,4,5,6-tetrahydropyrimidin-2-ylamino)piperidin-1-yl}benzoylamino]propionic acid

1,4-Dioxane (2.0 ml) and 1.0 ml of water were successively added to 15 mg of the compound prepared in Example 11 to prepare a solution. 10% Palladium-carbon (4.4 mg) was added to the solution, and a reaction was allowed to proceed at room temperature in a hydrogen atmosphere for 4 hr. The insolubles were filtered and were washed with 90 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (3.1 mg, 28%).
Physicochemical properties of the compound prepared in Example 12
(1) Color and form: Colorless solid
(2) Molecular formula: C₂₅H₃₂N₆O₆S
(3) Mass spectrum (TSPMS): m/z 545 (M+H)⁺
(4) Specific rotation: [α]_{D}²⁵ +70° (c 0.14, CH₃OH)
(5) ¹H NMR spectrum (400 MHz, CD₃OD) δ (ppm): 1.68 (1H, dddd, piperidine), 1.95 (2H, dddd, tetrahydropyrimidine), 1.92 - 2.02 (1H, m, piperidine), 2.67 (1H, dd, piperidine), 2.83 (1H, ddd, piperidine), 3.26 - 3.32 (1H, m, piperidine), 3.36 (4H, br t, tetrahydropyrimidine), 3.50 - 3.57 (1H, m, piperidine), 3.56 (1H, dd, CONHCH₂), 3.67 (1H, dd, CONHCH₂), 3.74 (1H, dd, CONHCH₂CH), 3.77 - 3.85 (1H, m, piperidine), 3.91 (1H, m, piperidine), 7.11 (1H, ddd, C₆H₄), 7.24 (1H, ddd, C₆H₄), 7.31 (1H, dd, C₆H₄), 7.42 (1H, dd, C₆H₄), 7.46 - 7.52 (2H, m, C₆H₅), 7.52 - 7.58 (1H, m, C₆H₅), 7.85 - 7.89 (2H, m, C₆H₅)

### Intermediate 23: Ethyl 3-{(4R)-azido-(3R)-methoxypiperidin-1-yl}benzoate

Tetrahydrofuran (5.0 ml) was added to sodium hydride (60%, 35 mg, 0.87 mmol) in an argon atmosphere to prepare a suspension. Separately, intermediate 20 (208 mg, 0.72 mmol) was dissolved in 2.0 ml of tetrahydrofuran. This solution was added dropwise to the above suspension at room temperature, and the mixture was stirred for 30 min. A solution (1.0 ml) of methyl iodide (67 µl, 1.1 mmol) in tetrahydrofuran was added dropwise thereto. The mixture was stirred for 4 hr, a saturated aqueous ammonium chloride solution was then added to stop the reaction, and 100 ml of water was added thereto. The mixture was extracted twice with 100 ml of ethyl acetate. The combined organic layers were then dried over anhydrous magnesium sulfate and were concentrated under the reduced pressure. The residue was purified by column chromatography on silica gel (development system: methylene chloride : methanol = 10 : 1) to give the title compound (77 mg, 35%).
Physicochemical properties of intermediate 23
(1) Color and form: Yellow oil
(2) Molecular formula: C₁₅H₂₀N₄O₃
(3) Mass spectrum (TSPMS): m/z 305 (M+H)⁺
(4) ¹H NMR spectrum (400 MHz, CDCl₃) δ (ppm): 1.39 (3H, t, Et), 1.70 (1H, m, piperidine), 2.06 (1H, m, piperidine), 2.68 (1H, dd, piperidine), 2.84 (1H, ddd, piperidine), 3.34 (1H, ddd, piperidine), 3.44 (1H, ddd, piperidine), 3.55 (3H, s, OMe), 3.63 (1H, br d, piperidine), 3.88 (1H, ddd, piperidine), 4.37 (2H, q, Et), 7.12 (1H, d, C₆H₄), 7.32 (1H, dd, C₆H₄), 7.55 (1H, d, C₆H₄), 7.60 (1H, br s, C₆H₄)

### Intermediate 24: 3-{(3R)-Methoxy-(4R)-(pyrimidin-2-ylamino)piperidin-1-yl}benzoic acid

1,4-Dioxane (4.0 ml) and 2.0 ml of water were successively added to intermediate 23 (69 mg, 0.23 mmol) to prepare a solution. 10% Palladium-carbon (18 mg) was added to the solution, and the mixture was stirred in a hydrogen atmosphere at room temperature for 4 hr. The insolubles were filtered and were washed with 90 ml of a solvent having the same composition as the mixed solvent used in the reaction. The filtrate and the washings were combined followed by concentration under the reduced pressure to give ethyl 3-{(4R)-amino-(3R)-methoxypiperidin-1-yl}benzoate (66 mg, 100%).

Dimethyl sulfoxide (2.5 ml) was added to the ethyl 3-{(4R)-amino-(3R)-methoxypiperidin-1-yl}benzoate (66 mg, 0.23 mmol) thus obtained to prepare a solution. Diisopropylethylamine (230 µl, 1.3 mmol) and 2-bromopyrimidine (42 mg, 0.27 mmol) were added in that order to the solution. A reaction was allowed to proceed at 120°C for 24 hr, and the temperature of the reaction mixture was then returned to room temperature. Water (500 ml) was added thereto, and the mixture was extracted twice with 500 ml of ethyl acetate. The combined organic layers were washed twice with 500 ml of water and once with 500 ml of saturated brine, were dried over anhydrous magnesium sulfate, and were then concentrated under the reduced pressure. The residue was purified by column chromatography on silica gel (development system: ethyl acetate) to give ethyl 3-{(3R)-methoxy-(4R)-(pyrimidin-2-ylamino)piperidin-1-yl}benzoate (34 mg, 40%).

Tetrahydrofuran (1.5 ml) and 0.50 ml of methanol were added in that order to the ethyl 3-{(3R)-methoxy-(4R)-(pyrimidin-2-ylamino)piperidin-1-yl}benzoate (34 mg, 0.096 mmol) thus obtained to prepare a solution, and 0.50 ml of a 1.0 M aqueous sodium hydroxide solution was added to the solution. A reaction was allowed to proceed at 50°C for 3 hr. The temperature of the reaction mixture was then returned to room temperature, the reaction mixture was adjusted to pH 3 by the addition of 1.0 M hydrochloric acid, and 50 ml of water was added thereto. The mixture was extracted twice with 50 ml of ethyl acetate, and the extract was then dried over anhydrous magnesium sulfate and was concentrated under the reduced pressure to give the title compound (29 mg, 95%).
Physicochemical properties of intermediate 24
(1) Color and form: Colorless solid
(2) Molecular formula: C₁₇H₂₀N₄O₃
(3) Mass spectrum (EIMS): m/z 328 (M)⁺
(4) ¹H NMR spectrum (400 MHz, CD₃OD) δ (ppm): 1.62 - 1.77 (1H, m, piperidine), 2.14 - 2.25 (1H, m, piperidine), 2.68 - 2.79 (1H, m, piperidine), 2.88 - 2.99 (1H, m, piperidine), 3.42 - 3.53 (1H, m, piperidine), 3.48 (3H, s, OMe), 3.62 - 3.73 (1H, m, piperidine), 3.95 - 4.05 (2H, m, piperidine), 6.59 (1H, t, pyrimidine), 7.24 (1H, d, C₆H₄), 7.33 (1H, dd, C₆H₄), 7.49 (1H, d, C₆H₄), 7.64 (1H, br s, C₆H₄), 8.26 (2H, d, pyrimidine)

### Example 13: t-Butyl (2S)-benzenesulfonylamino-3-[3-{(3R)-methoxy-(4R)-(pyrimidin-2-ylamino)piperidin-1-yl}benzoylamino]propionate

Dimethylformamide (1.5 ml) was added to intermediate 24 (28 mg, 0.086 mmol) to prepare a solution, and t-butyl (2S)-N-benzenesulfonyl-2,3-diaminopropionate (32 mg, 0.10 mmol) was added to the solution. Further, 1-hydroxybenzotriazole (19 mg, 0.14 mmol), N-methylmorpholine (47 µl, 0.43 mmol), and 1-ethyl-3-(3-dimethylaminopropyl)carbodiimide hydrochloride (34 mg, 0.18 mmol) were added thereto, and a reaction was allowed to proceed at room temperature for 2.5 hr. A saturated aqueous sodium hydrogencarbonate solution (10 ml) was added to stop the reaction, and 100 ml of water was added thereto. The mixture was extracted twice with 100 ml of ethyl acetate, and the combined organic layers were washed with 100 ml of saturated brine and were dried over anhydrous magnesium sulfate, followed by concentration under the reduced pressure. The residue was purified by column chromatography on silica gel (development system: methylene chloride : methanol = 10 : 1) to give the title compound (36 mg, 69%).
Physicochemical properties of the compound prepared in Example 13
(1) Color and form: Colorless solid
(2) Molecular formula: C₃₀H₃₈N₆O₆S
(3) Mass spectrum (TSPMS): m/z 611 (M+H)⁺
(4) Specific rotation: [α]_{D}²⁵ +17° (c 0.54, CH₂Cl₂)
(5) ¹H NMR spectrum (400 MHz, CDCl₃) δ (ppm): 1.29 (9H, s, t-Bu), 1.65 - 1.70 (1H, m, piperidine), 2.41 (1H, m, piperidine), 2.85 (1H, dd, piperidine), 3.01 (1H, ddd, piperidine), 3.37 (1H, ddd, piperidine), 3.49 (3H, s, OMe), 3.49 - 3.58 (1H, m, CONHCH₂), 3.60 - 3.68 (1H, m, piperidine), 3.87 - 4.03 (4H, m, piperidine and CONHCH₂CH), 6.65 (1H, t, pyrimidine), 7.09 (1H, d, C₆H₄), 7.21 (1H, d, C₆H₄), 7.32 (1H, dd, C₆H₄), 7.46 (1H, br s, C₆H₄), 7.48 - 7.61 (3H, m, C₆H₅), 7.84 - 7.88 (2H, m, C₆H₅), 8.29 (2H, d, pyrimidine)

### Example 14: (2S)-Benzenesulfonylamino-3-[{(3R)-methoxy-(4R)-(pyrimidin-2-ylamino)piperidin-1-yl}benzoylamino]-propionic acid

Methylene chloride (1.0 ml) was added to the compound (36 mg, 0.059 mmol) prepared in Example 13 to prepare a solution, and 1.0 ml of trifluoroacetic acid was added to the solution at room temperature. The mixture was stirred for 2 hr and was concentrated under the reduced pressure to give the title compound (37 mg, 71% (as tritrifluoroacetate)).
Physicochemical properties of the compound prepared in Example 14 (as tritrifluoroacetate)
(1) Color and form: Light yellow solid
(2) Molecular formula: C₂₆H₃₀N₆O₆S
(3) Mass spectrum (TSPMS): m/z 555 (M+H)⁺
(4) Specific rotation: [α]_{D}²⁵ +35° (c 0.52, CH₃OH)
(5) ¹H NMR spectrum (400 MHz, CD₃OD) δ (ppm): 1.84 (1H, dddd, piperidine), 2.18 (1H, dddd, piperidine), 2.73 (1H, dd, piperidine), 2.93 (1H, ddd, piperidine), 3.43 - 3.51 (2H, m, piperidine and CONHCH₂), 3.47 (3H, s, OMe), 3.76 (1H, dd, CONHCH₂), 3.80 (1H, br d, piperidine), 3.95 (1H, ddd, piperidine), 4.16 (1H, dd, piperidine), 4.21 (1H, dd, CONHCH₂CH), 6.87 (1H, t, pyrimidine), 7.22 (1H, dd, C₆H₄), 7.25 (1H, d, C₆H₄), 7.34 (1H, dd, C₆H₄), 7.42 - 7.48 (3H, m, C₆H₄ and C₆H₅), 7.50 - 7.55 (1H, m, C₆H₅), 7.81 - 7.86 (2H, m, C₆H₅), 8.47 (2H, d, pyrimidine)

### Example 15: (2S)-Benzenesulfonylamino-3-[3-{(3R)-methoxy-(4R)-(1,4,5,6-tetrahydropyrimidin-2-ylamino)-piperidin-1-yl}benzoylamino]propionic acid

1,4-Dioxane (2.0 ml) and 1.0 ml of water were added in that order to 32 mg of the compound prepared in Example 14 to prepare a solution. 10% Palladium-carbon (8.3 mg) was added to the solution, and the mixture was stirred in a hydrogen atmosphere at room temperature for 4 hr. The insolubles were filtered and were washed with 90 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia =8:8:1:1) and was then purified by Sephadex LH-20 (methanol) to give the title compound (15 mg, 45%).
Physicochemical properties of the compound prepared in Example 15
(1) Color and form: Colorless solid
(2) Molecular formula: C₂₆H₃₄N₆O₆S
(3) Mass spectrum (FABMS): m/z 559 (M+H)⁺
(4) Specific rotation: [α]_{D}²⁵ +93° (c 0.44, CH₃OH)
(5) ¹H NMR spectrum (400 MHz, CD₃OD) δ (ppm): 1.71 (1H, dddd, piperidine), 1.95 (2H, dddd, tetrahydropyrimidine), 1.95 - 2.03 (1H, m, piperidine), 2.59 (1H, dd, piperidine), 2.82 (1H, ddd, piperidine), 3.22 (1H, ddd, piperidine), 3.27 - 3.38 (1H, m, CONHCH₂), 3.36 (4H, br t, tetrahydropyrimidine), 3.50 (3H, s, OMe), 3.45 - 3.60 (1H, m, piperidine), 3.67 - 3.82 (3H, m, piperidine and CONHCH₂CH), 4.17 (1H, ddd, piperidine), 7.15 (1H, ddd, C₆H₄), 7.27 (1H, d, C₆H₄), 7.32 (1H, dd, C₆H₄), 7.46 - 7.52 (3H, m, C₆H₄ and C₆H₅), 7.53 - 7.59 (1H, m, C₆H₅), 7.84 - 7.89 (2H, m, C₆H₅)

### Intermediate 25: Methyl 3-bromo-5-fluorobenzoate

Methanol (30 ml) was added to 3-bromo-5-fluorobenzoic acid (3.1 g, 14 mmol) to prepare a solution. Concentrated sulfuric acid (3.0 ml) was added to the solution, and the mixture was heated under reflux for 1.5 hr. The temperature of the reaction mixture was returned to room temperature, and the reaction mixture was slowly poured into 400 ml of a saturated aqueous sodium hydrogencarbonate solution, followed by extraction twice with 250 ml of diethyl ether. The combined organic layers were dried over anhydrous magnesium sulfate and were concentrated under the reduced pressure to give the title compound (2.6 g, 80%).
Physicochemical properties of intermediate 25
(1) Color and form: Colorless oil
(2) Molecular formula: C₈H₆O₂BrF
(3) Mass spectrum (EIMS): m/z 232, 234 (M)⁺
(4) ¹H NMR spectrum (400 MHz, CDCl₃) δ (ppm): 3.94 (3H, s, CO₂Me), 7.44 (1H, ddd, C₆H₃), 7.67 (1H, ddd, C₆H₃), 7.98 (1H, s, C₆H₃)

### Intermediate 26: Methyl 5-fluoro-3-(4-oxopiperidin-1-yl)benzoate

Toluene (22 ml) was added to 4-piperidone monohydrate monohydrochloride (1.8 g, 13 mmol) and intermediate 25 (2.6 g, 11 mmol) in an argon atmosphere to prepare a solution. (R)-(+)-2,2'-Bis(diphenylphosphino)-1,1'-binaphthyl (699 mg, 1.1 mmol), cesium carbonate (5.5 g, 16.8 mmol), and palladium acetate (257 mg, 1.1 mmol) were added in that order to the solution, and the mixture was stirred at 100°C for 21 hr. The temperature of the reaction mixture was returned to room temperature, and the insolubles were then filtered and were washed with 100 ml of toluene. The filtrate and the washings were combined. Water (400 ml) was added thereto, and the mixture was extracted twice with 250 ml of ethyl acetate. The extract was then washed with 300 ml of saturated brine, was dried over anhydrous magnesium sulfate, and was concentrated under the reduced pressure. The residue was purified by column chromatography on silica gel (development system: methylene chloride : methanol = 20 : 1) to give the title compound (159 mg, 5.7%).
Physicochemical properties of intermediate 26
(1) Color and form: Light yellow syrup
(2) Molecular formula: C₁₃H₁₄NO₃F
(3) Mass spectrum (EIMS): m/z 251 (M)⁺
(4) ¹H NMR spectrum (400 MHz, CDCl₃) δ (ppm): 2.57 (4H, t, piperidone), 3.68 (4H, t, piperidone), 3.92 (3H, s, CO₂Me), 6.80 (1H, ddd, C₆H₃), 7.19 (1H, ddd, C₆H₃), 7.41 (1H, dd, C₆H₃)

### Intermediate 27: Methyl 5-fluoro-3-(4-hydroxypiperidin-1-yl)benzoate

Tetrahydrofuran (13 ml) was added to intermediate 26 (159 mg, 0.63 mmol) to prepare a solution. The solution was cooled to -78°C, and sodium boron hydride (34 mg, 0.90 mmol) was added to the cooled solution. The reaction temperature was returned to room temperature, and the mixture was stirred for 2.5 hr. Water (50 ml) was added thereto, and the mixture was extracted twice with 100 ml of ethyl acetate. The combined organic layers were washed with 100 ml of saturated brine, were dried over anhydrous magnesium sulfate, and were concentrated under the reduced pressure to give the title compound (161 mg, 100%).
Physicochemical properties of intermediate 27
(1) Color and form: Colorless solid
(2) Molecular formula: C₁₃H₁₆NO₃F
(3) Mass spectrum (EIMS): m/z 253 (M)⁺
(4) ¹H NMR spectrum (400 MHz, CDCl₃) δ (ppm): 1.67 (2H, dddd, piperidine), 2.01 (2H, m, piperidine), 3.02 (2H, ddd, piperidine), 3.61 (2H, ddd, piperidine), 3.90 (1H, m, piperidine), 3.90 (3H, s, CO₂Me), 6.77 (1H, ddd, C₆H₃), 7.13 (1H, ddd, C₆H₃), 7.39 (1H, dd, C₆H₃)

### Intermediate 28: Methyl 5-fluoro-3-{4-(methanesulfonyloxy)piperidin-1-yl}benzoate

Methylene chloride (6.0 ml) was added to intermediate 27 (159 mg, 0.63 mmol) to prepare a solution, and triethylamine (220 µl, 1.6 mmol) and methanesulfonyl chloride (60 µl, 0.75 mmol) were added in that order to the solution at room temperature. A reaction was allowed to proceed for 15 min, and 50 ml of water was added thereto, followed by extraction twice with 70 ml of methylene chloride. The combined residues were dried over anhydrous magnesium sulfate and were concentrated under the reduced pressure to give the title compound (200 mg, 96%).
Physicochemical properties of intermediate 28
(1) Color and form: Light yellow syrup
(2) Molecular formula: C₁₄H₁₈NO₅FS
(3) Mass spectrum (EIMS): m/z 331 (M)⁺
(4) ¹H NMR spectrum (400 MHz, CDCl₃) δ (ppm): 2.01 (2H, m, piperidine), 2.13 (2H, m, piperidine), 3.06 (3H, s, Ms), 3.20 (2H, ddd, piperidine), 3.53 (2H, ddd, piperidine), 3.91 (3H, s, CO₂Me), 4.94 (1H, tt, piperidine), 6.77 (1H, ddd, C₆H₃), 7.17 (1H, ddd, C₆H₃), 7.38 (1H, dd, C₆H₃)

### Intermediate 29: Methyl 3-(4-azidopiperidin-1-yl)-5-fluorobenzoate

Dimethylformamide (8.0 ml) was added to intermediate 28 (200 mg, 0.60 mmol) to prepare a solution. Sodium azide (82 mg, 1.2 mmol) was added to the solution, and a reaction was allowed to proceed at 80°C for 5 hr. The temperature of the reaction mixture was returned to room temperature, 50 ml of water was then added thereto, and the mixture was extracted twice with 50 ml of ethyl acetate. The combined organic layers were washed twice with 50 ml of saturated brine and were dried over anhydrous magnesium sulfate. The residue was purified by column chromatography on silica gel (development system: hexane : ethyl acetate = 6 : 1) to give the title compound (122 mg, 72%).
Physicochemical properties of intermediate 29
(1) Color and form: Colorless oil
(2) Molecular formula: C₁₃H₁₅N₄O₂F
(3) Mass spectrum (EIMS): m/z 278 (M)⁺
(4) ¹H NMR spectrum (400 MHz, CDCl₃) δ (ppm): 1.76 (2H, dddd, piperidine), 2.04 (2H, m, piperidine), 3.05 (2H, ddd, piperidine), 3.55 - 3.67 (3H, m, piperidine), 3.91 (3H, s, CO₂Me), 6.77 (1H, ddd, C₆H₃), 7.16 (1H, ddd, C₆H₃), 7.38 (1H, br s, C₆H₃)

### Intermediate 30: Methyl 3-(4-aminopiperidin-1-yl)-5-fluorobenzoate

1,4-Dioxane (8.0 ml), 3.0 ml of water, and 1.0 ml of acetic acid were added in that order to intermediate 29 (118 mg, 0.42 mmol) to prepare a solution. To the solution was added 40 mg of 10% palladium-carbon. The mixture was stirred in a hydrogen atmosphere at room temperature for 17 hr. The insolubles were filtered and were washed with 100 ml of 1,4-dioxane. The filtrate and the washings were combined followed by concentration under the reduced pressure. The residue was purified by column chromatography on silica gel (development system: methylene chloride : methanol : concentrated aqueous ammonia = 100 : 10 : 1) to give the title compound (54 mg, 51%).
Physicochemical properties of intermediate 30
(1) Color and form: Light yellow syrup
(2) Molecular formula: C₁₃H₁₇N₂O₂F
(3) Mass spectrum (EIMS): m/z 252 (M)⁺
(4) ¹H NMR spectrum (400 MHz, CDCl₃) δ (ppm): 1.46 (2H, m, piperidine), 1.93 (2H, m, piperidine), 2.82 - 2.94 (3H, m, piperidine), 3.71 (2H, m, piperidine), 3.90 (3H, s, CO₂Me), 6.76 (1H, ddd, C₆H₃), 7.12 (1H, ddd, C₆H₃), 7.52 (1H, dd, C₆H₃)

### Intermediate 31: Methyl 5-fluoro-3-{4-(pyrimidin-2-ylamino)piperidin-1-yl}benzoate

Dimethyl sulfoxide (2.0 ml) was added to intermediate 30 (53 mg, 0.21 mmol) to prepare a solution. Diisopropylethylamine (210 µl, 1.2 mmol) and 2-bromopyrimidine (35 mg, 0.21 mmol) were added in that order to the solution. A reaction was allowed to proceed at 120°C for 7.5 hr, and the temperature of the reaction mixture was then returned to room temperature. Water (200 ml) was added to the reaction mixture, and the mixture was extracted twice with 150 ml of ethyl acetate. The combined organic layers were washed once with 200 ml of water and once with 200 ml of saturated brine, were dried over anhydrous magnesium sulfate, and were then concentrated under the reduced pressure. The residue was purified by column chromatography on silica gel (development system: hexane : ethyl acetate = 1 : 1) to give the title compound (36 mg, 51%).
Physicochemical properties of intermediate 31
(1) Color and form: Light yellow solid
(2) Molecular formula: C₁₇H₁₉N₄O₂F
(3) Mass spectrum (EIMS): m/z 330 (M)⁺
(4) ¹H NMR spectrum (400 MHz, CDCl₃) δ (ppm): 1.63 (2H, m, piperidine), 2.18 (2H, m, piperidine), 3.01 (2H, m, piperidine), 3.72 (2H, m, piperidine), 3.91 (3H, s, CO₂Me), 4.03 (1H, m, piperidine), 6.55 (1H, t, pyrimidine), 6.78 (1H, ddd, C₆H₃), 7.14 (1H, ddd, C₆H₃), 7.40 (1H, m, C₆H₃), 8.29 (2H, d, pyrimidine)

### Intermediate 32: 5-Fluoro-3-{4-(pyrimidin-2-ylamino)-piperidin-1-yl}benzoic acid

Tetrahydrofuran (3.0 ml) and 1.0 ml of methanol were added in that order to intermediate 31 (33 mg, 0.099 mmol) to prepare a solution, and 1.0 ml of a 1.0 M aqueous sodium hydroxide solution was added to the solution. The mixture was stirred at 60°C for one hr. The temperature of the reaction mixture was then returned to room temperature, the reaction mixture was adjusted to pH 4 by the addition of 1.0 M hydrochloric acid, and 20 ml of water was added thereto. The precipitate was collected by filtration and was dried to give the title compound (28 mg, 91%).
Physicochemical properties of intermediate 32
(1) Color and form: Colorless solid
(2) Molecular formula: C₁₆H₁₇N₄O₂F
(3) Mass spectrum (EIMS): m/z 316 (M)⁺
(4) ¹H NMR spectrum (400 MHz, DMSO-d₆) δ (ppm): 1.55 (2H, br ddd, piperidine), 1.93 (2H, br d, piperidine), 2.91 (2H, br dd, piperidine), 3.79 (2H, br d, piperidine), 3.88 - 4.00 (1H, m, piperidine), 6.55 (1H, t, pyrimidine), 6.96 (1H, br d, C₆H₃), 7.12 (1H, d, C₆H₃), 7.29 (1H, s, C₆H₃), 8.27 (2H, d, pyrimidine)

### Example 16: t-Butyl (2S)-benzenesulfonylamino-3-[5-fluoro-3-{4-(pyrimidin-2-ylamino)piperidin-1-yl}benzoylamino]propionate

Dimethylformamide (1.0 ml) was added to intermediate 32 (27 mg, 0.084 mmol) to prepare a solution, and t-butyl (2S)-N-benzenesulfonyl-2,3-diaminopropionate (30 mg, 0.10 mmol) was added to the solution. Further, 1-hydroxybenzotriazole (18 mg, 0.14 mmol), N-methylmorpholine (47 µl, 0.43 mmol), and 1-ethyl-3-(3-dimethylaminopropyl)carbodiimide hydrochloride (35 mg, 0.18 mmol) were added thereto, and a reaction was allowed to proceed at room temperature for 14 hr. A saturated aqueous sodium hydrogencarbonate solution (10 ml) was added to stop the reaction, and 100 ml of water was added thereto. The mixture was extracted twice with 100 ml of ethyl acetate. The combined organic layers were washed with 100 ml of saturated brine, were dried over anhydrous magnesium sulfate, and were then concentrated under the reduced pressure. The residue was purified by column chromatography on silica gel (development system: methylene chloride : methanol = 10 : 1) to give the title compound (52 mg, 100%).
Physicochemical properties of the compound prepared in Example 16
(1) Color and form: Colorless solid
(2) Molecular formula: C₂₉H₃₅N₆O₅SF
(3) Mass spectrum (EIMS): m/z 599 (M)⁺
(4) Specific rotation: [α]_{D}²⁵ +31° (c 2.2, CH₂Cl₂)
(5) ¹H NMR spectrum (400 MHz, CDCl₃) δ (ppm): 1.30 (9H, s, t-Bu), 1.60 - 1.64 (2H, m, piperidine), 2.18 (2H, br d, piperidine), 3.03 (2H, br dd, piperidine), 3.54 (1H, ddd, CONHCH₂), 3.75 (2H, br d, piperidine), 3.91 (1H, ddd, CONHCH₂), 3.91 (1H, m, CONHCH₂CH), 3.98 - 4.08 (1H, m, piperidine), 6.54 (1H, t, pyrimidine), 6.72 (1H, ddd, C₆H₃), 6.85 (1H, br d, C₆H₃), 7.18 (1H, br dd, C₆H₃), 7.48 - 7.54 (2H, m, C₆H₅), 7.56 - 7.61 (1H, m, C₆H₅), 7.84 - 7.87 (2H, m, C₆H₅), 8.28 (2H, d, pyrimidine)

### Example 17: (2S)-Benzenesulfonylamino-3-[5-fluoro-3-{4-(pyrimidin-2-ylamino)piperidin-1-yl}benzoylamino]-propionic acid

Methylene chloride (4.0 ml) was added to the compound (50 mg, 0.084 mmol) prepared in Example 16 to prepare a solution, and 2.0 ml of trifluoroacetic acid was added to the solution at room temperature. A reaction was allowed to proceed for 3 hr, and the reaction solution was then concentrated under the reduced pressure to give the title compound (56 mg, 100% (as tritrifluoroacetate)).
Physicochemical properties of the compound prepared in Example 17 (as tritrifluoroacetate)
(1) Color and form: Colorless solid
(2) Molecular formula: C₂₅H₂₇N₆O₅SF
(3) Mass spectrum (EIMS): m/z 543 (M)⁺
(4) Specific rotation: [α]_{D}²⁵ +17° (c 1.0, CH₃OH)
(5) ¹H NMR spectrum (400 MHz, CD₃OD) δ (ppm): 1.74 (2H, m, piperidine), 2.12 (2H, m, piperidine), 3.00 (2H, m, piperidine), 3.48 (1H, dd, CONHCH₂), 3.73 (1H, dd, CONHCH₂), 3.86 (2H, br d, piperidine), 4.06 (1H, m, piperidine), 4.21 (1H, m, CONHCH₂CH), 6.82 (1H, t, pyrimidine), 6.84 - 6.91 (2H, m, C₆H₃), 7.22 (1H, m, C₆H₃), 7.45 (2H, m, C₆H₅), 7.52 (1H, m, C₆H₅), 7.83 (2H, m, C₆H₅), 8.44 (2H, d, pyrimidine)

### Example 18: (2S)-Benzenesulfonylamino-3-[5-fluoro-3-{4-(1,4,5,6-tetrahydropyrimidin-2-ylamino)piperidin-1-yl}benzoylamino]propionic acid

1,4-Dioxane (2.0 ml) and 0.20 ml of water were added in that order to 53 mg of the compound prepared in Example 17 to prepare a solution. To the solution was added 14 mg of 10% palladium-carbon. The mixture was stirred in a hydrogen atmosphere at room temperature for 6 hr. The insolubles were filtered and were washed with 50 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (27 mg, 61%).
Physicochemical properties of the compound prepared in Example 18
(1) Color and form: Colorless solid
(2) Molecular formula: C₂₅H₃₁N₆O₅SF
(3) Specific rotation: [α]_{D}²⁵ +64° (c 0.30, CH₃OH)
(4) ¹H NMR spectrum (400 MHz, CD₃OD) δ (ppm): 1.55 - 1.67 (2H, m, piperidine), 1.96 (2H, dddd, tetrahydropyrimidine), 1.96 - 2.03 (2H, m, piperidine), 2.95 (2H, ddd, piperidine), 3.36 (4H, br t, tetrahydropyrimidine), 3.45 - 3.51 (1H, m, piperidine), 3.52 (1H, dd, CONHCH₂), 3.69 (1H, dd, CONHCH₂), 3.77 (1H, dd, CONHCH₂CH), 3.81 (2H, br d, piperidine), 6.82 (1H, ddd, C₆H₃), 6.91 (1H, ddd, C₆H₃), 7.24 (1H, dd, C₆H₃), 7.46 - 7.52 (2H, m, C₆H₅), 7.53 - 7.58 (1H, m, C₆H₅), 7.84 - 7.88 (2H, m, C₆H₅)

### Intermediate 33: Methyl 3-bromo-6-fluorobenzoate

Methanol (30 ml) was added to 3-bromo-6-fluorobenzoic acid (3.0 g, 14 mmol) to prepare a solution, 3.0 ml of concentrated sulfuric acid was added to the solution, and the mixture was heated under reflux for 1.5 hr. The temperature of the reaction mixture was returned to room temperature, the reaction mixture was then slowly poured into 500 ml of a saturated aqueous sodium hydrogencarbonate solution, and the mixture was extracted twice with 500 ml of diethyl ether. The combined organic layers were washed with 300 ml of saturated brine, were dried over anhydrous magnesium sulfate, and were concentrated under the reduced pressure to give the title compound (2.6 g, 81%).
Physicochemical properties of intermediate 33
(1) Color and form: Light yellow oil
(2) Molecular formula: C₈H₆O₂BrF
(3) Mass spectrum (EIMS): m/z 234 (M)⁺
(4) ¹H NMR spectrum (400 MHz, CDCl₃) δ (ppm): 3.94 (3H, s, CO₂Me), 7.04 (1H, dd, C₆H₃), 7.62 (1H, dd, C₆H₃), 8.06 (1H, dd, C₆H₃)

### Intermediate 34: Methyl 6-fluoro-3-{4-(trimethylsilyloxy)piperidin-1-yl}benzoate

Toluene (2.0 ml) was added to 4-(trimethylsilyloxy)piperidine (209 mg, 1.2 mmol) and intermediate 33 (234 mg, 1.0 mmol) in an argon atmosphere to prepare a solution. (R)-(+)-2,2'-Bis(diphenylphosphino)-1,1'-binaphthyl (62 mg, 0.10 mmol), cesium carbonate (492 mg, 1.5 mmol), and palladium acetate (26 mg, 0.10 mmol) were added in that order to the solution, and the mixture was stirred at 100°C for 21 hr. The temperature of the reaction mixture was returned to room temperature, and the insolubles were then filtered and were washed with 100 ml of ethyl acetate. The filtrate and the washings were combined. Water (100 ml) was added thereto, and the mixture was extracted twice with 100 ml of ethyl acetate. The extract was then washed with 100 ml of saturated brine, was dried over anhydrous magnesium sulfate, and was concentrated under the reduced pressure. The residue was purified by column chromatography on silica gel (development system: hexane : ethyl acetate = 6 : 1) to give the title compound (68 mg, 21%).
Physicochemical properties of intermediate 34
(1) Color and form: Colorless oil
(2) Molecular formula: C₁₆H₂₄NO₃FSi
(3) Mass spectrum (EIMS): m/z 325 (M)⁺
(4) ¹H NMR spectrum (400 MHz, CDCl₃) δ (ppm): 0.14 (9H, s, OTMS), 1.65 - 1.75 (2H, m, piperidine), 1.89 (2H, m, piperidine), 2.91 (2H, ddd, piperidine), 3.45 (2H, m, piperidine), 3.81 (1H, tt, piperidine), 3.92 (3H, s, CO₂Me), 7.01 (1H, dd, C₆H₃), 7.08 (1H, ddd, C₆H₃), 7.44 (1H, dd, C₆H₃)

### Intermediate 35: Methyl 6-fluoro-3-(4-hydroxypiperidin-1-yl)benzoate

Methanol (2.0 ml) was added to intermediate 34 (65 mg, 0.20 mmol) to prepare a solution. To the solution was added 2.0 ml of 1.0 M hydrochloric acid at room temperature. The mixture was stirred for 30 min and was then adjusted to pH 6 by the addition of a 1.0 M aqueous sodium hydroxide solution, and 100 ml of water was added thereto. The mixture was extracted twice with 100 ml of ethyl acetate. The combined organic layers were washed with 100 ml of saturated brine, were dried over anhydrous magnesium sulfate, and were concentrated under the reduced pressure to give the title compound (47 mg, 93%).
Physicochemical properties of intermediate 35
(1) Color and form: Light yellow syrup
(2) Molecular formula: C₁₃H₁₆NO₃F
(3) Mass spectrum (EIMS): m/z 253 (M)⁺
(4) ¹H NMR spectrum (400 MHz, CDCl₃) δ (ppm): 1.66 - 1.76 (2H, m, piperidine), 1.98 - 2.08 (2H, m, piperidine), 2.87 - 2.97 (2H, m, piperidine), 3.45 - 3.53 (2H, m, piperidine), 3.83 - 3.91 (1H, m, piperidine), 3.93 (3H, s, CO₂Me), 7.03 (1H, br dd, C₆H₃), 7.08 (1H, br s, C₆H₃), 7.45 (1H, br s, C₆H₃)

### Intermediate 36: Methyl 6-fluoro-3-{4-(methanesulfonyloxy)piperidin-1-yl}benzoate

Methylene chloride (10 ml) was added to intermediate 35 (227 mg, 0.89 mmol) to prepare a solution, and triethylamine (320 µl, 2.3 mmol) and methanesulfonyl chloride (85 µl, 1.1 mmol) were added in that order to the solution at room temperature. The mixture was stirred for 30 min, and 100 ml of water was then added thereto, followed by extraction twice with 100 ml of methylene chloride. The combined residues were dried over anhydrous magnesium sulfate and were concentrated under the reduced pressure to give the title compound (286 mg, 97%).
Physicochemical properties of intermediate 36
(1) Color and form: Light yellow syrup
(2) Molecular formula: C₁₄H₁₈NO₅FS
(3) Mass spectrum (EIMS): m/z 331 (M)⁺
(4) ¹H NMR spectrum (400 MHz, CDCl₃) δ (ppm): 2.10 (2H, br s, piperidine), 2.25 (2H, br s, piperidine), 3.08 (3H, s, Ms), 3.10 - 3.20 (2H, m, piperidine), 3.40 - 3.50 (2H, m, piperidine), 3.94 (3H, s, CO₂Me), 4.94 (1H, br s, piperidine), 7.08 (2H, br dd, C₆H₃), 7.54 (1H, br s, C₆H₃)

### Intermediate 37: Methyl 3-(4-azidopiperidin-1-yl)-6-fluorobenzoate

Dimethylformamide (10 ml) was added to intermediate 36 (281 mg, 0.85 mmol) to prepare a solution. Sodium azide (111 mg, 1.7 mmol) was added to the solution, and a reaction was allowed to proceed at 80°C for 5.5 hr. The temperature of the reaction mixture was returned to room temperature, and 100 ml of water was then added thereto, followed by extraction twice with 50 ml of ethyl acetate. The combined organic layers were washed once with 100 ml of water and once with 50 ml of saturated brine, were then dried over anhydrous magnesium sulfate, and were concentrated under the reduced pressure. The residue was then purified by column chromatography on silica gel (development system: hexane : ethyl acetate = 4 : 1) to give the title compound (189 mg, 80%).
Physicochemical properties of intermediate 37
(1) Color and form: Colorless oil
(2) Molecular formula: C₁₃H₁₅N₄O₂F
(3) Mass spectrum (EIMS): m/z 278 (M)⁺
(4) ¹H NMR spectrum (400 MHz, CDCl₃) δ (ppm): 1.75 - 1.87 (2H, m, piperidine), 2.05 (2H, br s, piperidine), 2.96 (2H, br dd, piperidine), 3.43 - 3.51 (2H, m, piperidine), 3.61 (1H, br s, piperidine), 3.93 (3H, s, CO₂Me), 7.05 (1H, br dd, C₆H₃), 7.10 (1H, br s, C₆H₃), 7.47 (1H, br s, C₆H₃)

### Example 19: t-Butyl (2S)-benzenesulfonylamino-3-[6-fluoro-3-{4-(pyrimidin-2-ylamino)piperidin-1-yl}benzoylamino]propionate

1,4-Dioxane (10 ml) and 5.0 ml of water were added in that order to intermediate 37 (185 mg, 0.67 mmol) to prepare a solution, and 46 mg of 10% palladium-carbon was added to the solution. The mixture was stirred in a hydrogen atmosphere at room temperature for 6 hr. The insolubles were filtered and were washed with 100 ml of 1,4-dioxane. The filtrate and the washings were combined followed by concentration under the reduced pressure to give methyl 3-(4-aminopiperidin-1-yl)-6-fluorobenzoate (134 mg, 80%).

Dimethyl sulfoxide (2.0 ml) was added to the methyl 3-(4-aminopiperidin-1-yl)-6-fluorobenzoate (134 mg, 0.53 mmol) thus obtained to prepare a solution. Diisopropylethylamine (630 µl, 3.6 mmol) and 2-bromopyrimidine (119 mg, 0.75 mmol) were added in that oder to the solution. A reaction was allowed to proceed at 80°C for 4 hr, and the temperature of the reaction mixture was then returned to room temperature. Water (100 ml) was added to the reaction mixture, and the mixture was extracted twice with 70 ml of ethyl acetate. The combined organic layers were washed once with 100 ml of water and once with 100 ml of saturated brine, were dried over anhydrous magnesium sulfate, and were then concentrated under the reduced pressure to give methyl 6-fluoro-3-{4-(pyrimidin-2-ylamino)piperidin-1-yl}benzoate (172 mg, 98%).

Tetrahydrofuran (6.0 ml) and 2.0 ml of methanol were added in that order to the methyl 6-fluoro-3-{4-pyrimidin-2-ylamino)piperidin-1-yl}benzoate (172 mg, 0.52 mmol) thus obtained to prepare a solution, and 2.0 ml of a 1.0 M aqueous sodium hydroxide solution was added to the solution. A reaction was allowed to proceed at 60°C for 30 min, and the temperature of the reaction mixture was then returned to room temperature. The reaction mixture was adjusted to pH 4 by the addition of 1.0 M hydrochloric acid, 100 ml of water was added thereto, and the mixture was extracted twice with 70 ml of ethyl acetate. The combined organic layers were washed with 70 ml of saturated brine, were dried over anhydrous magnesium sulfate, and were then concentrated under the reduced pressure to give 6-fluoro-3-{4-pyrimidin-2-ylamino)piperidin-1-yl}benzoic acid (72 mg, 44%).

Dimethylformamide (3.0 ml) was added to the 6-fluoro-3-{4-(pyrimidin-2-ylamino)piperidin-1-yl}benzoic acid (72 mg, 0.23 mmol) thus obtained to prepare a solution, and t-butyl (2S)-N-benzenesulfonyl-2,3-diaminopropionate (91 mg, 0.18 mmol) was added to the solution. Further, 1-hydroxybenzotriazole (56 mg, 0.41 mmol), N-methylmorpholine (130 µl, 1.2 mmol), and 1-ethyl-3-(3-dimethylaminopropyl)carbodiimide hydrochloride (106 mg, 0.55 mmol) were added thereto, and a reaction was allowed to proceed at room temperature for 9 hr. A saturated aqueous sodium hydrogencarbonate solution was added to stop the reaction, and 100 ml of water was added thereto. The mixture was extracted twice with 70 ml of ethyl acetate. The combined organic layers were washed twice with 70 ml of saturated brine, were dried over anhydrous magnesium sulfate, and were then concentrated under the reduced pressure. The residue was purified by column chromatography on silica gel (development system: methylene chloride : methanol : benzene : ethyl acetate = 9 : 1 : 6 : 4). Next, 1.0 ml of methanol was added to the purified product to prepare a solution, and 20 ml of water was added dropwise to the solution. The resultant precipitate was collected by filtration and was then dried to give the title compound (53 mg, 39%).
Physicochemical properties of the compound prepared in Example 19
(1) Color and form: Colorless solid
(2) Molecular formula: C₂₉H₃₅N₆O₅SF
(3) Mass spectrum (EIMS): m/z 598 (M)⁺
(4) Specific rotation: [α]_{D}²⁵ +30° (c 1.1, CH₂Cl₂)
(5) ¹H NMR spectrum (400 MHz, CDCl₃) δ (ppm): 1.29 (9H, s, t-Bu), 1.59 - 1.72 (2H, m, piperidine), 2.12 - 2.22 (2H, m, piperidine), 2.88 - 2.97 (2H, m, piperidine), 3.56 - 3.65 (2H, m, piperidine), 3.70 - 3.85 (2H, m, CONHCH₂), 3.90 - 4.00 (1H, m, piperidine), 4.13 (1H, ddd, CONHCH₂CH), 6.54 (1H, t, pyrimidine), 6.98 - 7.10 (2H, m, C₆H₃), 7.43 - 7.49 (2H, m, C₆H₅), 7.50 - 7.55 (1H, m, C₆H₅), 7.57 (1H, dd, C₆H₃), 7.82 - 7.88 (2H, m, C₆H₅), 8.29 (2H, d, pyrimidine)

### Example 20: (2S)-Benzenesulfonylamino-3-[6-fluoro-3-{4-(pyrimidin-2-ylamino)piperidin-1-yl}benzoylamino]propionic acid

Methylene chloride (3.0 ml) was added to the compound (49 mg, 0.081 mmol) prepared in Example 19 to prepare a solution, and 3.0 ml of trifluoroacetic acid was added to the solution at room temperature. A reaction was allowed to proceed for 2.5 hr, and the reaction solution was concentrated under the reduced pressure to give the title compound (52 mg, 72% (as tritrifluoroacetate)).
Physicochemical properties of the compound prepared in Example 20 (as tritrifluoroacetate).
(1) Color and form: Colorless solid
(2) Molecular formula: C₂₅H₂₇N₆O₅SF
(3) Mass spectrum (FABMS): m/z 543 (M+H)⁺
(4) Specific rotation: [α]_{D}²⁵ +19° (c 0.51, CH₃OH)
(5) ¹H NMR spectrum (400 MHz, CD₃OD) δ (ppm): 1.88 (2H, m, piperidine), 2.21 (2H, m, piperidine), 3.14 (2H, m, piperidine), 3.47 (1H, dd, CONHCH₂), 3.75 (2H, m, piperidine), 3.81 (1H, dd, CONHCH₂), 4.10 (1H, m, piperidine), 4.23 (1H, dd, CONHCH₂CH), 6.87 (1H, t, pyrimidine), 7.18 (1H, dd, C₆H₃), 7.35 (1H, ddd, C₆H₃), 7.42 - 7.54 (3H, m, C₆H₅), 7.56 (1H, dd, C₆H₃), 7.82 - 7.86 (2H, m, C₆H₅), 8.49 (2H, d, pyrimidine)

### Example 21: (2S)-Benzenesulfonylamino-3-[6-fluoro-3-{4-(1,4,5,6-tetrahydropyrimidin-2-ylamino)piperidin-1-yl}benzoylamino]propionic acid

1,4-Dioxane (3.0 ml) and 0.30 ml of water were added in that order to 46 mg of the compound prepared in Example 20 to prepare a solution, and 13 mg of 10% palladium-carbon was added to the solution. The mixture was stirred in a hydrogen atmosphere at room temperature for 5 hr. The insolubles were filtered and were washed with 50 ml of 1,4-dioxane. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (8.3 mg, 19%).
Physicochemical properties of the compound prepared in Example 21
(1) Color and form: Colorless solid
(2) Molecular formula: C₂₅H₃₁N₆O₅SF
(3) Mass spectrum (TSPMS): m/z 547 (M+H)⁺
(4) Specific rotation: [α]_{D}²⁵ +63° (c 0.20, CH₃OH)
(5) ¹H NMR spectrum (400 MHz, CD₃OD) δ (ppm): 1.65 (2H, dddd, piperidine), 1.96 (2H, dddd, tetrahydropyrimidine), 1.96 - 2.04 (2H, m, piperidine), 2.86 (2H, ddd, piperidine), 3.36 (4H, br t, tetrahydropyrimidine), 3.41 (1H, m, piperidine), 3.60 - 3.66 (2H, m, piperidine), 3.61 (1H, dd, CONHCH₂), 3.70 (1H, dd, CONHCH₂), 3.75 (1H, dd, CONHCH₂CH), 7.06 (1H, dd, C₆H₃), 7.11 (1H, ddd, C₆H₃), 7.41 (1H, m, C₆H₃), 7.46 - 7.52 (2H, m, C₆H₅), 7.52 - 7.58 (1H, m, C₆H₅), 7.84 - 7.88 (2H, m, C₆H₅)

### Intermediate 38: 3-Bromo-2-fluorobenzoic acid

Tetrahydrofuran (60 ml) was added to diisopropylamine (9.6 ml, 68 mmol) in an argon atmosphere. n-Butyllithium (hexane solution, 1.5 M, 38 ml, 57 mmol) was added dropwise thereto at -10°C, and the mixture was stirred for one hr. Separately, 55 ml of tetrahydrofuran was added to 1-bromo-2-fluorobenzene (10 g, 57 mmol) to prepare a solution which was then added dropwise to the lithium reagent solution at -78°C. The mixture was stirred for 2 hr and was then stirred for additional 30 min while blowing carbon dioxide thereinto. The temperature of the reaction mixture was returned to room temperature, and the reaction mixture was concentrated under the reduced pressure. Water (200 ml) was added to the residue to prepare a solution, and the solution was washed twice with 100 ml of diethyl ether. The aqueous layer was adjusted to pH 1 by the addition of 1.0 M hydrochloric acid, was extracted twice with 300 ml of methylene chloride, was dried over anhydrous magnesium sulfate, and was concentrated under the reduced pressure to give the title compound (7.1 g, 57%).
Physicochemical properties of intermediate 38
(1) Color and form: Colorless solid
(2) Molecular formula: C₇H₄O₂BrF
(3) Mass spectrum (EIMS): m/z 218, 220 (M)⁺
(4) ¹H NMR spectrum (400 MHz, CDCl₃) δ (ppm): 7.14 (1H, ddd, C₆H₃), 7.81 (1H, ddd, C₆H₃), 7.98 (1H, ddd, C₆H₃)

### Intermediate 39: Ethyl 3-bromo-2-fluorobenzoate

Ethanol (30 ml) was added to intermediate 38 (3.0 g, 14 mmol) to prepare a solution. Concentrated sulfuric acid (0.30 ml) was added to the solution, and the mixture was heated under reflux for 8 hr. The temperature of the reaction mixture was returned to room temperature, the reaction mixture was then slowly poured into 500 ml of a saturated aqueous sodium hydrogencarbonate solution, and the mixture was extracted twice with 500 ml of ethyl acetate. The combined organic layers were dried over anhydrous magnesium sulfate and were concentrated under the reduced pressure. The residue was purified by column chromatography on silica gel (development system: hexane : ethyl acetate = 4 : 1) to give the title compound (2.7 g, 79%).
Physicochemical properties of intermediate 39
(1) Color and form: Colorless oil
(2) Molecular formula: C₉H₈O₂BrF
(3) Mass spectrum (EIMS): m/z 246, 248 (M)⁺
(4) ¹H NMR spectrum (400 MHz, CDCl₃) δ (ppm): 1.40 (3H, t, Et), 4.41 (2H, q, Et), 7.10 (1H, ddd, C₆H₃), 7.73 (1H, ddd, C₆H₃), 7.87 (1H, ddd, C₆H₃)

### Intermediate 40: Ethyl 2-fluoro-3-(4-hydroxypiperidin-1-yl)benzoate

Toluene (30 ml) was added to 4-(t-butyldimethylsilyloxy)piperidine (4.2 g, 20 mmol) and intermediate 39 (3.6 g, 15 mmol) in an argon atmosphere to prepare a solution. Tri(t-butyl)phosphine (346 mg, 1.7 mmol), sodium t-butoxide (2.1 g, 22 mmol), and palladium acetate (340 mg, 1.5 mmol) were added in that order to the solution, and the mixture was stirred at 80°C for 19 hr. The temperature of the reaction mixture was returned to room temperature, the insolubles were then filtered, and 400 ml of water was added thereto. The mixture was extracted twice with 200 ml of ethyl acetate, and the extract was then dried over anhydrous magnesium sulfate and was concentrated under the reduced pressure. The residue was purified by column chromatography on silica gel (development system: methylene chloride : methanol = 10 : 1) and was then purified by column chromatography on silica gel (development system: hexane : ethyl acetate = 1 : 2) to give the title compound (82 mg, 2.1%).
Physicochemical properties of intermediate 40
(1) Color and form: Light yellow syrup
(2) Molecular formula: C₁₄H₁₈NO₃F
(3) Mass spectrum (EIMS): m/z 267 (M)⁺
(4) ¹H NMR spectrum (400 MHz, CDCl₃) δ (ppm): 1.39 (3H, t, Et), 1.76 (2H, dddd, piperidine), 2.00 - 2.09 (2H, m, piperidine), 2.87 (2H, ddd, piperidine), 3.35 (2H, m, piperidine), 3.87 (1H, dtt, piperidine), 4.38 (2H, q, Et), 7.08 (1H, dd, C₆H₃), 7.13 (1H, ddd, C₆H₃), 7.47 (1H, ddd, C₆H₃)

### Intermediate 41: Ethyl 2-fluoro-3-{(4-methanesulfonyloxy)piperidin-1-yl}benzoate

Methylene chloride (3.0 ml) was added to intermediate 40 (80 mg, 0.30 mmol) to prepare a solution, and triethylamine (84 µl, 0.60 mmol) and methanesulfonyl chloride (28 µl, 0.36 mmol) were added in that order to the solution at room temperature. A reaction was allowed to proceed for 10 min, 50 ml of water was then added thereto, and the mixture was extracted twice with 50 ml of methylene chloride. The combined residues were dried over anhydrous magnesium sulfate and were concentrated under the reduced pressure to give the title compound (106 mg, 100%).
Physicochemical properties of intermediate 41
(1) Color and form: Light yellow syrup
(2) Molecular formula: C₁₅H₂₀NO₅FS
(3) Mass spectrum (EIMS): m/z 345 (M)⁺
(4) ¹H NMR spectrum (400 MHz, CDCl₃) δ (ppm): 1.39 (3H, t, Et), 2.03 - 2.23 (4H, m, piperidine), 3.01 (2H, ddd, piperidine), 3.06 (3H, s, Ms), 3.32 (2H, ddd, piperidine), 4.39 (2H, q, Et), 4.92 (1H, dtt, piperidine), 7.09 (1H, dd, C₆H₃), 7.13 (1H, ddd, C₆H₃), 7.48 - 7.53 (1H, ddd, C₆H₃)

### Intermediate 42: Ethyl 3-(4-azidopiperidin-1-yl)-2-fluorobenzoate

Dimethylformamide (3.0 ml) was added to intermediate 41 (106 mg, 0.30 mmol) to prepare a solution. Sodium azide (43 mg, 0.66 mmol) was added to the solution, and the mixture was stirred at 100°C for 2 hr. The temperature of the reaction mixture was returned to room temperature, 100 ml of water was then added thereto, and the mixture was extracted twice with 100 ml of ethyl acetate. The combined organic layers were washed twice with 100 ml of water and once with 100 ml of saturated brine, were then dried over anhydrous magnesium sulfate, and were concentrated under the reduced pressure to give the title compound (77 mg, 88%).
Physicochemical properties of intermediate 42
(1) Color and form: Colorless oil
(2) Molecular formula: C₁₄H₁₇N₄O₂F
(3) Mass spectrum (TSPMS): m/z 293 (M+H)⁺
(4) ¹H NMR spectrum (400 MHz, CDCl₃) δ (ppm): 1.39 (3H, t, Et), 1.85 (2H, dddd, piperidine), 2.01 - 2.10 (2H, m, piperidine), 2.90 (2H, ddd, piperidine), 3.30 - 3.38 (2H, m, piperidine), 3.59 (1H, tt, piperidine), 4.39 (2H, q, Et), 7.08 (1H, dd, C₆H₃), 7.12 (1H, ddd, C₆H₃), 7.46 - 7.53 (1H, m, C₆H₃)

### Intermediate 43: Ethyl 3-(4-aminopiperidin-1-yl)-2-fluorobenzoate

Ethanol (15 ml) was added to intermediate 42 (77 mg, 0.26 mmol) to prepare a solution, and 19 mg of 10% palladium-carbon was added to the solution. The mixture was stirred in a hydrogen atmosphere at room temperature for 5 hr. The insolubles were filtered and were washed with 100 ml of ethanol. The filtrate and the washings were combined followed by concentration under the reduced pressure to give the title compound (85 mg, 97%).
Physicochemical properties of intermediate 43
(1) Color and form: Colorless syrup
(2) Molecular formula: C₁₄H₁₉N₂O₂F
(3) Mass spectrum (EIMS): m/z 266 (M)⁺
(4) ¹H NMR spectrum (400 MHz, CDCl₃) δ (ppm): 1.39 (3H, t, Et), 1.50 - 1.63 (2H, m, piperidine), 1.90 - 1.98 (2H, m, piperidine), 2.75 (2H, ddd, piperidine), 2.81 (1H, tt, piperidine), 3.41 (2H, br d, piperidine), 4.38 (2H, q, Et), 7.07 (1H, dd, C₆H₃), 7.10 (1H, ddd, C₆H₃), 7.46 (1H, ddd, C₆H₃)

### Intermediate 44: Ethyl 2-fluoro-3-{4-(pyrimidin-2-ylamino)piperidin-1-yl}benzoate

Dimethyl sulfoxide (2.5 ml) was added to intermediate 43 (67 mg, 0.25 mmol) to prepare a solution, and diisopropylethylamine (240 µl, 1.4 mmol) and 2-bromopyrimidine (46 mg, 0.29 mmol) were added in that order to the solution. A reaction was allowed to proceed at 120°C for 9.5 hr, and the temperature of the reaction mixture was then returned to room temperature. Water (100 ml) was added to the reaction mixture, and the mixture was extracted twice with 70 ml of ethyl acetate. The combined organic layers were washed twice with 100 ml of water and once with 100 ml of saturated brine, were dried over anhydrous magnesium sulfate, and were then concentrated under the reduced pressure. The residue was purified by column chromatography on silica gel (development system: hexane : ethyl acetate = 1 : 4) to give the title compound (39 mg, 38%).
Physicochemical properties of intermediate 44
(1) Color and form: Light yellow solid
(2) Molecular formula: C₁₈H₂₁N₄O₂F
(3) Mass spectrum (EIMS): m/z 344 (M)⁺
(4) ¹H NMR spectrum (400 MHz, CDCl₃) δ (ppm): 1.39 (3H, t, Et), 1.74 (2H, dddd, piperidine), 2.15 - 2.23 (2H, m, piperidine), 2.91 (2H, ddd, piperidine), 3.49 - 3.47 (2H, m, piperidine), 3.95 - 4.06 (1H, m, piperidine), 4.39 (2H, q, Et), 6.54 (1H, t, pyrimidine), 7.09 (1H, dd, C₆H₃), 7.14 (1H, ddd, C₆H₃), 7.48 (1H, ddd, C₆H₃), 8.29 (2H, d, pyrimidine)

### Intermediate 45: 2-Fluoro-3-{4-pyrimidin-2-ylamino)-piperidin-1-yl}benzoic acid

Tetrahydrofuran (2.0 ml) and 0.60 ml of methanol were added in that order to intermediate 44 (37 mg, 0.11 mmol) to prepare a solution, and 0.60 ml of a 1.0 M aqueous sodium hydroxide solution was added to the solution. A reaction was allowed to proceed at 50°C for 3 hr, and the temperature of the reaction mixture was then returned to room temperature. The reaction mixture was then adjusted to pH 4 by the addition of 1.0 M hydrochloric acid, and 10 ml of water was added thereto. The precipitate was collected by filtration and was dried to give the title compound (18 mg, 53%).
Physicochemical properties of intermediate 45
(1) Color and form: Colorless solid
(2) Molecular formula: C₁₆H₁₇N₄O₂F
(3) Mass spectrum (TSPMS): m/z 317 (M+H)⁺
(4) ¹H NMR spectrum (400 MHz, DMSO-d₆) δ (ppm): 1.67 (2H, m, piperidine), 1.93 - 2.01 (2H, m, piperidine), 2.74 - 2.82 (2H, m, piperidine), 3.24 - 3.41 (2H, m, piperidine), 3.82 - 3.90 (1H, m, piperidine), 6.55 (1H, t, pyrimidine), 7.16 (1H, dd, C₆H₃), 7.26 (1H, ddd, C₆H₃), 7.37 (1H, ddd, C₆H₃), 8.27 (2H, d, pyrimidine)

### Example 22: t-Butyl (2S)-benzenesulfonylamino-3-[2 fluoro-3-{4-(pyrimidin-2-ylamino)piperidin-1-yl}benzoylamino]propionate

Dimethylformamide (1.0 ml) was added to intermediate 45 (17 mg, 0.055 mmol) to prepare a solution, and t-butyl (2S)-N-benzenesulfonyl-2,3-diaminopropionate (17 mg, 0.055 mmol) was added to the solution. Further, 1-hydroxybenzotriazole (12 mg, 0.088 mmol), N-methylmorpholine (30 µl, 0.28 mmol), and 1-ethyl-3-(3-dimethylaminopropyl)carbodiimide hydrochloride (25 mg, 0.13 mmol) were added thereto, and a reaction was allowed to proceed at room temperature for 15 hr. A saturated aqueous sodium hydrogencarbonate solution (30 ml) was added to stop the reaction, followed by extraction twice with 50 ml of ethyl acetate. The combined organic layers were washed twice with 100 ml of water and once with 100 ml of saturated brine, were dried over anhydrous magnesium sulfate, and were then concentrated under the reduced pressure. The residue was purified by column chromatography on silica gel (development system: methylene chloride : methanol = 10 : 1) to give the title compound (34 mg, 100%).
Physicochemical properties of the compound prepared in Example 22
(1) Color and form: Colorless solid
(2) Molecular formula: C₂₉H₃₅N₆O₅SF
(3) Mass spectrum (TSPMS): m/z 599 (M+H)⁺
(4) ¹H NMR spectrum (400 MHz, CDCl₃) δ (ppm): 1.30 (9H, s, t-Bu), 1.76 (2H, dddd, piperidine), 2.22 (2H, br d, piperidine), 2.92 (2H, dddd, piperidine), 3.36 - 3.44 (2H, m, piperidine), 3.80 - 3.97 (2H, t, CONHCH₂), 3.97 - 4.07 (2H, m, piperidine and CONHCH₂CH), 6.55 (1H, t, pyrimidine), 7.09 - 7.16 (2H, m, C₆H₃), 7.44 - 7.50 (2H, m, C₆H₅), 7.51 - 7.58 (2H, m, C₆H₃ and C₆H₅), 7.83 - 7.88 (2H, m, C₆H₅), 8.30 (2H, d, pyrimidine)

### Example 23: (2S)-Benzenesulfonylamino-3-[2-fluoro-3-{4-(pyrimidin-2-ylamino)piperidin-1-yl}benzoylamino]propionic acid

Methylene chloride (2.0 ml) was added to the compound (32 mg, 0.054 mmol) prepared in Example 22 to prepare a solution, and 2.0 ml of trifluoroacetic acid was added to the solution at room temperature. A reaction was allowed to proceed for 4 hr, and the reaction mixture was concentrated under the reduced pressure to give the title compound (34 mg, 70% (as tritrifluoroacetate)).
(1) Color and form: Light yellow solid
(2) Molecular formula: C₂₅H₂₇N₆O₅SF
(3) Mass spectrum (TSPMS): m/z 543 (M+H)⁺

### Example 24: (2S)-Benzenesulfonylamino-3-[2-fluoro-3-{4-(1,4,5,6-tetrahydropyrimidin-2-ylamino)piperidin-1-yl}benzoylamino]propionic acid

1,4-Dioxane (4.0 ml) and 2.0 ml of water were added in that order to 34 mg of the compound prepared in Example 23 to prepare a solution, and 8.1 mg of 10% palladium-carbon was added to the solution. The mixture was stirred in a hydrogen atmosphere at room temperature for 4 hr. The insolubles were filtered and were washed with 60 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (16 mg, 78%).
Physicochemical properties of the compound prepared in Example 24
(1) Color and form: Colorless solid
(2) Molecular formula: C₂₅H₃₁N₆O₅SF
(3) Mass spectrum (TSPMS): m/z 547 (M+H)⁺
(4) Specific rotation: [α]_{D}²⁵ +54° (c 0.27, CH₃OH)
(5) ¹H NMR spectrum (400 MHz, CD₃OD) δ (ppm): 1.76 (2H, br ddd, piperidine), 1.96 (2H, dddd, tetrahydropyrimidine), 2.02 (2H, br d, piperidine), 2.83 (2H, br dd, piperidine), 3.33 - 3.50 (7H, m, piperidine and tetrahydropyrimidine), 3.66 (1H, d, CONHCH₂), 3.68 (1H, d, CONHCH₂), 3.74 (1H, dd, CONHCH₂CH), 7.13 - 7.20 (2H, m, C₆H₃), 7.34 - 7.40 (1H, m, C₆H₃), 7.47 - 7.53 (2H, m, C₆H₅), 7.53 - 7.59 (1H, m, C₆H₅), 7.85 - 7.89 (2H, m, C₆H₅)

### Intermediate 46: Methyl 3-nitro-5-(trifluoromethyl)-benzoate

Methanol (20 ml) was added to 3-nitro-5-(trifluoromethyl)benzoic acid (5.1 g, 22 mmol) to preapre a solution, and 2.0 ml of concentrated sulfuric acid was added to the solution. The mixture was heated under reflux for 1.5 hr. The temperature of the reaction mixture was returned to room temperature, and the reaction mixture was then slowly poured into sodium hydrogencarbonate. The insolubles were filtered, 300 ml of water was then added thereto, and the mixture was extracted twice with 200 ml of ethyl acetate. The combined organic layers were dried over anhydrous magnesium sulfate and were concentrated under the reduced pressure. The residue was purified by column chromatography on silica gel (development system: hexane : ethyl acetate = 6 : 1) to give the title compound (5.0 g, 91%).
Physicochemical properties of intermediate 46
(1) Color and form: Colorless oil
(2) Molecular formula: C₉H₆NO₄F₃
(3) Mass spectrum (TSPMS): m/z 249 (M+H)⁺
(4) ¹H NMR spectrum (400 MHz, CDCl₃) δ (ppm): 4.04 (3H, s, CO₂Me), 8.63 (1H, br s, C₆H₃), 8.68 (1H, br s, C₆H₃), 9.05 (1H, br s, C₆H₃)

### Intermediate 47: Methyl 3-amino-5-(trifluoromethyl)benzoate

Methanol (20 ml) was added to intermediate 46 (5.0 g, 20 mmol) to prepare a solution, and 3.0 g of 10% palladium-carbon was added to the solution. The mixture was stirred in a hydrogen atmosphere at room temperature for 23.5 hr. The insolubles were filtered and were concentrated under the reduced pressure to give the title compound (4.3 g, 100%).
Physicochemical properties of intermediate 47
(1) Color and form: Colorless syrup
(2) Molecular formula: C₉H₈NO₂F₃
(3) Mass spectrum (EIMS): m/z 219 (M)⁺
(4) ¹H NMR spectrum (400 MHz, CDCl₃) δ (ppm): 3.92 (3H, s, CO₂Me), 7.05 (1H, br s, C₆H₃), 7.49 (1H, br s, C₆H₃), 7.65 (1H, br s, C₆H₃)

### Intermediate 48: Methyl 3-(4-oxopiperidin-1-yl)-5-(trifluoromethyl)benzoate

3-Chloropropionyl chloride (3.8 g, 30 mmol) was placed in a reaction vessel equipped with a drying tube, and 70 ml of methylene chloride was added thereto. Under ice cooling, aluminum chloride (4.8 g, 36 mmol) was added to the contents of the reaction vessel. Subsequently, the contents of the reaction vessel were stirred for 2 hr while blowing ethylene gas into the reaction vessel. The reaction mixture was slowly poured into 300 ml of ice water. Concentrated hydrochloric acid (8.0 ml) was added thereto, and the mixture was extracted with 300 ml of methylene chloride. The organic layer was dried over anhydrous magnesium sulfate and was concentrated under the reduced pressure to give 1,5-dichloropentan-3-one. Intermediate 47 (4.3 g, 20 mmol) was added to this compound, and the mixture was dissolved in 200 ml of methanol. p-Toluenesulfonic acid monohydrate (4.6 g, 24 mmol) was added to the solution, and a reaction was allowed to proceed at 65°C for 7 hr, and the reaction mixture was then concentrated under the reduced pressure. A saturated aqueous sodium hydrogencarbonate solution (300 ml) was added to the residue, and the mixture was extracted twice with 200 ml of methylene chloride. The combined organic layers were washed with 300 ml of a saturated aqueous sodium hydrogencarbonate solution, were dried over anhydrous magnesium sulfate, and were then concentrated under the reduced pressure. Immediately after that, 70 ml of formic acid and 7.0 ml of water were added to the residue to prepare a solution. A reaction was allowed to proceed at room temperature for 2 hr, and the reaction mixture was then concentrated under the reduced pressure. A saturated aqueous sodium hydrogencarbonate solution (200 ml) was added to the residue, and the mixture was extracted twice with 200 ml of ethyl acetate. The combined organic layers were washed with 200 ml of a saturated aqueous sodium hydrogencarbonate solution, were dried over anhydrous magnesium sulfate, and were concentrated under the reduced pressure. The residue was purified by column chromatography on silica gel (development system: hexane : ethyl acetate = 2 : 1) to give the title compound (4.5 g, 76%).
Physicochemical properties of intermediate 48
(1) Color and form: Colorless syrup
(2) Molecular formula: C₁₄H₁₄NO₃F₃
(3) Mass spectrum (EIMS): m/z 301 (M)⁺
(4) ¹H NMR spectrum (400 MHz, CDCl₃) δ (ppm): 2.61 (4H, t, piperidone), 3.72 (4H, t, piperidone), 3.95 (3H, s, CO₂Me), 7.30 (1H, br s, C₆H₃), 7.46 (1H, br s, C₆H₃), 7.77 (1H, br s, C₆H₃)

### Intermediate 49: Methyl 3-(4-hydroxypiperidin-1-yl)-5-(trifluoromethyl)benzoate

Tetrahydrofuran (150 ml) was added to intermediate 48 (4.5 g, 15 mmol) to prepare a solution. Sodium boron hydride (601 mg, 16 mmol) was added to the solution at room temperature, and the mixture was stirred for 3.5 hr. Water (300 ml) was added thereto, and the mixture was extracted with 300 ml of ethyl acetate, followed by washing with 100 ml of saturated brine. The extract was dried over anhydrous magnesium sulfate and was concentrated under the reduced pressure to give the title compound (4.3 g, 94%).
Physicochemical properties of intermediate 49
(1) Color and form: Colorless syrup
(2) Molecular formula: C₁₄H₁₆NO₃F₃
(3) Mass spectrum (TSPMS): m/z 304 (M+H)⁺
(4) ¹H NMR spectrum (400 MHz, CDCl₃) δ (ppm): 1.70 (2H, dddd, piperidine), 1.99 - 2.07 (2H, m, piperidine), 3.07 (2H, ddd, piperidine), 3.66 (2H, ddd, piperidine), 3.89 - 3.96 (1H, m, piperidine), 3.94 (3H, s, CO₂Me), 7.28 (1H, br s, C₆H₃), 7.69 (1H, br s, C₆H₃), 7.74 (1H, br s, C₆H₃)

### Intermediate 50: Methyl 3-{4-(methanesulfonyloxy)-piperidin-1-yl}-5-(trifluoromethyl)benzoate

Methylene chloride (140 ml) was added to intermediate 49 (4.3 g, 14 mmol) to prepare a solution, and triethylamine (4.0 ml, 28 mmol) and methanesulfonyl chloride (1.3 ml, 17 mmol) were added in that order to the solution at room temperature. A reaction was allowed to proceed for 20 min, and 400 ml of water was then added to the reaction mixture, followed by extraction twice with 200 ml of methylene chloride. The combined residues were dried over anhydrous magnesium sulfate and were concentrated under the reduced pressure to give the title compound (5.1 g, 94%).
Physicochemical properties of intermediate 50
(1) Color and form: Light yellow syrup
(2) Molecular formula: C₁₅H₁₈NO₅F₃S
(3) Mass spectrum (TSPMS): m/z 382 (M+H)⁺
(4) ¹H NMR spectrum (400 MHz, CDCl₃) δ (ppm): 2.00 - 2.10 (2H, m, piperidine), 2.11 - 2.21 (2H, m, piperidine), 3.06 (3H, s, Ms), 3.22 (2H, ddd, piperidine), 3.56 (2H, ddd, piperidine), 3.94 (3H, s, CO₂Me), 4.95 (1H, dtt, piperidine), 7.28 (1H, br s, C₆H₃), 7.74 (2H, br s, C₆H₃)

### Intermediate 51: Methyl 3-(4-azidopiperidin-1-yl)-5-(trifluoromethyl)benzoate

Dimethylformamide (30 ml) was added to intermediate 50 (5.1 g, 13 mmol) to prepare a solution. Sodium azide (1.9 g, 29 mmol) was added to the solution, and the mixture was stirred at 80°C for 16 hr. The temperature of the reaction mixture was returned to room temperature, 400 ml of water was then added to the reaction mixture, and the mixture was extracted twice with 300 ml of ethyl acetate. The combined organic layers were washed twice with 400 ml of water and once with 400 ml of saturated brine, were then dried over anhydrous magnesium sulfate, and were concentrated under the reduced pressure. The residue was purified by column chromatography on silica gel (development system: hexane : ethyl acetate = 6 : 1) to give the title compound (2.8 g, 64%).
Physicochemical properties of intermediate 51
(1) Color and form: Light yellow solid
(2) Molecular formula: C₁₄H₁₅N₄O₂F₃
(3) Mass spectrum (TSPMS): m/z 329 (M+H)⁺
(4) ¹H NMR spectrum (400 MHz, CDCl₃) δ (ppm): 1.79 (2H, dddd, piperidine), 2.01 - 2.10 (2H, m, piperidine), 3.10 (2H, ddd, piperidine), 3.58 - 3.69 (3H, m, piperidine), 3.93 (3H, s, CO₂Me), 7.27 (1H, br s, C₆H₃), 7.72 (1H, br s, C₆H₃), 7.73 (1H, br s, C₆H₃)

### Intermediate 52: Methyl 3-(4-aminopiperidin-1-yl)-5-(trifluoromethyl)benzoate

Ethanol (60 ml) was added to intermediate 51 (1.8 g, 5.5 mmol) to prepare a solution, and 357 mg of 10% palladium-carbon was added to the solution. The mixture was stirred in a hydrogen atmosphere at room temperature for 9 hr. The insolubles were filtered and were washed with 100 ml of ethanol. The filtrate and the washings were combined followed by concentration under the reduced pressure. The residue was purified by column chromatography on silica gel (development system: methylene chloride : methanol : concentrated aqueous ammonia = 100 : 10 : 1) to give the title compound (1.0 g, 61%).
Physicochemical properties of intermediate 52
(1) Color and form: Colorless syrup
(2) Molecular formula: C₁₄H₁₇N₂O₂F₃
(3) Mass spectrum (TSPMS): m/z 303 (M+H)⁺
(4) ¹H NMR spectrum (400 MHz, CDCl₃) δ (ppm): 1.40 - 1.54 (2H, m, piperidine), 1.95 (2H, br d, piperidine), 2.85 - 2.95 (3H, m, piperidine), 3.72 - 3.79 (2H, m, piperidine), 3.93 (3H, s, CO₂Me), 7.27 (1H, br s, C₆H₃), 7.68 (1H, br s, C₆H₃), 7.73 (1H, br s, C₆H₃)

### Intermediate 53: Methyl 3-{4-(pyrimidin-2-ylamino)piperidin-1-yl}-5-(trifluoromethyl)benzoate

Dimethyl sulfoxide (6.6 ml) was added to intermediate 52 (1.0 g, 3.3 mmol) to prepare a solution, and diisopropylethylamine (3.2 ml, 18 mmol) and 2-bromopyrimidine (593 mg, 3.7 mmol) were added in that order to the solution. A reaction was allowed to proceed at 100°C for 9 hr, and the temperature of the reaction mixture was then returned to room temperature. Water (100 ml) was added thereto, and the mixture was extracted three times with 100 ml of ethyl acetate. The combined organic layers were washed three times with 200 ml of water and once with 200 ml of saturated brine, were dried over anhydrous magnesium sulfate, and were then concentrated under the reduced pressure. The residue was purified by column chromatography on silica gel (development system: hexane : ethyl acetate = 1 : 2) to give the title compound (1.1 g, 90%).
Physicochemical properties of intermediate 53
(1) Color and form: Light yellow solid
(2) Molecular formula: C₁₈H₁₉N₄O₂F₃
(3) Mass spectrum (TSPMS): m/z 381 (M+H)⁺
(4) ¹H NMR spectrum (400 MHz, CDCl₃) δ (ppm): 1.65 (2H, dddd, piperidine), 2.18 - 2.25 (2H, m, piperidine), 3.06 (2H, ddd, piperidine), 3.73 - 3.80 (2H, m, piperidine), 3.94 (3H, s, CO₂Me), 4.00 - 4.10 (1H, m, piperidine), 6.55 (1H, t, pyrimidine), 7.29 (1H, br s, C₆H₃), 7.71 (1H, br s, C₆H₃), 7.75 (1H, br s, C₆H₃), 8.29 (2H, d, pyrimidine)

### Intermediate 54: 3-{4-(Pyrimidin-2-ylamino)piperidin-1-yl}-5-(trifluoromethyl)benzoic acid

Tetrahydrofuran (7.5 ml) and methanol 2.5 ml were added in that order to intermediate 53 (259 mg, 0.68 mmol) to prepare a solution, and 2.5 ml of a 1.0 M aqueous sodium hydroxide solution was added to the solution. A reaction was allowed to proceed at 50°C for 4 hr. The temperature of the reaction mixture was then returned to room temperature, and the reaction mixture was adjusted to pH 4 by the addition of 1.0 M hydrochloric acid and was concentrated under the reduced pressure to bring the volume to about 5.0 ml. The precipitate was collected by filtration, washed with water, and was then dried to give the title compound (98 mg, 40%).
Physicochemical properties of intermediate 54
(1) Color and form: Colorless solid
(2) Molecular formula: C₁₇H₁₇N₄O₂F₃
(3) Mass spectrum (TSPMS): m/z 367 (M+H)⁺
(4) ¹H NMR spectrum (400 MHz, DMSO-d₆) δ (ppm): 1.50 - 1.63 (2H, m, piperidine), 1.95 (2H, br d, piperidine), 2.97 (2H, br t, piperidine), 3.86 (2H, br d, piperidine), 3.91 - 3.99 (1H, m, piperidine), 6.56 (1H, t, pyrimidine), 7.43 (1H, br s, C₆H₃), 7.49 (1H, br s, C₆H₃), 7.68 (1H, br s, C₆H₃), 8.27 (2H, d, pyrimidine)

### Example 25: t-Butyl (2S)-benzenesulfonylamino-3-[3-{4-(pyrimidin-2-ylamino)piperidin-1-yl}-5-(trifluoromethyl)benzoylamino]propionate

Dimethylformamide (2.0 ml) was added to intermediate 54 (65 mg, 0.18 mmol) to prepare a solution, and t-butyl (2S)-N-benzenesulfonyl-2,3-diaminopropionate (59 mg, 0.20 mmol) was added to the solution. Further, 1-hydroxybenzotriazole (38 mg, 0.27 mmol), N-methylmorpholine (97 µl, 0.89 mmol), and 1-ethyl-3-(3-dimethylaminopropyl)carbodiimide hydrochloride (78 mg, 0.41 mmol) were added thereto, and a reaction was allowed to proceed at room temperature for 12 hr. A saturated aqueous sodium hydrogencarbonate solution (10 ml) was added to stop the reaction, and 100 ml of water was added thereto. The mixture was extracted twice with 100 ml of ethyl acetate. The organic layers were combined, and the combined organic layers were washed twice with 100 ml of water and once with 100 ml of saturated brine, were dried over anhydrous magnesium sulfate, and were then concentrated under the reduced pressure. The residue was purified by column chromatography on silica gel (development system: hexane : ethyl acetate = 1 : 2) to give the title compound (97 mg, 85%).
Physicochemical properties of the compound prepared in Example 25
(1) Color and form: Colorless solid
(2) Molecular formula: C₃₀H₃₅N₆O₅SF₃
(3) Mass spectrum (TSPMS): m/z 649 (M+H)⁺
(4) Specific rotation: [α]_{D}²⁵ +45° (c 0.63, CHCl₃)
(5) ¹H NMR spectrum (400 MHz, CDCl₃) δ (ppm): 1.30 (9H, s, t-Bu), 1.49 - 1.74 (2H, m, piperidine), 2.20 (2H, br d, piperidine), 3.07 (2H, br t, piperidine), 3.57 (2H, ddd, CONHCH₂), 3.77 - 3.82 (2H, m, piperidine), 3.88 - 3.98 (2H, m, CONHCH₂CH), 4.00 - 4.09 (1H, m, piperidine), 6.55 (1H, t, pyrimidine), 7.22 (1H, br s, C₆H₃), 7.38 (1H, br s, C₆H₃), 7.46 - 7.52 (2H, m, C₆H₅), 7.54 - 7.60 (2H, m, C₆H₃ and C₆H₅), 7.83 - 7.87 (2H, m, C₆H₅), 8.29 (2H, d, pyrimidine)

### Example 26: (2S)-Benzenesulfonylamino-3-[3-{4-(pyrimidin-2-ylamino)piperidin-1-yl}-5-(trifluoromethyl)benzoylamino]propionic acid

Methylene chloride (4.0 ml) was added to the compound (91 mg, 0.14 mmol) prepared in Example 25 to prepare a solution, and 4.0 ml of trifluoroacetic acid was added to the solution at room temperature. A reaction was allowed to proceed for 4 hr, and reaction mixture was concentrated under the reduced pressure to give the title compound (96 mg, 73% (as tritrifluoroacetate)).
Physicochemical properties of the compound prepared in Example 26 (as tritrifluoroacetic acid)
(1) Color and form: Light yellow solid
(2) Molecular formula: C₂₆H₃₁N₆O₅SF₃
(3) Mass spectrum (TSPMS): m/z 597 (M+H)⁺

### Example 27: (2S)-Benzenesulfonylamino-3-[3-{4-(1,4,5,6-tetrahydropyrimidin-2-ylamino)piperidin-1-yl}-5-(trifluoromethyl)benzoylamino]propionic acid

1,4-Dioxane (6.0 ml) and 3.0 ml of water were added in that order to 96 mg of the compound prepared in Example 26 to prepare a solution. To the solution was added 43 mg of 10% palladium-carbon. The mixture was stirred in a hydrogen atmosphere at room temperature for 4.5 hr. The insolubles were filtered and were washed with 60 ml of a solvent having the same composition as the mixed solvent used in the reaction. The filtrate and the washings were combined followed by concentration under the reduced pressure, and the residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (67 mg, 100%).
Physicochemical properties of the compound prepared in Example 27
(1) Color and form: Colorless solid
(2) Molecular formula: C₂₆H₃₁N₆O₅SF₃
(3) Mass spectrum (FABMS): m/z 597 (M+H)⁺
(4) Specific rotation: [α]_{D}²⁵ +54° (c 0.50, CH₃OH)
(5) ¹H NMR spectrum (400 MHz, CDCl₃) δ (ppm): 1.69 (2H, dddd, piperidine), 1.96 (2H, dddd, tetrahydropyrimidine), 2.01 (2H, br d, piperidine), 2.98 (2H, br t, piperidine), 3.36 (4H, br t, tetrahydropyrimidine), 3.45 - 3.54 (1H, m, piperidine), 3.54 (1H, dd, CONHCH₂), 3.72 (1H, dd, CONHCH₂), 3.80 (1H, dd, CONHCH₂CH), 3.85 (2H, br d, piperidine), 7.29 (1H, br s, C₆H₃), 7.44 - 7.50 (3H, br s, C₆H₅ and C₆H₃), 7.50 - 7.56 (1H, m, C₆H₅), 7.64 (1H, br s, C₆H₃), 7.83 - 7.87 (2H, m, C₆H₅)

### Example 28: t-Butyl (2S)-(benzyloxycarbonyl)amino-3-[3-{4-(pyrimidin-2-ylamino)piperidin-1-yl}benzoylamino]-propionate

Dimethylformamide (10 ml) was added to intermediate 7 (201 mg, 0.67 mmol) to prepare a solution, and t-butyl (2S)-N-benzyloxycarbonyl-2,3-diaminopropionate (212 mg, 0.74 mmol) was added to the solution. Further, 1-hydroxybenzotriazole (142 mg, 1.0 mmol), N-methylmorpholine (370 µl, 3.4 mmol) and 1-ethyl-3-(3-dimethylaminopropyl)carbodiimide hydrochloride (270 mg, 1.4 mmol) were added thereto, and a reaction was allowed to proceed at room temperature for 13 hr. A saturated aqueous sodium hydrogencarbonate solution (20 ml) was added to stop the reaction, and 400 ml of water was added thereto. The mixture was extracted twice with 250 ml of ethyl acetate. The organic layers were combined, and the combined organic layers were washed with 400 ml of saturated brine, were dried over anhydrous magnesium sulfate, and were then concentrated under the reduced pressure. The residue was purified by column chromatography on silica gel (development system: methylene chloride : methanol = 20 : 1) to give the title compound (367 mg, 95%).
Physicochemical properties of the compound prepared in Example 28
(1) Color and form: Colorless solid
(2) Molecular formula: C₃₁H₃₈N₆O₅
(3) Mass spectrum (EIMS): m/z 574 (M)⁺
(4) Specific rotation: [α]_{D}²⁵ -2.4° (c 1.1, CH₂Cl₂)
(5) ¹H NMR spectrum (400 MHz, CDCl₃) δ (ppm): 1.47 (9H, s, t-Bu), 1.64 (2H, m, piperidine), 2.18 (2H, m, piperidine), 2.99 (2H, dd, piperidine), 3.72 (2H, br d, piperidine), 3.82 (2H, m, CONHCH₂), 4.01 (1H, m, piperidine), 4.46 (1H, m, CONHCH₂CH), 5.11 (2H, s, CO₂CH₂Ph), 6.54 (1H, t, pyrimidine), 7.07 (1H, dd, C₆H₄), 7.12 (1H, br d, C₆H₄), 7.28 - 7.35 (6H, m, C₆H₅ and C₆H₄), 7.42 (1H, br s, C₆H₄), 8.28 (2H, d, pyrimidine)

### Example 29: t-Butyl (2S)-amino-3-[3-{4-(pyrimidin-2-ylamino)piperidin-1-yl}benzoylamino]propionate

Tetrahydrofuran (60 ml) was added to the compound (230 mg, 0.40 mmol) prepared in Example 28 to prepare a solution. To the solution was added 226 mg of 10% palladium-carbon. The mixture was stirred in a hydrogen atmosphere at room temperature for 4 hr. The insolubles were filtered and were washed with 200 ml of tetrahydrofuran. The filtrate and the washings were combined followed by concentration under the reduced pressure. The residue was purified by column chromatography on silica gel (development system: methylene chloride : methanol = 10 : 1) to give the title compound (111 mg, 63%).
Physicochemical properties of the compound prepared in Example 29
(1) Color and form: Colorless solid
(2) Molecular formula: C₂₃H₃₂N₆O₃
(3) Mass spectrum (EIMS): m/z 440 (M)⁺
(4) Specific rotation: [α]_{D}²⁵ +6.5° (c 1.0, CH₂Cl₂)
(5) ¹H NMR spectrum (400 MHz, CDCl₃) δ (ppm): 1.48 (9H, s, t-Bu), 1.62 - 1.74 (2H, m, piperidine), 2.18 (2H, br d, piperidine), 2.99 (2H, br dd, piperidine), 3.48 (1H, ddd, CONHCH₂), 3.61 (1H, dd, CONHCH₂CH), 3.68 - 3.76 (2H, m, piperidine), 3.83 (1H, ddd, CONHCH₂), 3.96 - 4.06 (1H, m, piperidine), 6.54 (1H, t, pyrimidine), 7.07 (1H, dd, C₆H₄), 7.14 (1H, br d, C₆H₄), 7.29 (1H, dd, C₆H₄), 7.43 (1H, br s, C₆H₄), 8.28 (2H, d, pyrimidine)

### Example 30: t-Butyl (2S)-acetamido-3-[3-{4-(pyrimidin-2-ylamino)piperidin-1-yl}benzoylamino]propionate

Methylene chloride (10 ml) was added to the compound (101 mg, 0.25 mmol) prepared in Example 29 to prepare a solution. Triethylamine (70 µl, 0.50 mmol) and acetic anhydride (24 µl, 0.30 mmol) were added in that order to the solution at room temperature, and a reaction was allowed to proceed for one hr. Water (50 ml) was added thereto, and the mixture was extracted twice with 100 ml of methylene chloride. The extract was dried over anhydrous magnesium sulfate and was concentrated under the reduced pressure. The residue was purified by column chromatography on silica gel (development system: methylene chloride : methanol = 10 : 1) to give the title compound (94 mg, 77%).
Physicochemical properties of the compound prepared in Example 30
(1) Color and form: Colorless solid
(2) Molecular formula: C₂₅H₃₄N₆O₄
(3) Mass spectrum (EIMS): m/z 482 (M)⁺
(4) Specific rotation: [α]_{D}²⁵ -6.8° (c 1.0, CH₂Cl₂)
(5) ¹H NMR spectrum (400 MHz, CDCl₃) δ (ppm): 1.49 (9H, s, t-Bu), 1.60 - 1.72 (2H, m, piperidine), 2.05 (3H, s, Ac), 2.19 (2H, m, piperidine), 3.00 (2H, m, piperidine), 3.68 - 3.75 (3H, ddd, piperidine and CONHCH₂), 3.86 (1H, ddd, CONHCH₂), 4.01 (1H, m, piperidine), 4.67 (1H, ddd, CONHCH₂CH), 6.54 (1H, t, pyrimidine), 7.07 (1H, dd, C₆H₄), 7.08 (1H, m, C₆H₄), 7.30 (1H, dd, C₆H₄), 7.42 (1H, dd, C₆H₄), 8.27 (2H, d, pyrimidine)

### Example 31: (2S)-Acetamido-3-[3-{4-(pyrimidin-2-ylamino)piperidin-1-yl}benzoylamino]propionic acid

Methylene chloride (8.0 ml) was added to the compound (93 mg, 0.20 mmol) prepared in Example 30 to prepare a solution, and 4.0 ml of trifluoroacetic acid was added to the solution at room temperature. A reaction was allowed to proceed for 4 hr, and the reaction mixture was concentrated under the reduced pressure to give the title compound (91 mg, 84% (as tritrifluoroacetate)).
Physicochemical properties of the compound prepared in Example 31 (as tritrifluoroacetate)
(1) Color and form: Colorless solid
(2) Molecular formula: C₂₁H₂₆N₆O₄
(3) Mass spectrum (FABMS): m/z 427 (M+H)⁺
(4) Specific rotation: [α]_{D}²⁵ +0.16° (c 1.2, CH₃OH)
(5) ¹H NMR spectrum (400 MHz, DMSO-d₆) δ (ppm): 1.61 (2H, m, piperidine), 1.85 (3H, s, Ac), 1.96 (2H, br d, piperidine), 2.88 (2H, br dd, piperidine), 3.50 (1H, m, CONHCH₂), 3.61 (1H, ddd, CONHCH₂), 3.76 (2H, m, piperidine), 3.92 (1H, m, piperidine), 4.41 (1H, ddd, CONHCH₂CH), 6.58 (1H, t, pyrimidine), 7.15 (1H, br d, C₆H₄), 7.20 - 7.32 (2H, m, C₆H₄), 7.39 (1H, br s, C₆H₄), 8.29 (2H, d, pyrimidine)

### Example 32: (2S)-Acetamido-3-[3-{4-(1,4,5,6-tetrahydropyrimidin-2-ylamino)piperidin-1-yl}benzoylamino]-propionic acid

1,4-Dioxane (2.0 ml) and 0.20 ml of water were added in that order to 64 mg of the compound prepared in Example 31 to prepare a solution. To the solution was added 15 mg of 10% palladium-carbon. The mixture was stirred in a hydrogen atmosphere at room temperature for 4 hr. The insolubles were filtered and were washed with 100 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (29 mg, 56%).
Physicochemical properties of the compound prepared in Example 32
(1) Color and form: Colorless solid
(2) Molecular formula: C₂₁H₃₀N₆O₄
(3) Mass spectrum (ESIMS): m/z 431 (M+H)⁺
(4) Specific rotation: [α]_{D}²⁵ +11° (c 0.32, CH₃OH)
(5) ¹H NMR spectrum (400 MHz, CD₃OD) δ (ppm): 1.64 (2H, dddd, piperidine), 1.97 (3H, s, Ac), 1.92 - 2.05 (4H, m, piperidine and tetrahydropyrimidine), 2.90 (2H, br dd, piperidine), 3.37 (4H, br t, tetrahydropyrimidine), 3.48 (1H, m, piperidine), 3.67 (1H, dd, CONHCH₂), 3.70 - 3.78 (2H, m, piperidine), 3.74 (1H, dd, CONHCH₂), 4.48 (1H, dd, CONHCH₂CH), 7.12 (1H, m, C₆H₄), 7.23 (1H, br d, C₆H₄), 7.30 (1H, dd, C₆H₄), 7.38 (1H, br s, C₆H₄)

### Example 33: t-Butyl (2S)-{2-(morpholin-4-yl-acetyl)amino}-3-[3-{4-(pyrimidin-2-ylamino)piperidin-1-yl}benzoylamino]propionate

Dimethylformamide (10 ml) was added to morpholin-4-ylacetic acid (36 mg, 0.25 mmol) and the compound (110 mg, 0.25 mmol) prepared in Example 29 to prepare a solution. 1-Hydroxybenzotriazole (53 mg, 0.37 mmol), N-methylmorpholine (140 µl, 1.3 mmol), and 1-ethyl-3-(3-dimethylaminopropyl)carbodiimide hydrochloride (97 mg, 0.50 mmol) were further added to the solution, and a reaction was allowed to proceed at room temperature for 19 hr. A saturated aqueous sodium hydrogencarbonate solution (5.0 ml) was added to stop the reaction, and 100 ml of water was added thereto. The mixture was extracted twice with 100 ml of ethyl acetate. The combined organic layers were washed with 100 ml of saturated brine, were dried over anhydrous magnesium sulfate, and were then concentrated under the reduced pressure. The residue was purified by column chromatography on silica gel (development system: methylene chloride : methanol = 10 : 1) to give the title compound (94 mg, 66%).
Physicochemical properties of the compound prepared in Example 33
(1) Color and form: Colorless solid
(2) Molecular formula: C₂₉H₄₁N₇O₅
(3) Mass spectrum (EIMS): m/z 567 (M)⁺
(4) Specific rotation: [α]_{D}²⁵ -19° (c 1.1, CH₂Cl₂)
(5) ¹H NMR spectrum (400 MHz, CDCl₃) δ (ppm): 1.50 (9H, s, t-Bu), 1.65 (2H, m, piperidine), 2.18 (2H, m, piperidine), 2.55 (4H, m, piperidine and morpholine), 3.00 (2H, m, piperidine), 3.04 (2H, br d, COCH₂N), 3.74 (7H, m, morpholine and CONHCH₂), 3.91 (1H, ddd, CONHCH₂), 4.02 (1H, m, piperidine), 4.68 (1H, ddd, CONHCH₂CH), 6.55 (1H, t, pyrimidine), 7.07 (1H, m, C₆H₄), 7.15 (1H, br d, C₆H₄), 7.30 (1H, dd, C₆H₄), 7.44 (1H, br s, C₆H₄), 8.28 (2H, d, pyrimidine)

### Example 34: (2S)-{2-(Morpholin-4-yl-acetyl)amino}-3-[3-{4-(pyrimidin-2-ylamino)piperidin-1-yl}benzoylamino]propionic acid

Methylene chloride (7.0 ml) was added to the compound (91 mg, 0.16 mmol) prepared in Example 33 to prepare a solution, and 3.5 ml of trifluoroacetic acid was added to the solution at room temperature. A reaction was allowed to proceed for 4 hr, and the reaction mixture was concentrated under the reduced pressure to give the title compound (130 mg, 95% (as tritrifluoroacetate)).
Physicochemical properties of the compound prepared in Example 34 (as tritrifluoroacetate)
(1) Color and form: Colorless solid
(2) Molecular formula: C₂₅H₃₃N₇O₄
(3) Mass spectrum (FABMS): m/z 512 (M+H)⁺
(4) Specific rotation: [α]_{D}²⁵ +2.4° (c 1.1, CH₃OH)
(5) ¹H NMR spectrum (400 MHz, CD₃OD) δ (ppm): 1.80 (2H, dddd, piperidine), 2.18 (2H, m, piperidine), 3.05 (2H, ddd, piperidine), 3.31 (4H, m, morpholine), 3.76 (1H, dd, CONHCH₂), 3.80 - 3.93 (4H, m, morpholine), 3.81 (2H, br d, piperidine), 3.92 (1H, ddd, CONHCH₂), 3.98 (2H, d, COCH₂N), 4.07 (1H, tt, piperidine), 4.77 (1H, dd, CONHCH₂CH), 6.79 (1H, t, pyrimidine), 7.28 (1H, ddd, C₆H₄), 7.34 (1H, ddd, C₆H₄), 7.38 (1H, dd, C₆H₄), 7.53 (1H, dd, C₆H₄), 8.43 (2H, d, pyrimidine)

### Example 35: (2S)-{2-(Morpholin-4-yl-acetyl)amino}-3-[3-{4-(1,4,5,6-tetrahydropyrimidin-2-ylamino)piperidin-1-yl}benzoylamino]propionic acid

1,4-Dioxane (2.0 ml) and 0.20 ml of water were added in that order to 92 mg of the compound prepared in Example 34 to prepare a solution. To the solution was added 21 mg of 10% palladium-carbon. The mixture was stirred in a hydrogen atmosphere at room temperature for 5 hr. The insolubles were filtered and were washed with 66 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (42 mg, 72%).
Physicochemical properties of the compound prepared in Example 35
(1) Color and form: Colorless solid
(2) Molecular formula: C₂₅H₃₇N₇O₅
(3) Specific rotation: [α]_{D}²⁵ +2.2° (c 0.75, CH₃OH)
(4) ¹H NMR spectrum (400 MHz, DMSO-d₆) δ (ppm): 1.48 (2H, dddd, piperidine), 1.77 - 1.92 (4H, m, piperidine and tetrahydropyrimidine), 2.39 (4H, m, morpholine), 2.79 (2H, br dd, piperidine), 2.88 (2H, d, COCH₂N), 3.24 (4H, br dd, tetrahydropyrimidine), 3.40 - 3.51 (3H, m, piperidine and CONHCH₂), 3.55 (4H, br s, morpholine), 3.68 (2H, br dd, piperidine), 4.03 (1H, dd, CONHCH₂CH), 7.01 (1H, dd, C₆H₄), 7.13 (1H, d, C₆H₄), 7.25 (1H, dd, C₆H₄), 7.32 (1H, br s, C₆H₄)

### Example 36: t-Butyl 3-[3-{4-(pyrimidin-2-ylamino)-piperidin-1-yl}benzoylamino]-(2S)-{(2,4,6-trimethylbenzenesulfonyl)amino}propionate

Dimethylformamide (2.0 ml) was added to the compound (45 mg, 0.10 mmol) prepared in Example 29 to prepare a solution. Diisopropylethylamine (36 µl, 0.20 mmol) and 2,4,6-trimethylbenzenesulfonyl chloride (23 µl, 0.10 mmol) were added in that order to the solution at room temperature, and a reaction was allowed to proceed for 30 min. Piperazine was added to stop the reaction, and 20 ml of a saturated aqueous sodium hydrogencarbonate solution and 30 ml of water were added thereto. The mixture was extracted twice with 50 ml of ethyl acetate. The combined organic layers were washed twice with 50 ml of water and once with 50 ml of saturated brine, were dried over anhydrous magnesium sulfate, and were concentrated under the reduced pressure. The residue was purified by column chromatography on silica gel (development system: methylene chloride : methanol = 10 : 1) to give the title compound (61 mg, 96%).
Physicochemical properties of the compound prepared in Example 36
(1) Color and form: Colorless solid
(2) Molecular formula: C₃₂H₄₂N₆O₅S
(3) Mass spectrum (TSPMS): m/z 623 (M+H)⁺
(4) Specific rotation: [α]_{D}²⁵ +0.18° (c 1.3, CH₂Cl₂)
(5) ¹H NMR spectrum (400 MHz, CDCl₃) δ (ppm): 1.31 (9H, s, t-Bu), 1.55 - 1.70 (2H, m, piperidine), 2.14 (2H, br d, piperidine), 2.27 (3H, s, Me), 2.64 (6H, s, Me), 3.00 (2H, br dd, piperidine), 3.58 (1H, ddd, CONHCH₂), 3.70 - 3.78 (2H, m, piperidine), 3.81 (1H, ddd, CONHCH₂CH), 3.87 (1H, ddd, CONHCH₂), 3.98 - 4.08 (1H, m, piperidine), 6.54 (1H, t, pyrimidine), 6.93 (2H, s, C₆H₂), 7.07 (1H, dd, C₆H₄), 7.15 (1H, d, C₆H₄), 7.30 (1H, dd, C₆H₄), 7.43 (1H, dd, C₆H₄), 8.28 (2H, d, pyrimidine)

### Example 37: 3-[3-{4-(Pyrimidin-2-ylamino)piperidin-1-yl}benzoylamino]-(2S)-{(2,4,6-trimethylbenzenesulfonyl)-amino}propionic acid

Methylene chloride (4.0 ml) was added to the compound (60 mg, 0.097 mmol) prepared in Example 36 to prepare a solution. Trifluoroacetic acid (4.0 ml) was added to the solution at room temperature. A reaction was allowed to proceed for 4 hr, and the reaction mixture was concentrated under the reduced pressure to give the title compound (69 mg, 79% (as tritrifluoroacetate)).
Physicochemical properties of the compound prepared in Example 37 (as tritrifluoroacetate)
(1) Color and form: Colorless solid
(2) Molecular formula: C₂₈H₃₄N₆O₅S
(3) Mass spectrum (TSPMS): m/z 567 (M+H)⁺
(4) Specific rotation: [α]_{D}²⁵ +22° (c 1.0, CH₃OH)
(5) ¹H NMR spectrum (400 MHz, CD₃OD) δ (ppm): 1.89 (2H, dddd, piperidine), 2.20 (3H, s, Me), 2.23 (2H, br d, piperidine), 2.61 (6H, s, Me), 3.20 (2H, br dd, piperidine), 3.48 (1H, dd, CONHCH₂), 3.75 (1H, ddd, CONHCH₂), 3.83 (2H, br d, piperidine), 4.09 - 4.17 (2H, m, piperidine and CONHCH₂CH), 6.84 (1H, t, pyrimidine), 6.89 (2H, s, C₆H₂), 7.33 - 7.44 (3H, m, C₆H₄), 7.60 (1H, br s, C₆H₄), 8.47 (2H, d, pyrimidine)

### Example 38: 3-[3-{4-(1,4,5,6-Tetrahydropyrimidin-2-ylamino)piperidin-1-yl}benzoylamino]-(2S)-{(2,4,6-trimethylbenzenesulfonyl)amino}propionic acid

1,4-Dioxane (4.0 ml) and 2.0 ml of water were added in that order to 64 mg of the compound prepared in Example 37 to prepare a solution. To the solution was added 18 mg of 10% palladium-carbon. A reaction was allowed to proceed in a hydrogen atmosphere at room temperature for 5 hr. The insolubles were filtered and were washed with 60 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (31 mg, 55%).
Physicochemical properties of the compound prepared in Example 38
(1) Color and form: Colorless solid
(2) Molecular formula: C₂₈H₃₈N₆O₅S
(3) Mass spectrum (FABMS): m/z 571 (M+H)⁺
(4) Specific rotation: [α]_{D}²⁵ +75° (c 0.32, CH₃OH)
(5) ¹H NMR spectrum (400 MHz, CD₃OD) δ (ppm): 1.64 (2H, ddd, piperidine), 1.96 (2H, dddd, tetrahydropyrimidine), 2.01 (2H, br d, piperidine), 2.23 (3H, s, Me), 2.64 (6H, s, Me), 2.91 (2H, br dd, piperidine), 3.36 (4H, br t, tetrahydropyrimidine), 3.44 - 3.52 (1H, m, piperidine), 3.54 (1H, dd, CONHCH₂), 3.63 (1H, dd, CONHCH₂), 3.69 (1H, dd, CONHCH₂CH), 3.76 (2H, br d, piperidine), 6.94 (2H, s, C₆H₂), 7.12 (1H, ddd, C₆H₄), 7.23 (1H, ddd, C₆H₄), 7.30 (1H, dd, C₆H₄), 7.43 (1H, dd, C₆H₄)

### Example 39: t-Butyl (2S)-{(4-methoxybenzenesulfonyl)amino}-3-[3-{4-(pyrimidin-2-ylamino)piperidin-1-yl}benzoylamino]propionate

Dimethylformamide (3.0 ml) was added to the compound (61 mg, 0.14 mmol) prepared in Example 29 to prepare a solution. Diisopropylethylamine (48 µl, 0.28 mmol) and 4-methoxybenzenesulfonyl chloride (29 mg, 0.14 mmol) were added in that order to the solution at room temperature, and a reaction was allowed to proceed for one hr. Piperazine was added to stop the reaction, and 20 ml of a saturated aqueous sodium hydrogencarbonate solution and 30 ml of water were added thereto. The mixture was extracted twice with 50 ml of ethyl acetate. The combined organic layers were washed twice with 50 ml of water and once with 50 ml of saturated brine, were dried over anhydrous magnesium sulfate, and were concentrated under the reduced pressure to give the title compound (79 mg, 94%).
Physicochemical properties of the compound prepared in Example 39
(1) Color and form: Colorless solid
(2) Molecular formula: C₃₀H₃₈N₆O₆S
(3) Mass spectrum (TSPMS): m/z 611 (M+H)⁺
(4) Specific rotation: [α]_{D}²⁵ +40° (c 1.6, CH₂Cl₂)
(5) ¹H NMR spectrum (400 MHz, CDCl₃) δ (ppm): 1.32 (9H, s, t-Bu), 1.60 - 1.72 (2H, m, piperidine), 2.19 (2H, br d, piperidine), 3.00 (2H, br dd, piperidine), 3.51 - 3.61 (1H, m, CONHCH₂), 3.69 - 3.79 (2H, m, piperidine), 3.85 (3H, s, OMe), 3.86 - 3.96 (2H, m, CONHCH₂CH), 3.97 - 4.08 (1H, m, piperidine), 6.53 (1H, t, pyrimidine), 6.95 (2H, d, C₆H₄OMe), 7.07 (1H, dd, C₆H₄), 7.17 (1H, d, C₆H₄), 7.30 (1H, dd, C₆H₄), 7.43 (1H, dd, C₆H₄), 7.78 (2H, d, C₆H₄OMe), 8.28 (2H, d, pyrimidine)

### Example 40: (2S)-{(4-Methoxybenzenesulfonyl)amino}-3-[3-{4-(pyrimidin-2-ylamino)piperidin-1-yl}benzoylamino]propionic acid

Methylene chloride (3.0 ml) was added to the compound (32 mg, 0.052 mmol) prepared in Example 39 to prepare a solution, and 3.0 ml of trifluoroacetic acid was added to the solution at room temperature. A reaction was allowed to proceed for 4 hr, and the reaction mixture was concentrated under the reduced pressure to give the title compound (37 mg, 79% (as tritrifluoroacetate)).
Physicochemical properties of the compound prepared in Example 40 (as tritrifluoroacetate)
(1) Color and form: Light yellow solid
(2) Molecular formula: C₂₆H₃₀N₆O₆S
(3) Mass spectrum (TSPMS): m/z 555 (M+H)⁺
(4) Specific rotation: [α]_{D}²⁵ +19° (c 1.0, CH₃OH)
(5) ¹H NMR spectrum (400 MHz, CD₃OD) δ (ppm): 1.87 (2H, dddd, piperidine), 2.21 (2H, br d, piperidine), 3.19 (2H, br dd, piperidine), 3.48 (1H, dd, CONHCH₂), 3.66 (3H, s, OMe), 3.75 (1H, dd, CONHCH₂), 3.79 - 3.87 (2H, m, piperidine), 4.05 - 4.15 (1H, m, piperidine), 4.17 (1H, m, CONHCH₂CH), 6.81 (1H, t, pyrimidine), 6.93 (2H, d, C₆H₄OMe), 7.33 - 7.44 (3H, m, C₆H₄), 7.59 (1H, br s, C₆H₄), 7.75 (2H, d, C₆H₄OMe), 8.44 (2H, d, pyrimidine)

### Example 41: (2S)-{(4-Methoxybenzenesulfonyl)amino}-3-[3-{4-(1,4,5,6-tetrahydropyrimidin-2-ylamino)piperidin-1-yl}benzoylamino]propionic acid

1,4-Dioxane (3.0 ml) and 1.5 ml of water were added in that order to 35 mg of the compound prepared in Example 40 to prepare a solution, and 14 mg of 10% palladium-carbon was added to the solution. The mixture was stirred in a hydrogen atmosphere at room temperature for 4 hr. The insolubles were filtered and were washed with 60 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (8.7 mg, 22%).
Physicochemical properties of the compound prepared in Example 41
(1) Color and form: Colorless solid
(2) Molecular formula: C₂₆H₃₄N₆O₆S
(3) Mass spectrum (TSPMS): m/z 559 (M+H)⁺
(4) Specific rotation: [α]_{D}²⁵ +71° (c 0.30, CH₃OH)
(5) ¹H NMR spectrum (400 MHz, CD₃OD) δ (ppm): 1.58 - 1.70 (2H, m, piperidine), 1.96 (2H, dddd, tetrahydropyrimidine), 2.01 (2H, br d, piperidine), 2.92 (2H, ddd, piperidine), 3.36 (4H, br t, tetrahydropyrimidine), 3.44 - 3.52 (1H, m, piperidine), 3.54 (1H, dd, CONHCH₂), 3.65 - 3.76 (2H, m, CONHCH₂CH), 3.76 (2H, br d, piperidine), 3.82 (3H, s, OMe), 6.95 - 7.00 (2H, m, C₆H₄OMe), 7.13 (1H, ddd, C₆H₄), 7.24 (1H, ddd, C₆H₄), 7.30 (1H, dd, C₆H₄), 7.43 (1H, dd, C₆H₄), 7.76 - 7.80 (2H, m, C₆H₄OMe)

### Example 42: (2S)-{(4-Hydroxybenzenesulfonyl)amino}-3-[3-{4-(pyrimidin-2-ylamino)piperidin-1-yl}benzoylamino]propionic acid

1,2-Dichloroethane (7.0 ml) was added to the compound (44 mg, 0.072 mmol) prepared in Example 39 to prepare a solution, and a 1.0 M boron tribromidemethylene chloride solution (0.40 ml) was added to the solution. A reaction was allowed to proceed at 40°C for 3.5 hr, and 3.0 ml of 1,4-dioxane, 1.0 ml of water, and 1.0 ml of triethylamine were then added in that order thereto. The mixture was concentrated under the reduced pressure. 1,4-Dioxane (20 ml) was added to the residue, followed by filtration. The filtrate was then concentrated under the reduced pressure to give the title compound (28 mg, 71%).
Physicochemical properties of the compound prepared in Example 42
(1) Color and form: Colorless solid
(2) Molecular formula: C₂₅H₂₈N₆O₆S
(3) Mass spectrum (TSPMS): m/z 541 (M+H)⁺

### Example 43: (2S)-{(4-Hydroxybenzenesulfonyl)amino}-3-[3-{4-(1,4,5,6-tetrahydropyrimidin-2-ylamino)piperidin-1-yl}benzoylamino]propionic acid

1,4-Dioxane (20 ml) and 10 ml of water were added in that order to 28 mg of the compound prepared in Example 42 to prepare a solution, 24 mg of 10% palladium-carbon was added to the solution, and a reaction was allowed to proceed in a hydrogen atmosphere at room temperature for 6 hr. The insolubles were filtered and were washed with 60 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (11 mg, 37%).
Physicochemical properties of the compound prepared in Example 43
(1) Color and form: Colorless solid
(2) Molecular formula: C₂₅H₃₂N₆O₆S
(3) Mass spectrum (TSPMS): m/z 545 (M+H)⁺
(4) Specific rotation: [α]_{D}²⁵ +76° (c 0.28, CH₃OH)
(5) ¹H NMR spectrum (400 MHz, CD₃OD) δ (ppm): 1.58 - 1.70 (2H, m, piperidine), 1.96 (2H, dddd, tetrahydropyrimidine), 2.01 (2H, br d, piperidine), 2.87 - 2.98 (2H, m, piperidine), 3.36 (4H, br t, tetrahydropyrimidine), 3.43 - 3.53 (1H, m, piperidine), 3.53 (1H, dd, CONHCH₂), 3.66 - 3.74 (2H, m, CONHCH₂CH), 3.76 (2H, br d, piperidine), 6.81 - 6.85 (2H, m, C₆H₄OH), 7.13 (1H, ddd, C₆H₄), 7.25 (1H, ddd, C₆H₄), 7.31 (1H, dd, C₆H₄), 7.44 (1H, dd, C₆H₄), 7.66 - 7.71 (2H, m, C₆H₄OH)

### Intermediate 55: (3S)-Aminopiperidin-2-one

Methanol (770 ml) was added to L-ornithine hydrochloride (131 g, 0.78 mol) in an argon atmosphere to prepare a suspension, and thionyl chloride (170 ml, 2.0 mol) was added dropwise to the suspension at an internal temperature of -45°C over a period of 30 min or longer, followed by stirring for 30 min. The temperature of the reaction mixture was then returned to room temperature, and the reaction mixture was vigorously stirred for 19 hr and was concentrated under the reduced pressure. Water (500 ml) was then added to the residue to prepare a solution. The solution was subjected to column chromatography using a column packed with an Amberlite IRA-400 (OH⁻) anion exchange resin (1.1 kg), eluting with water to give the title compound as a crude product. Methanol (500 ml) was added to the crude product to prepare a solution, and the solution was slowly poured into 5.0 liters of chloroform. The suspension thus obtained was then filtered, and the filtrate was concentrated under the reduced pressure to give the title compound (81 g, 69%).
Physicochemical properties of intermediate 55
(1) Color and form: Colorless solid
(2) Molecular formula: C₅H₁₀N₂O
(3) Mass spectrum (EIMS): m/z 114 (M)⁺
(4) ¹H NMR spectrum (400 MHz, CD₃OD) δ (ppm): 1.48 (1H, m, piperidine), 1.72 (2H, m, piperidine), 1.99 (1H, dddd, piperidine), 3.16 (2H, dd, piperidine), 3.24 (1H, dd, piperidine)

### Intermediate 56: (3S)-Aminopiperidine

Tetrahydrofuran (100 ml) was added to aluminum lithium hydride (3.3 g, 87 mmol) to prepare a suspension, and intermediate 55 (4.1 g, 27 mmol) was added to the suspension under ice cooling. The temperature of the reaction mixture was returned to room temperature, and the reaction mixture was then stirred for 5.5 hr, and, while vigorus stirring, 3.3 ml of water, 3.3 ml of a 5.0 M aqueous sodium hydroxide solution, and 10 ml of water were added in that order to stop the reaction, followed by filtration. The filtrate was then dried over anhydrous magnesium sulfate. A 4.0 M hydrochloric acid-ethyl acetate solution (14 ml) was added thereto, and the mixture was concentrated under the reduced pressure. The residue was subjected to azeotropic distillation with methanol to give dihydrochloride of the title compound (5.1 g, 100%).
Physicochemical properties of intermediate 56
(1) Color and form: Brown solid
(2) Molecular formula: C₅H₁₂N₂
(3) Mass spectrum (EIMS): m/z 100 (M)⁺
(4) ¹H NMR spectrum (400 MHz, CD₃OD) (as dihydrochloride) δ (ppm): 1.75 (1H, dddd, piperidine), 1.90 (1H, m, piperidine), 2.09 (1H, ddddd, piperidine), 2.23 (1H, br d, piperidine), 3.02 (1H, ddd, piperidine), 3.09 (1H, dd, piperidine), 3.41 (1H, br d, piperidine), 3.62 (2H, m, piperidine)

### Intermediate 57: 3-{(3S)-Aminopiperidin-1-yl}benzonitrile

Intermediate 56 (1.3 g, 11 mmol), 3-fluorobenzonitrile (395 mg, 2.3 mmol), and sodium hydrogencarbonate (537 mg, 10 mmol) were placed in a pressure test tube, and 4.0 ml of dimethyl sulfoxide was added to prepare a suspension. The pressure test tube was hermetically sealed, and the suspension was stirred at 120°C for 23 hr. The temperature of the reaction mixture was returned to room temperature, and 500 ml of water was then added to the reaction mixture. The mixture was extracted three times with 300 ml of ethyl acetate. The combined organic layers were then extracted three times with 200 ml of 0.1 M hydrochloric acid. The combined aqueous layers were adjusted to pH 9 by the addition of sodium hydrogencarbonate, followed by extraction six times with 300 ml of ethyl acetate. The combined organic layers were dried over anhydrous magnesium sulfate and were concentrated under the reduced pressure to give the title compound (137 mg, 30%).
(1) Color and form: Light yellow syrup
(2) Molecular formula: C₁₂H₁₅N₃
(3) Mass spectrum (EIMS): m/z 201 (M)⁺
(4) ¹H NMR spectrum (400 MHz, CD₃OD) δ (ppm): 1.32 (1H, ddd, piperidine), 1.50 - 1.67 (1H, m, piperidine), 1.79 - 1.87 (1H, m, piperidine), 1.96 (1H, dddd, piperidine), 2.62 (1H, dd, piperidine), 2.82 (1H, ddd, piperidine), 2.88 (1H, dddd, piperidine), 3.53 (1H, ddd, piperidine), 3.65 (1H, dddd, piperidine), 7.05 (1H, ddd, C₆H₄), 7.20 - 7.25 (2H, m, C₆H₄), 7.33 (1H, ddd, C₆H₄)

### Intermediate 58: 3-{(3S)-(Pyrimidin-2-ylamino)piperidin-1-yl}benzonitrile

Dimethyl sulfoxide (6.0 ml) was added to intermediate 57 (137 mg, 0.68 mmol) to prepare a solution, and diisopropylethylamine (655 µl, 3.7 mmol) and 2-bromopyrimidine (122 mg, 0.77 mmol) were added in that order to the solution. A reaction was allowed to proceed at 120°C for 16.5 hr. The temperature of the reaction mixture was then returned to room temperature, and 300 ml of water was added to the reaction mixture. The mixture was extracted twice with 300 ml of ethyl acetate. The combined organic layers were washed twice with 200 ml of water and once with 300 ml of saturated brine. The extract was dried over anhydrous magnesium sulfate and was then concentrated under the reduced pressure. The residue was purified by column chromatography on silica gel (development system: hexane : ethyl acetate = 1 : 2) to give the title compound (144 mg, 75%).
Physicochemical properties of intermediate 58
(1) Color and form: Light yellow syrup
(2) Molecular formula: C₁₆H₁₇N₅
(3) Mass spectrum (EIMS): m/z 279 (M)⁺
(4) Specific rotation: [α]_{D}²⁵ +8.6° (c 0.67, CH₂Cl₂)
(5) ¹H NMR spectrum (400 MHz, CDCl₃) δ (ppm): 1.60 - 1.81 (2H, m, piperidine), 1.83 - 1.93 (1H, m, piperidine), 1.96 - 2.04 (1H, m, piperidine), 2.95 (1H, dd, piperidine), 3.11 (1H, dd, piperidine), 3.41 (1H, ddd, piperidine), 3.75 (1H, dd, piperidine), 4.15 (1H, ddddd, piperidine), 6.58 (1H, t, pyrimidine), 7.06 (1H, d, C₆H₄), 7.15 (1H, dd, C₆H₄), 7.24 (1H, br s, C₆H₄), 7.29 (1H, dd, C₆H₄), 8.32 (2H, d, pyrimidine)

### Intermediate 59: 3-{(3S)-(Pyrimidin-2-ylamino)piperidin-1-yl}benzoic acid

Tetrahydrofuran (6.0 ml) and 2.0 ml of methanol were added in that order to intermediate 58 (123 mg, 0.44 mmol) to prepare a solution, and 2.0 ml of a 5.0 M aqueous sodium hydroxide solution was added to the solution. The mixture was heated under reflux for 40 hr. The temperature of the reaction mixture was then returned to room temperature, and the reaction mixture was adjusted to pH 4 by the addition of 1.0 M hydrochloric acid. The precipitate was collected by filtration and was dried to give the title compound (79 mg, 60%).
Physicochemical properties of intermediate 59
(1) Color and form: Colorless solid
(2) Molecular formula: C₁₆H₁₈N₄O₂
(3) Mass spectrum (EIMS): m/z 298 (M)⁺
(4) ¹H NMR spectrum (400 MHz, DMSO-d₆) δ (ppm): 1.46 - 1.67 (2H, m, piperidine), 1.74 - 1.84 (1H, m, piperidine), 1.91 - 1.99 (1H, m, piperidine), 2.64 (1H, dd, piperidine), 2.77 (1H, br dd, piperidine), 3.67 (1H, br d, piperidine), 3.83 (1H, br d, piperidine), 3.86 - 3.97 (1H, m, piperidine), 6.58 (1H, t, pyrimidine), 7.13 (1H, d, C₆H₄), 7.19 (1H, ddd, C₆H₄), 7.30 (1H, dd, C₆H₄), 7.53 (1H, br s, C₆H₄), 8.29 (2H, d, pyrimidine)

### Example 44: t-Butyl (2S)-benzenesulfonylamino-3-[3-{(3S)-(pyrimidin-2-ylamino)piperidin-1-yl}benzoylamino]-propionate

Dimethylformamide (5.0 ml) was added to intermediate 59 (79 mg, 0.26 mmol) to prepare a solution. t-Butyl (2S)-N-benzenesulfonyl-2,3-diaminopropionate (79 mg, 0.26 mmol) was added to the solution. Further, 1-hydroxybenzotriazole (55 mg, 0.39 mmol), N-methylmorpholine (145 µl, 1.3 mmol), and 1-ethyl-3-(3-dimethylaminopropyl)carbodiimide hydrochloride (103 mg, 0.52 mmol) were added thereto, and a reaction was allowed to proceed at room temperature for 5.5 hr. A saturated aqueous sodium hydrogencarbonate solution (30 ml) was added to stop the reaction, and 100 ml of water was added thereto. The mixture was extracted twice with 100 ml of ethyl acetate. The combined organic layers were washed twice with 100 ml of water, and once with 100 ml of saturated brine, were dried over anhydrous magnesium sulfate, and were then concentrated under the reduced pressure. The residue was purified by column chromatography on silica gel (development system: benzene : ethyl acetate = 1 : 2) to give the title compound (130 mg, 85%).
Physicochemical properties of the compound prepared in Example 44
(1) Color and form: Colorless solid
(2) Molecular formula: C₂₉H₃₆N₆O₅S
(3) Mass spectrum (TSPMS): m/z 581 (M+H)⁺
(4) Specific rotation: [α]_{D}²⁵ +40° (c 0.54, CH₂Cl₂)
(5) ¹H NMR spectrum (400 MHz, CDCl₃) δ (ppm): 1.29 (9H, s, t-Bu), 1.62 - 1.71 (1H, m, piperidine), 1.71 - 1.81 (1H, m, piperidine), 1.85 - 2.00 (2H, m, piperidine), 3.02 (1H, dd, piperidine), 3.10 - 3.20 (1H, m, piperidine), 3.32 - 3.39 (1H, m, piperidine), 3.60 (1H, ddd, CONHCH₂), 3.67 (1H, dd, piperidine), 3.85 - 3.96 (2H, m, CONHCH₂CH), 4.16 - 4.25 (1H, m, piperidine), 6.53 (1H, t, pyrimidine), 7.10 (1H, dd, C₆H₄), 7.16 (1H, d, C₆H₄), 7.29 (1H, dd, C₆H₄), 7.44 (1H, dd, C₆H₄), 7.46 - 7.52 (2H, m, C₆H₅), 7.54 - 7.59 (1H, m, C₆H₅), 7.83 - 7.87 (2H, m, C₆H₅), 8.29 (2H, d, pyrimidine)

### Example 45: (2S)-Benzenesulfonylamino-3-[3-{(3S)-(pyrimidin-2-ylamino)piperidin-1-yl}benzoylamino]propionic acid

Methylene chloride (4.0 ml) was added to the compound (126 mg, 0.22 mmol) prepared in Example 44 to prepare a solution, and 4.0 ml of trifluoroacetic acid was added to the solution at room temperature. A reaction was allowed to proceed for 2 hr, and the reaction mixture was concentrated under the reduced pressure to give the title compound (148 mg, 78% (as tritrifluoroacetate)).
Physicochemical properties of the compound prepared in Example 45 (as tritrifluoroacetate)
(1) Color and form: Light yellow solid
(2) Molecular formula: C₂₅H₂₈N₆O₅S
(3) Mass spectrum (TSPMS): m/z 525 (M+H)⁺
(4) Specific rotation: [α]_{D}²⁵ +15° (c 0.76, CH₃OH)
(5) ¹H NMR spectrum (400 MHz, CD₃OD) δ (ppm): 1.68 - 1.90 (2H, m, piperidine), 1.92 - 2.02 (1H, m, piperidine), 2.02 - 2.11 (1H, m, piperidine), 3.05 - 3.17 (2H, m, piperidine), 3.46 (1H, dd, CONHCH₂), 3.55 (1H, ddd, piperidine), 3.72 - 3.82 (2H, m, piperidine and CONHCH₂), 4.19 (1H, dd, CONHCH₂CH), 4.26 (1H, ddddd, piperidine), 6.82 (1H, t, pyrimidine), 7.22 - 7.28 (2H, m, C₆H₄), 7.35 (1H, dd, C₆H₄), 7.41 - 7.47 (2H, m, C₆H₅), 7.48 - 7.55 (2H, dd, C₆H₅ and C₆H₄), 7.78 - 7.83 (2H, m, C₆H₅), 8.47 (2H, br s, pyrimidine)

### Example 46: (2S)-Benzenesulfonylamino-3-[3-{(3S)-(1,4,5,6-tetrahydropyrimidin-2-ylamino)piperidin-1-yl} benzoylamino]propionic acid

1,4-Dioxane (4.0 ml) and 2.0 ml of water were added in that order to 129 mg of the compound prepared in Example 45 to prepare a solution, 33 mg of 10% palladium-carbon was added to the solution, and the mixture was stirred in a hydrogen atmosphere at room temperature for 5 hr. The insolubles were filtered and were washed with 90 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (59 mg, 75%).
Physicochemical properties of the compound prepared in Example 46
(1) Color and form: Colorless solid
(2) Molecular formula: C₂₅H₃₂N₆O₅S
(3) Mass spectrum (TSPMS): m/z 529 (N+H)⁺
(4) Specific rotation: [α]_{D}²⁵ +66° (c 0.57, CH₃OH)
(5) ¹H NMR spectrum (400 MHz, CD₃OD) δ (ppm): 1.60 (1H, ddd, piperidine), 1.70 - 1.81 (1H, m, piperidine), 1.85 - 1.93 (4H, m, piperidine and tetrahydropyrimidine), 3.06 (1H, dd, piperidine), 3.10 - 3.15 (1H, m, piperidine), 3.28 (1H, m, piperidine), 3.33 (4H, br t, tetrahydropyrimidine), 3.48 (1H, dd, piperidine), 3.52 (1H, dd, CONHCH₂), 3.65 - 3.72 (1H, m, piperidine), 3.71 (1H, dd, CONHCH₂), 3.77 (1H, dd, CONHCH₂CH), 7.13 (1H, ddd, C₆H₄), 7.26 (1H, ddd, C₆H₄), 7.31 (1H, dd, C₆H₄), 7.46 (1H, dd, C₆H₄), 7.47 - 7.53 (2H, m, C₆H₅), 7.53 - 7.59 (1H, m, C₆H₅), 7.85 - 7.90 (2H, m, C₆H₅)

### Intermediate 60: (3R)-Aminopiperidin-2-one

Methanol (25 ml) was added to D-ornithine hydrochloride (4.0 g, 24 mmol) to prepare a suspension which was then cooled to -78°C. Thionyl chloride (5.1 ml, 59 mmol) was added dropwise to the cooled suspension in the internal temperature range from -78°C to -45°C over a period of 20 min, and, 15 min after the completion of the dropwise addition, the temperature of the mixture was raised to room temperature, followed by vigorous stirring for 13 hr. The solution was then concentrated under the reduced pressure, and the residue was dried by means of a vacuum pump for 3 hr. The amorphous material thus obtained was subjected to column chromatography using a column packed with an Amberlite IRA-400 (OH⁻) anion exchange resin (25 g), eluting with water to give the title compound as a crude product. This crude product was purified by column chromatography on silica gel (development system: dichloromethane : ethanol : water : aqueous ammonia = 8 : 8 : 1 : 1) to give the title compound (1.7 g, 63%).
Physicochemical properties of intermediate 60
(1) Color and form: Colorless solid
(2) Molecular formula: C₅H₁₀N₂O
(3) Mass spectrum (EIMS): m/z 114 (M)⁺
(4) ¹H NMR spectrum (400 MHz, CD₃OD) δ (ppm): 1.48 (1H, m, piperidine), 1.72 (2H, m, piperidine), 1.99 (1H, dddd, piperidine), 3.16 (2H, dd, piperidine), 3.24 (1H, dd, piperidine)

### Intermediate 61: (3R)-Aminopiperidine

Tetrahydrofuran (100 ml) was added to aluminum lithium hydride (0.92 g, 24.8 mmol) to prepare a suspension which was then cooled with ice. Intermediate 60 (1.1 g, 9.9 mmol) was slowly added to this cooled suspension in the internal temperature range of 5°C to 16°C, and, 10 min after the completion of the addition of the intermediate, the temperature of the mixture was raised to room temperature, followed by vigorous stirring for 3 hr. The mixture was cooled with ice, and 5.6 ml of a 5 M aqueous sodium hydroxide solution was added thereto. The temperature of the mixture was raised to room temperature, and the mixture was then vigorously stirred for 2.0 hr. Anhydrous sodium sulfate was then added thereto, and the mixture was stirred for additional 10 min and was filtered through Celite to remove sodium sulfate, followed by washing with tetrahydrofuran. A 4 M solution (5.0 ml, 20.0 mmol) of hydrochloric acid in ethyl acetate was added to the filtrate, and the solvent was removed by distillation under the reduced pressure. The residue was subjected to azeotropic distillation with methanol to give a dihydrochloride of the title compound (1.03 g, 60%).
Physicochemical properties of intermediate 61 (as dihydrochloride)
(1) Color and form: Colorless solid
(2) Molecular formula: C₅H₁₂N₂
(3) Mass spectrum (EIMS): m/z 100 (M)⁺
(4) ¹H NMR spectrum (400 MHz, CD₃OD) δ (ppm): 1.75 (1H, dddd, piperidine), 1.90 (1H, m, piperidine), 2.09 (1H, ddddd, piperidine), 2.23 (1H, br d, piperidine), 3.02 (1H, ddd, piperidine), 3.09 (1H, dd, piperidine), 3.41 (1H, br d, piperidine), 3.62 (2H, m, piperidine)

### Intermediate 62: 3-{(3R)-(Pyrimidin-2-ylamino)piperidin-1-yl}benzonitrile

Intermediate 61 (402 mg, 2.3 mmol), 3-fluorobenzonitrile (1.4 g, 12 mmol), and sodium hydrogencarbonate (584 mg, 7.0 mmol) were placed in a pressure test tube, and 4.6 ml of dimethyl sulfoxide was added to prepare a suspension. The pressure test tube was hermetically sealed, and the contents of the pressure test tube were stirred at 120°C for 20 hr. The temperature of the reaction mixture was returned to room temperature, and 100 ml of water was then added thereto. The mixture was extracted three times with 100 ml of ethyl acetate. The combined organic layers were then extracted three times with 50 ml of 1.0 M hydrochloric acid. The combined aqueous layers were adjusted to pH 10 by the addition of sodium hydrogencarbonate, and the mixture was extracted three times with 100 ml of ethyl acetate. The combined organic layers were dried over anhydrous magnesium sulfate and were concentrated under the reduced pressure to give 3-{(3R)-aminopiperidin-1-yl}benzonitrile (122 mg, 26%).

Subsequently, 6.0 ml of dimethyl sulfoxide was added thereto to prepare a solution, and diisopropylethylamine (582 µl, 3.3 mmol) and 2-bromopyrimidine (109 mg, 0.66 mmol) were added in that order to the solution. The mixture was stirred at 120°C for 7 hr, and the temperature of the reaction mixture was then returned to room temperature. Water (100 ml) was added to the reaction mixture, and the mixture was extracted twice with 100 ml of ethyl acetate. The combined organic layers were washed twice with 100 ml of water, were dried over anhydrous magnesium sulfate, and were then concentrated under the reduced pressure. The residue was purified by column chromatography on silica gel (development system: hexane : ethyl acetate = 1 : 1) to give the title compound (59 mg, 35%).
Physicochemical properties of intermediate 62
(1) Color and form: Light yellow syrup
(2) Molecular formula: C₁₆H₁₇N₅
(3) Mass spectrum (TSPMS): m/z 280 (M+H)⁺
(4) Specific rotation: [α]_{D}²⁵ -7.0° (c 0.65, CH₂Cl₂)
(5) ¹H NMR spectrum (400 MHz, CDCl₃) δ (ppm): 1.60 - 1.95 (3H, m, piperidine), 1.96 - 2.05 (1H, m, piperidine), 2.97 (1H, dd, piperidine), 3.11 (1H, ddd, piperidine), 3.37 - 3.45 (1H, m, piperidine), 3.74 (1H, dd, piperidine), 4.12 - 4.21 (1H, m, piperidine), 6.60 (1H, t, pyrimidine), 7.06 (1H, ddd, C₆H₄), 7.15 (1H, ddd, C₆H₄), 7.24 (1H, dd, C₆H₄), 7.29 (1H, dd, C₆H₄), 8.33 (2H, d, pyrimidine)

### Intermediate 63: 3-{(3R)-(Pyrimidin-2-ylamino)piperidin-1-yl}benzoic acid

To intermediate 62 (57 mg, 0.20 mmol) was added 4.0 ml of 50% sulfuric acid. The solution thus obtained was heated under reflux for 2 hr. The temperature of the reaction mixture was returned to room temperature, and the reaction mixture was adjusted to pH 4 by the addition of sodium hydrogencarbonate. The precipitate was collected by filtration and was dried to give the title compound (44 mg, 71%).
Physicochemical properties of intermediate 63
(1) Color and form: Colorless solid
(2) Molecular formula: C₁₆H₁₈N₄O₂
(3) Mass spectrum (TSPMS): m/z 299 (M+H)⁺
(4) ¹H NMR spectrum (400 MHz, DMSO-d₆) δ (ppm): 1.45 - 1.66 (2H, m, piperidine), 1.74 - 1.82 (1H, m, piperidine), 1.95 (1H, br d, piperidine), 2.63 (1H, dd, piperidine), 2.76 (1H, ddd, piperidine), 3.65 (1H, br d, piperidine), 3.81 (1H, br d, piperidine), 3.86 - 3.96 (1H, m, piperidine), 6.57 (1H, t, pyrimidine), 7.11 (1H, d, C₆H₄), 7.18 (1H, ddd, C₆H₄), 7.28 (1H, dd, C₆H₄), 7.52 (1H, br s, C₆H₄), 8.29 (2H, d, pyrimidine)

### Example 47: t-Butyl (2S)-benzenesulfonylamino-3-[3-{(3R)-(pyrimidin-2-ylamino)piperidin-1-yl}benzoylamino]propionate

Dimethylformamide (6.0 ml) was added to intermediate 63 (42 mg, 0.14 mmol) to prepare a solution, and t-butyl (2S)-N-benzenesulfonyl-2,3-diaminopropionate (43 mg, 0.14 mmol) was added to the solution. Further, 1-hydroxybenzotriazole (30 mg, 0.21 mmol), N-methylmorpholine (77 µl, 0.70 mmol), and 1-ethyl-3-(3-dimethylaminopropyl)carbodiimide hydrochloride (59 mg, 0.31 mmol) were added thereto, and the mixture was stirred at room temperature for 6 hr. A saturated aqueous sodium hydrogencarbonate solution (30 ml) was added to stop the reaction, and 100 ml of water was added thereto. The mixture was extracted twice with 100 ml of ethyl acetate. The combined organic layers were washed twice with 100 ml of water and once with 100 ml of saturated brine, were dried over anhydrous magnesium sulfate, and were then concentrated under the reduced pressure to give the title compound (80 mg, 100%).
Physicochemical properties of the compound prepared in Example 47
(1) Color and form: Colorless solid
(2) Molecular formula: C₂₉H₃₆N₆O₅S
(3) Mass spectrum (TSPMS): m/z 581 (M+H)⁺
(4) Specific rotation: [α]_{D}²⁵ +34° (c 0.64, CH₂Cl₂)
(5) ¹H NMR spectrum (400 MHz, CDCl₃) δ (ppm): 1.29 (9H, s, t-Bu), 1.63 - 1.72 (1H, m, piperidine), 1.73 - 1.82 (1H, m, piperidine), 1.86 - 2.00 (2H, m, piperidine), 3.03 (1H, dd, piperidine), 3.16 (1H, ddd, piperidine), 3.30 - 3.39 (1H, m, piperidine), 3.61 (1H, ddd, CONHCH₂), 3.67 (1H, dd, piperidine), 3.88 (1H, m, CONHCH₂), 3.90 - 3.96 (1H, m, CONHCH₂CH), 4.10 - 4.25 (1H, m, piperidine), 6.53 (1H, t, pyrimidine), 7.10 (1H, dd, C₆H₄), 7.17 (1H, d, C₆H₄), 7.29 (1H, dd, C₆H₄), 7.44 (1H, dd, C₆H₄), 7.46 - 7.52 (2H, m, C₆H₅), 7.53 - 7.59 (1H, m, C₆H₅), 7.83 - 7.87 (2H, m, C₆H₅), 8.29 (2H, d, pyrimidine)

### Example 48: (2S)-Benzenesulfonylamino-3-[3-{(3R)-(pyrimidin-2-ylamino)piperidin-1-yl}benzoylamino]propionic acid

Methylene chloride (5.0 ml) was added to the compound (76 mg, 0.13 mmol) prepared in Example 44 to prepare a solution, and 5.0 ml of trifluoroacetic acid was added to the solution at room temperature. A reaction was allowed to proceed for 7 hr, and the reaction mixture was concentrated under the reduced pressure. Methanol (2.0 ml) was then added to the residue to prepare a solution which was then added dropwise to 200 ml of diisopropyl ether. The precipitate was collected by filtration and was dried to give the title compound (65 mg, 50% (as tritrifluoroacetate)).
Physicochemical properties of the compound prepared in Example 45 (as tritrifluoroacetate)
(1) Color and form: Light yellow solid
(2) Molecular formula: C₂₅H₂₈N₆O₅S
(3) Mass spectrum (TSPMS): m/z 525 (M+H)⁺
(4) Specific rotation: [α]_{D}²⁵ +34° (c 0.50, CH₃OH)
(5) ¹H NMR spectrum (400 MHz, CD₃OD) δ (ppm): 1.59 - 1.70 (1H, m, piperidine), 1.73 - 1.85 (1H, m, piperidine), 1.87 - 1.96 (1H, m, piperidine), 1.98 - 2.05 (1H, m, piperidine), 2.89 (1H, dd, piperidine), 3.01 (1H, ddd, piperidine), 3.46 - 3.58 (2H, m, piperidine and CONHCH₂), 3.68 - 3.75 (1H, m, CONHCH₂), 3.80 (1H, br d, piperidine), 4.14 (1H, ddddd, piperidine), 4.19 (1H, dd, CONHCH₂CH), 6.65 (1H, t, pyrimidine), 7.16 - 7.20 (2H, m, C₆H₄), 7.29 (1H, dd, C₆H₄), 7.39 - 7.45 (3H, m, C₆H₅ and C₆H₄), 7.47 - 7.52 (1H, m, C₆H₅), 7.79 - 7.83 (2H, m, C₆H₅), 8.33 (2H, br d, pyrimidine)

### Example 49: (2S)-Benzenesulfonylamino-3-[3-{(3R)-(1,4,5,6-tetrahydropyrimidin-2-ylamino)piperidin-1-yl}benzoylamino]propionic acid

1,4-Dioxane (2.0 ml) and 1.0 ml of water were added in that order to 63 mg of the compound prepared in Example 45 to prepare a solution, 18 mg of 10% palladium-carbon was added to the solution, and the mixture was stirred in a hydrogen atmosphere at room temperature for 5 hr. The insolubles were filtered and were washed with 90 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (29 mg, 74%).
Physicochemical properties of the compound prepared in Example 49
(1) Color and form: Colorless solid
(2) Molecular formula: C₂₅H₃₂N₆O₅S
(3) Mass spectrum (TSPMS): m/z 529 (M+H)⁺
(4) Specific rotation: [α]_{D}²⁵ +72° (c 0.55, CH₃OH)
(5) ¹H NMR spectrum (400 MHz, CD₃OD) δ (ppm): 1.55 - 1.65 (1H, m, piperidine), 1.71 - 1.81 (1H, m, piperidine), 1.84 - 1.95 (4H, m, piperidine and tetrahydropyrimidine), 3.05 (1H, dd, piperidine), 3.08 - 3.18 (1H, m, piperidine), 3.25 - 3.38 (5H, m, piperidine and tetrahydropyrimidine), 3.42 - 3.54 (1H, m, piperidine), 3.54 (1H, dd, CONHCH₂), 3.65 - 3.77 (3H, m, piperidine and CONHCH₂CH), 7.13 (1H, ddd, C₆H₄), 7.27 (1H, ddd, C₆H₄), 7.32 (1H, dd, C₆H₄), 7.47 (1H, dd, C₆H₄), 7.48 - 7.53 (2H, m, C₆H₅), 7.54 - 7.60 (1H, m, C₆H₅), 7.85 - 7.90 (2H, m, C₆H₅)

### Intermediate 64: 3-{4-(Aminomethyl)piperidin-1-yl}benzonitrile

Dimethyl sulfoxide (10 ml) was added to 3-fluorobenzonitrile (967 mg, 8.0 mmol) and 4-(aminomethyl)piperidine (5.0 g, 44 mmol) to prepare a solution which was then stirred at 100°C for 30.5 hr. The temperature of the reaction mixture was returned to room temperature, 1.0 liter of water was then added to the reaction mixture, and the mixture was extracted twice with 400 ml of ethyl acetate. The combined organic layers were washed twice with 200 ml of water and once with 400 ml of saturated brine, were dried over anhydrous magnesium sulfate, and were then concentrated under the reduced pressure to give the title compound (39 mg, 38%).
Physicochemical properties of intermediate 64
(1) Color and form: Light yellow syrup
(2) Molecular formula: C₁₃H₁₇N₃
(3) Mass spectrum (TSPMS): m/z 216 (M+H)⁺
(4) ¹H NMR spectrum (400 MHz, CDCl₃) δ (ppm): 1.33 (2H, ddd, piperidine), 1.43 - 1.55 (1H, m, piperidine), 1.85 (2H, br d, piperidine), 2.64 (2H, d, NHCH₂), 2.77 (2H, ddd, piperidine), 3.73 (2H, br d, piperidine), 7.05 (1H, ddd, C₆H₄), 7.09 - 7.14 (2H, m, C₆H₄), 7.29 (1H, dd, C₆H₄)

### Intermediate 65: 3-{4-(Pyrimidin-2-ylaminomethyl)piperidin-1-yl}benzonitrile

Dimethyl sulfoxide (10 ml) was added to intermediate 64 (214 mg, 0.99 mmol) to prepare a solution. Diisopropylethylamine (220 µl, 1.3 mmol) and 2-bromopyrimidine (241 mg, 1.5 mmol) were added in that order to the solution. A reaction was allowed to proceed at 120°C for 12.5 hr, and the temperature of the reaction mixture was then returned to room temperature. Water (1.0 liter) was added to the reaction mixture, and the mixture was extracted twice with 400 ml of ethyl acetate. The combined organic layers were washed twice with 300 ml of water and once with 500 ml of saturated brine, were dried over anhydrous magnesium sulfate, and were then concentrated under the reduced pressure. The residue was purified by column chromatography on silica gel (development system: hexane : ethyl acetate = 1 : 4) to give the title compound (156 mg, 54%).
Physicochemical properties of intermediate 65
(1) Color and form: Light yellow syrup
(2) Molecular formula: C₁₇H₁₉N₅
(3) Mass spectrum (TSPMS): m/z 294 (M+H)⁺
(4) ¹H NMR spectrum (400 MHz, CDCl₃) δ (ppm): 1.41 (2H, dddd, piperidine), 1.76 - 1.87 (1H, m, piperidine), 1.86 - 1.94 (2H, m, piperidine), 2.78 (2H, ddd, piperidine), 3.38 (2H, dd, NHCH₂), 3.69 - 3.76 (2H, m, piperidine), 6.54 (1H, t, pyrimidine), 7.05 (1H, ddd, C₆H₄), 7.09 - 7.13 (2H, m, C₆H₄), 7.29 (1H, m, C₆H₄), 8.28 (1H, d, pyrimidine)

### Intermediate 66: 3-{4-(Pyrimidin-2-ylaminomethyl)piperidin-1-yl}benzoic acid

Tetrahydrofuran (3.0 ml) and 1.0 ml of methanol were added in that order to intermediate 65 (51 mg, 0.17 mmol) to prepare a solution, and 1.0 ml of a 1.0 M aqueous sodium hydroxide solution was added to the solution. The mixture was heated under reflux for 23 hr, the temperature of the reaction mixture was returned to room temperature, and the reaction mixture was then adjusted to pH 4 by the addition of 1.0 M hydrochloric acid. The precipitate was collected by filtration and was dried to give the title compound (44 mg, 80%).
Physicochemical properties of intermediate 66
(1) Color and form: Colorless solid
(2) Molecular formula: C₁₇H₂₀N₄O₂
(3) Mass spectrum (TSPMS): m/z 313 (M+H)⁺
(4) ¹H NMR spectrum (400 MHz, DMSO-d₆) δ (ppm): 1.20 - 1.30 (2H, m, piperidine), 1.74 - 1.83 (3H, m, piperidine), 2.67 (2H, br dd, piperidine), 3.38 (2H, m, NHCH₂), 3.72 (2H, br d, piperidine), 6.52 (1H, t, pyrimidine), 7.15 - 7.25 (2H, m, C₆H₄), 7.29 (1H, dd, C₆H₄), 7.44 (1H, br s, C₆H₄), 8.24 (1H, d, pyrimidine)

### Example 50: t-Butyl (2S)-benzenesulfonylamino-3-[3-{4-(pyrimidin-2-ylaminomethyl)piperidin-1-yl}benzoylamino]propionate

Dimethylformamide (3.5 ml) was added to intermediate 66 (44 mg, 0.14 mmol) to prepare a solution, and t-butyl (2S)-N-benzenesulfonyl-2,3-diaminopropionate (66 mg, 0.22 mmol) was added to the solution. Further, 1-hydroxybenzotriazole (36 mg, 0.26 mmol), N-methylmorpholine (96 µl, 0.87 mmol), and 1-ethyl-3-(3-dimethylaminopropyl)carbodiimide hydrochloride (67 mg, 0.35 mmol) were added thereto, and a reaction was allowed to proceed at room temperature for 16 hr. A saturated aqueous sodium hydrogencarbonate solution (30 ml) was added to stop the reaction, and 100 ml of water was added thereto. The mixture was extracted twice with 100 ml of ethyl acetate. The combined organic layers were washed twice with 100 ml of water and once with 100 ml of saturated brine, were dried over anhydrous magnesium sulfate, and were then concentrated under the reduced pressure. The residue was purified by column chromatography on silica gel (development system: methylene chloride : methanol = 10 : 1) to give the title compound (43 mg, 52%).
Physicochemical properties of the compound prepared in Example 50
(1) Color and form: Colorless solid
(2) Molecular formula: C₃₀H₃₈N₆O₅S
(3) Mass spectrum (TSPMS): m/z 595 (M+H)⁺
(4) Specific rotation: [α]_{D}²⁵ +33° (c 1.5, CH₂Cl₂)
(5) ¹H NMR spectrum (400 MHz, CDCl₃) δ (ppm): 1.29 (9H, s, t-Bu), 1.37 - 1.50 (2H, m, piperidine), 1.73 - 1.85 (1H, m, piperidine), 1.90 (2H, br d, piperidine), 2.77 (2H, br dd, piperidine), 3.37 (2H, dd, NHCH₂), 3.53 - 3.62 (1H, m, CONHCH₂), 3.78 (2H, br d, piperidine), 3.85 - 3.95 (2H, m, CONHCH₂CH), 6.52 (1H, t, pyrimidine), 7.05 (1H, d, C₆H₄), 7.14 (1H, d, C₆H₄), 7.28 (1H, dd, C₆H₄), 7.40 (1H, br s, C₆H₄), 7.46 - 7.60 (3H, m, C₆H₅), 7.83 - 7.88 (2H, m, C₆H₅), 8.27 (2H, d, pyrimidine)

### Example 51: (2S)-Benzenesulfonylamino-3-[3-{4-(pyrimidin-2-ylaminomethyl)piperidin-1-yl}benzoylamino]propionic acid

Methylene chloride (1.0 ml) was added to the compound (43 mg, 0.073 mmol) prepared in Example 50 to prepare a solution, and 1.0 ml of trifluoroacetic acid was added to the solution at room temperature. A reaction was allowed to proceed for 4 hr, and the reaction mixture was concentrated under the reduced pressure to give the title compound (51 mg, 80% (as tritrifluoroacetate)).
Physicochemical properties of the compound prepared in Example 51 (as tritrifluoroacetate)
(1) Color and form: Light yellow solid
(2) Molecular formula: C₂₆H₃₀N₆O₅S
(3) Mass spectrum (TSPMS): m/z 539 (M+H)⁺
(4) Specific rotation: [α]_{D}²⁵ +18° (c 0.55, CH₃OH)
(5) ¹H NMR spectrum (400 MHz, CD₃OD) δ (ppm): 1.56 - 1.70 (2H, m, piperidine), 1.98 - 2.13 (3H, m, piperidine), 3.21 - 3.29 (2H, m, piperidine), 3.44 (2H, d, NHCH₂), 3.48 (1H, dd, CONHCH₂), 3.78 (1H, dd, CONHCH₂), 3.80 (2H, br d, piperidine), 4.22 (1H, dd, CONHCH₂CH), 6.75 (1H, t, pyrimidine), 7.43 - 7.60 (7H, m, C₆H₅ and C₆H₄), 7.81 - 7.86 (2H, m, C₆H₅), 8.39 (2H, d, pyrimidine)

### Example 52: (2S)-Benzenesulfonylamino-3-[3-{4-(1,4,5,6-tetrahydropyrimidin-2-ylaminomethyl)piperidin-1-yl}benzoylamino]propionic acid

1,4-Dioxane (2.0 ml) and 1.0 ml of water were added in that order to 48 mg of the compound prepared in Example 51 to prepare a solution, and 12 mg of 10% palladium-carbon was added to the solution. The mixture was stirred in a hydrogen atmosphere at room temperature for 7 hr. The insolubles were filtered and were washed with 60 ml of a solvent having the same composition as the mixed solvent used in the reaction. The filtrate and the washings were combined followed by concentration under the reduced pressure. The residue was purified by column chromatography on silica gel (development system: methylene chloride : ethanol : water : concentrated aqueous ammonia = 8 : 8 : 1 : 1) and was then purified by Sephadex LH-20 (methanol) to give the title compound (17 mg, 58%).
Physicochemical properties of the compound prepared in Example 52
(1) Color and form: Colorless solid
(2) Molecular formula: C₂₆H₃₄N₆O₅S
(3) Mass spectrum (TSPMS): m/z 543 (M+H)⁺
(4) Specific rotation: [α]_{D}²⁵ +66° (c 0.32, CH₃OH)
(5) ¹H NMR spectrum (400 MHz, CD₃OD) δ (ppm): 1.37 (2H, ddd, piperidine), 1.65 - 1.76 (1H, m, piperidine), 1.81 (2H, br d, piperidine), 1.94 (2H, dddd, tetrahydropyrimidine), 2.75 (2H, ddd, piperidine), 3.03 (2H, d, NHCH₂), 3.36 (4H, br t, tetrahydropyrimidine), 3.55 (1H, dd, CONHCH₂), 3.69 (1H, dd, CONHCH₂), 3.75 (1H, dd, CONHCH₂CH), 3.80 (2H, br d, piperidine), 7.11 (1H, ddd, C₆H₄), 7.22 (1H, ddd, C₆H₄), 7.29 (1H, dd, C₆H₄), 7.43 (1H, dd, C₆H₄), 7.46 - 7.52 (2H, m, C₆H₅), 7.52 - 7.58 (1H, m, C₆H₅), 7.84 - 7.89 (2H, m, C₆H₅)

### Intermediate 67: Ethyl 3-{(3S)-acetoxy-(2S)-azidomethylpyrrolidin-1-yl}benzoate

Methylene chloride (10 ml) was added to intermediate 17 (1.1 g, 3.4 mmol) to prepare a solution, triethylamine (960 µl, 6.9 mmol) and methanesulfonyl chloride (320 µl, 4.1 mmol) were added to the solution at room temperature, and a reaction was allowed to proceed for 10 min. Water (200 ml) was added to the reaction mixture, and the mixture was extracted twice with 200 ml of methylene chloride. The combined organic layers were dried over anhydrous magnesium sulfate, were concentrated under the reduced pressure to give ethyl 3-{(4S)-acetoxy-(3R)-methanesulfonyloxy}piperidin-1-yl}benzoate (1.2 g, 94%).

Dimethylformamide (35 ml) was added to the ethyl 3-{(4S)-acetoxy-(3R)-methanesulfonyloxy}piperidin-1-yl}benzoate (1.2 g, 3.2 mmol) thus obtained to prepare a solution, sodium azide (451 mg, 6.8 mmol) was added to the solution, and a reaction was allowed to proceed at 100°C for 20.5 hr. The temperature of the reaction mixture was returned to room temperature, 1.0 liter of water was then added thereto, and the mixture was extracted twice with 500 ml of ethyl acetate. The combined organic layers were washed twice with 750 ml of water and once with 500 ml of saturated brine, were then dried over anhydrous magnesium sulfate, and were concentrated under the reduced pressure. The residue was then purified by column chromatography on silica gel (development system: hexane : ethyl acetate = 4 : 1) to give the title compound (902 mg, 84%).
Physicochemical properties of intermediate 67
(1) Color and form: Colorless syrup
(2) Molecular formula: C₁₆H₂₀N₄O₄
(3) Mass spectrum (TSPMS): m/z 333 (M+H)⁺
(4) Specific rotation: [α]_{D}²⁵ +14° (c 1.0, CH₂Cl₂)
(5) ¹H NMR spectrum (400 MHz, CDCl₃) δ (ppm): 1.39 (3H, t, Et), 2.16 (3H, s, Ac), 2.27 (1H, dddd, pyrrolidine), 2.45 (1H, dddd, pyrrolidine), 3.27 (1H, ddd, pyrrolidine), 3.35 (1H, dd, NHCH₂), 3.62 (1H, dd, NHCH₂), 3.64 (1H, ddd, pyrrolidine), 4.20 (1H, ddd, pyrrolidine), 4.37 (2H, q, Et), 5.34 (1H, ddd, pyrrolidine), 6.79 (1H, ddd, C₆H₄), 7.29 (1H, dd, C₆H₄), 7.30 (1H, dd, C₆H₄), 7.43 (1H, ddd, C₆H₄)

### Intermediate 68: Ethyl 3-{(2S)-aminomethyl-(3S)-hydroxypyrrolidin-1-yl}benzoate

Tetrahydrofuran (7.5 ml) was added to intermediate 67 (247 mg, 0.74 mmol) to prepare a solution, sodium ethoxide (65 mg, 0.89 mmol) was added to the solution, and a reaction was allowed to proceed at 30°C for 3.5 hr. The reaction mixture was adjusted to pH 4 by the addition of 1.0 M hydrochloric acid, and 100 ml of water was added thereto. The mixture was extracted twice with 100 ml of ethyl acetate, and the combined organic layers were then dried over anhydrous magnesium sulfate and were concentrated under the reduced pressure. The residue was purified by column chromatography on silica gel (development system: hexane : ethyl acetate = 2 : 1) to give ethyl 3-{(2S)-azidomethyl-(3S)-hydroxypyrrolidin-1-yl}benzoate (146 mg, 68%).

1,4-Dioxane (4.0 ml) and 2.0 ml of water were added in that order to the ethyl 3-{(2S)-azidomethyl-(3S)-hydroxypyrrolidin-1-yl}benzoate (146 mg, 0.50 mmol) thus obtained to prepare a solution, 39 mg of 10% palladium-carbon was added to the solution, and the mixture was stirred in a hydrogen atmosphere at room temperature for 4.5 hr. The insolubles were filtered and were washed with 90 ml of a solvent having the same composition as the mixed solvent used in the reaction. The filtrate and the washings were combined followed by concentration under the reduced pressure to give the title compound (141 mg, 100%).
Physicochemical properties of intermediate 68
(1) Color and form: Colorless solid
(2) Molecular formula: C₁₄H₂₀N₂O₃
(3) Mass spectrum (EIMS): m/z 264 (M+H)⁺
(4) ¹H NMR spectrum (400 MHz, CDCl₃) δ (ppm): 1.39 (3H, t, Et), 2.05 - 2.15 (1H, m, pyrrolidine), 2.23 (1H, dddd, pyrrolidine), 3.09 - 3.33 (3H, m, pyrrolidine and NHCH₂), 3.60 - 3.69 (1H, m, pyrrolidine), 3.78 - 3.86 (1H, m, pyrrolidine), 4.37 (2H, q, Et), 4.61 (1H, ddd, pyrrolidine), 6.80 (1H, br d, C₆H₄), 7.27 - 7.33 (2H, m, C₆H₄), 7.38 (1H, d, C₆H₄)

### Example 53: t-Butyl (2S)-benzenesulfonylamino-3-[3-{(3S)-hydroxy-(2S)-(pyrimidin-2-ylaminomethyl)pyrrolidin-1-yl}benzoylamino]propionate

Dimethyl sulfoxide (5.0 ml) was added to intermediate 68 (141 mg, 0.53 mmol) to prepare a solution, 2-bromopyrimidine (82 mg, 0.52 mmol) and diisopropylethylamine (510 µl, 2.9 mmol) were added in that order to the solution, and a reaction was allowed to proceed at 120°C for 5 hr. The temperature of the reaction mixture was returned to room temperature, 500 ml of water was then added to the reaction mixture, and the mixture was extracted twice with 300 ml of ethyl acetate. The combined organic layers were washed with 500 ml of water and 500 ml of saturated brine, were then dried over anhydrous magnesium sulfate, and were concentrated under the reduced pressure. The residue was purified by column chromatography on silica gel (development system: methylene chloride : methanol = 20 : 1) to give ethyl 3-{(3S)-hydroxy-(2S)-(pyrimidin-2-ylaminomethyl)-pyrrolidin-1-yl}benzoate (111 mg, 61%).

Tetrahydrofuran (3.0 ml) and 1.0 ml of methanol were added in that order to the 3-{(3S)-hydroxy-(2S)-(pyrimidin-2-ylaminomethyl)pyrrolidin-1-yl}benzoic acid (111 mg, 0.33 mmol) thus obtained to prepare a solution, and 1.0 ml of a 1.0 M aqueous sodium hydroxide solution was added to the solution. A reaction was allowed to proceed at 50°C for one hr, and the temperature of the reaction mixture was then returned to room temperature. The reaction mixture was adjusted to pH 4 by the addition of 1.0 M hydrochloric acid, and 100 ml of water was added thereto. The mixture was extracted twice with 100 ml of ethyl acetate. The combined organic layers were dried over anhydrous magnesium sulfate, were then concentrated under the reduced pressure to give 3-{(3S)-hydroxy-(2S)-(pyrimidin-2-ylaminomethyl)pyrrolidin-1-yl}benzoic acid (86 mg, 84%).

Dimethylformamide (12 ml) was added to the 3-{(3S)-hydroxy-(2S)-(pyrimidin-2-ylaminomethyl)-pyrrolidin-1-yl}benzoic acid (86 mg, 0.27 mmol) thus obtained to prepare a solution. Benzotriazol-1-yloxytri(dimethylamino)phosphonium hexafluorophosphate (217 mg, 0.49 mmol) and diisopropylethylamine (216 µl, 1.3 mmol) were added to the solution, and a reaction was allowed to proceed at room temperature for one hr. t-Butyl (2S)-N-benzenesulfonyl-2,3-diaminopropionate (101 mg, 0.33 mmol) was added to the above active ester solution at room temperature. A reaction was allowed to proceed at room temperature for 15 min, and 20 ml of water was then added to the reaction mixture. The precipitate was collected by filtration, and ethyl acetate was added thereto to prepare a solution. The solution was dried over anhydrous magnesium sulfate and was then concentrated under the reduced pressure. The residue was purified by column chromatography on silica gel (development system: methylene chloride : methanol : benzene : ethyl acetate = 9 : 1 : 6 : 4) to give the title compound (56 mg, 35%).
Physicochemical properties of the compound prepared in Example 53
(1) Color and form: Colorless solid
(2) Molecular formula: C₂₉H₃₆N₆O₆S
(3) Mass spectrum (TSPMS): m/z 597 (M+H)⁺
(4) Specific rotation: [α]_{D}²⁵ +57° (c 0.55, CH₂Cl₂)
(5) ¹H NMR spectrum (400 MHz, CDCl₃) δ (ppm): 1.28 (9H, s, t-Bu), 1.90 - 2.00 (1H, m, pyrrolidine), 2.11 - 2.22 (1H, m, pyrrolidine), 3.37 (1H, ddd, pyrrolidine), 3.55 (1H, dd, NHCH₂), 3.66 (1H, ddd, pyrrolidine), 3.78 - 3.85 (3H, m, pyrrolidine and CONHCH₂), 3.89 (1H, dd, NHCH₂), 4.00 (1H, dd, CONHCH₂CH), 4.46 (1H, ddd, pyrrolidine), 6.59 (1H, t, pyrimidine), 6.81 (1H, dd, C₆H₄), 7.08 (1H, br s, C₆H₄), 7.14 (1H, d, C₆H₄), 7.31 (1H, dd, C₆H₄), 7.48 - 7.54 (2H, m, C₆H₅), 7.56 - 7.61 (1H, m, C₆H₅), 7.87 - 7.92 (2H, m, C₆H₅), 8.34 (2H, d, pyrimidine)

### Example 54: (2S)-Benzenesulfonylamino-3-[3-{(3S)-hydroxy-(2S)-(pyrimidin-2-ylaminomethyl)pyrrolidin-1-yl}benzoylamino]propionic acid

Methylene chloride (3.0 ml) was added to the compound (54 mg, 0.091 mmol) prepared in Example 53 to prepare a solution, and 3.0 ml of trifluoroacetic acid was added to the solution at room temperature. A reaction was allowed to proceed for 5 hr, and the reaction mixture was concentrated under the reduced pressure. The residue was purified by column chromatography on silica gel (development system: methylene chloride : methanol : concentrated aqueous ammonia = 100 : 10 : 1) and was then purified by Sephadex LH-20 (methanol) to give the title compound (13 mg, 26%).
Physicochemical properties of the compound prepared in Example 54
(1) Color and form: Colorless solid
(2) Molecular formula: C₂₅H₂₈N₆O₆S
(3) Mass spectrum (FABMS): m/z 541 (M+H)⁺
(4) Specific rotation: [α]_{D}²⁵ +20° (c 0.37, CH₃OH)
(5) ¹H NMR spectrum (400 MHz, CD₃OD) δ (ppm): 2.09 (1H, dddd, pyrrolidine), 2.16 - 2.26 (1H, m, pyrrolidine), 3.21 (1H, ddd, pyrrolidine), 3.48 - 3.61 (3H, m, pyrrolidine and NHCH₂), 3.72 (1H, dd, CONHCH₂), 3.87 (1H, dd, CONHCH₂), 3.88 - 3.95 (1H, m, CONHCH₂CH), 4.18 (1H, dd, pyrrolidine), 4.49 (1H, dd, pyrrolidine), 6.61 (1H, t, pyrimidine), 6.95 - 7.00 (2H, m, C₆H₄), 7.20 - 7.26 (2H, m, C₆H₄), 7.40 - 7.47 (2H, m, C₆H₅), 7.48 - 7.54 (1H, m, C₆H₅), 7.81 - 7.86 (2H, m, C₆H₅), 8.30 (2H, br s, pyrimidine)

### Example 55: (2S)-Benzenesulfonylamino-3-[3-{(3S)-hydroxy-(2S)-(1,4,5,6-tetrahydropyrimidin-2-ylaminomethyl)pyrrolidin-1-yl}benzoylamino]propionic acid

1,4-Dioxane (4.0 ml) and 2.0 ml of water were added in that order to 29 mg of the compound prepared in Example 54 to prepare a solution, and 8.3 mg of 10% palladium-carbon was added to the solution. The mixture was stirred in a hydrogen atmosphere at room temperature for 5 hr. The insolubles were filtered and were washed with 90 ml of a solvent having the same composition as the mixed solvent used in the reaction. The filtrate and the washings were combined followed by concentration under the reduced pressure. The residue was purified by Sephadex LH-20 (methanol) to give the title compound (3.1 mg, 21%).
Physicochemical properties of the compound prepared in Example 55
(1) Color and form: Colorless solid
(2) Molecular formula: C₂₅H₃₂N₆O₆S
(3) Mass spectrum (FABMS): m/z 545 (M+H)⁺
(4) Specific rotation: [α]_{D}²⁵ +89° (c 0.058, CH₃OH)
(5) ¹H NMR spectrum (400 MHz, CD₃OD) δ (ppm): 1.64 (2H, ddd, tetrahydropyrimidine), 2.00 - 2.12 (1H, m, pyrrolidine), 2.21 - 2.31 (1H, m, pyrrolidine), 3.10 - 3.26 (6H, m, tetrahydropyrimidine and NHCH₂), 3.51 (1H, dd, CONHCH₂), 3.51 - 3.62 (2H, m, pyrrolidine), 3.76 (1H, dd, CONHCH₂), 3.86 (1H, dd, CONHCH₂CH), 4.01 (1H, ddd, pyrrolidine), 4.48 (1H, ddd, pyrrolidine), 6.74 - 6.78 (1H, m, C₆H₄), 7.04 - 7.10 (2H, m, C₆H₄), 7.27 (1H, dd, C₆H₄), 7.50 - 7.56 (2H, m, C₆H₅), 7.56 - 7.62 (1H, m, C₆H₅), 7.86 - 7.91 (2H, m, C₆H₅)

### Intermediate 69: Ethyl 3-{(2S)-azidomethyl-(3S)-methoxypyrrolidin-1-yl}benzoate

Tetrahydrofuran (5.0 ml) was added to sodium hydride (60%, 95 mg, 2.4 mmol) in an argon atmosphere to prepare a suspension. Separately, ethyl 3-{(2S)-azidomethyl-(3S)-hydroxy-pyrrolidin-1-yl}benzoate (440 mg, 1.5 mmol) was dissolved in 10 ml of tetrahydrofuran to prepare a solution which was then added dropwise to the suspension at room temperature, followed by stirring for one hr. Methyl iodide (67 µl, 1.1 mmol) was added dropwise to the mixture, and the mixture was stirred for 30 min. A saturated aqueous ammonium chloride solution was then added to stop the reaction, and 100 ml of water was added thereto. The mixture was extracted twice with 100 ml of ethyl acetate. The combined organic layers were then dried over anhydrous magnesium sulfate and were concentrated under the reduced pressure. The residue was purified by column chromatography on silica gel (development system: hexane : ethyl acetate = 4 : 1) to give the title compound (309 mg, 67%).
Physicochemical properties of intermediate 69
(1) Color and form: Colorless syrup
(2) Molecular formula: C₁₅H₂₀N₄O₃
(3) Mass spectrum (EIMS): m/z 304 (M)⁺
(4) Specific rotation: [α]_{D}²⁵ +51° (c 1.1, CH₂Cl₂)
(5) ¹H NMR spectrum (400 MHz, CDCl₃) δ (ppm): 1.39 (3H, t, Et), 2.08 - 2.20 (1H, m, pyrrolidine), 2.34 (1H, dddd, pyrrolidine), 3.23 (1H, ddd, pyrrolidine), 3.32 (1H, dd, NHCH₂), 3.48 (3H, s, OMe), 3.60 (1H, ddd, pyrrolidine), 3.66 (1H, dd, NHCH₂), 4.02 - 4.10 (2H, m, pyrrolidine), 4.37 (2H, q, Et), 6.80 (1H, dd, C₆H₄), 7.27 - 7.32 (2H, m, C₆H₄), 7.40 (1H, ddd, C₆H₄)

### Intermediate 70: Ethyl 3-{(2S)-aminomethyl-(3S)-methoxypyrrolidin-1-yl}benzoate

1,4-Dioxane (10 ml) and 5.0 ml of water were added in that order to intermediate 69 (268 mg, 0.88 mmol) to prepare a solution, and 70 mg of 10% palladium-carbon was added to the solution. A reaction was allowed to proceed in a hydrogen atmosphere at room temperature for 5 hr. The insolubles were filtered and were washed with 90 ml of a solvent having the same composition as the mixed solvent used in the reaction. The filtrate and the washings were combined followed by concentration under the reduced pressure to give the title compound (182 mg, 74%).
Physicochemical properties of intermediate 70
(1) Color and form: Colorless syrup
(2) Molecular formula: C₁₅H₂₂N₂O₃
(3) Mass spectrum (FABMS): m/z 279 (M+H)⁺
(4) ¹H NMR spectrum (400 MHz, CD₃OD) δ (ppm): 1.37 (3H, t, Et), 2.05 (1H, dddd, pyrrolidine), 2.37 (1H, dddd, pyrrolidine), 2.74 (1H, dd, NHCH₂), 2.92 (1H, dd, NHCH₂), 3.16 (1H, ddd, pyrrolidine), 3.46 (3H, s, OMe), 3.56 (1H, dd, pyrrolidine), 3.89 (1H, ddd, pyrrolidine), 4.15 (1H, ddd, pyrrolidine), 4.34 (2H, q, Et), 6.89 (1H, ddd, C₆H₄), 7.22 - 7.32 (3H, m, C₆H₄)

### Intermediate 71: Ethyl 3-{(3S)-methoxy-(2S)-(pyrimidin-2-ylaminomethyl)pyrrolidin-1-yl}benzoate

Dimethyl sulfoxide (7.5 ml) was added to intermediate 70 (179 mg, 0.64 mmol) to prepare a solution, and diisopropylethylamine (850 µl, 4.9 mmol) and 2-bromopyrimidine (140 mg, 0.88 mmol) were added in that order to the solution. A reaction was allowed to proceed at 120°C for 5 hr, and the temperature of the reaction mixture was then returned to room temperature. Water (800 ml) was added thereto, and the mixture was extracted twice with 500 ml of ethyl acetate. The combined organic layers were washed twice with 500 ml of water and once with 500 ml of saturated brine, were dried over anhydrous magnesium sulfate, and were then concentrated under the reduced pressure. The residue was purified by column chromatography on silica gel (development system: hexane : ethyl acetate = 1 : 8) to give the title compound (180 mg, 78%).
Physicochemical properties of intermediate 71
(1) Color and form: Colorless solid
(2) Molecular formula: C₁₉H₂₄N₄O₃
(3) Mass spectrum (EIMS): m/z 356 (M)⁺
(4) Specific rotation: [α]_{D}²⁵ -26° (c 0.71, CH₂Cl₂)
(5) ¹H NMR spectrum (400 MHz, CDCl₃) δ (ppm): 1.38 (3H, t, Et), 2.11 (1H, dddd, pyrrolidine), 2.32 - 2.40 (1H, m, pyrrolidine), 3.18 (1H, ddd, pyrrolidine), 3.33 (1H, ddd, NHCH₂), 3.47 (3H, s, OMe), 3.54 (1H, ddd, pyrrolidine), 4.00 - 4.15 (3H, m, pyrrolidine and NHCH₂), 4.37 (2H, q, Et), 6.54 (1H, t, pyrimidine), 7.12 (1H, dd, C₆H₄), 7.29 (1H, dd, C₆H₄), 7.38 (1H, ddd, C₆H₄), 7.59 (1H, br s, C₆H₄), 8.35 (2H, br s, pyrimidine)

### Intermediate 72: 3-{(3S)-Methoxy-(2S)-(pyrimidin-2-ylaminomethyl)pyrrolidin-1-yl}benzoic acid

Tetrahydrofuran (3.0 ml) and 1.0 ml of methanol were added in that order to intermediate 71 (178 mg, 0.50 mmol) to prepare a solution, and 1.0 ml of a 1.0 M aqueous sodium hydroxide solution was added to the solution. A reaction was allowed to proceed at 50°C for 3 hr, and the temperature of the reaction mixture was then returned to room temperature. The reaction mixture was adjusted to pH 4 by the addition of 1.0 M hydrochloric acid, and 100 ml of water was added thereto. The resultant precipitate was collected by filtration, and ethyl acetate was then added to the collected precipitate to prepare a solution, which was then dried over anhydrous magnesium sulfate, was concentrated under the reduced pressure to give the title compound (118 mg, 72%).
Physicochemical properties of intermediate 72
(1) Color and form: Colorless solid
(2) Molecular formula: C₁₇H₂₀N₄O₃
(3) ¹H NMR spectrum (400 MHz, DMSO-d₆) δ (ppm): 2.02 (1H, dddd, pyrrolidine), 2.20 - 2.30 (1H, m, pyrrolidine), 3.09 (1H, ddd, pyrrolidine), 3.33 - 3.39 (1H, m, NHCH₂), 3.37 (3H, s, OMe), 3.44 (1H, ddd, pyrrolidine), 3.56 (1H, ddd, NHCH₂), 4.05 (1H, ddd, pyrrolidine), 4.06 - 4.13 (1H, ddd, pyrrolidine), 6.57 (1H, t, pyrimidine), 7.05 (1H, dd, C₆H₄), 7.18 (1H, d, C₆H₄), 7.24 (1H, dd, C₆H₄), 7.46 (1H, br s, C₆H₄), 8.31 (2H, br s, pyrimidine)

### Example 56: t-Butyl (2S)-henzenesulfonylamino-3-[3-{(3S)-methoxy-(2S)-(pyrimidin-2-ylaminomethyl)-pyrrolidin-1-yl}benzoylamino]propionate

Dimethylformamide (7.0 ml) was added to intermediate 72 (116 mg, 0.35 mmol) to prepare a solution, and t-butyl (2S)-N-benzenesulfonyl-2,3-diaminopropionate (130 mg, 0.42 mmol) was added to the solution. Further, 1-hydroxybenzotriazole (73 mg, 0.53 mmol), N-methylmorpholine (194 µl, 1.8 mmol), and 1-ethyl-3-(3-dimethylaminopropyl)carbodiimide hydrochloride (138 mg, 0.70 mmol) were added thereto, and a reaction was allowed to proceed at room temperature for 10.5 hr. A saturated aqueous sodium hydrogencarbonate solution (30 ml) was added to stop the reaction, and 500 ml of water was added thereto. The mixture was extracted twice with 300 ml of ethyl acetate, and the combined organic layers were washed with 200 ml of saturated brine, were dried over anhydrous magnesium sulfate, and were then concentrated under the reduced pressure to give the title compound (237 mg, 100%).
Physicochemical properties of the compound prepared in Example 56
(1) Color and form: Colorless solid
(2) Molecular formula: C₃₀H₃₈N₆O₆S
(3) Mass spectrum (TSPMS): m/z 611 (M+H)⁺
(4) Specific rotation: [α]_{D}²⁵ +10° (c 1.0, CH₂Cl₂)
(5) ¹H NMR spectrum (400 MHz, CDCl₃) δ (ppm): 1.29 (9H, s, t-Bu), 2.06 - 2.16 (1H, m, pyrrolidine), 2.30 - 2.40 (1H, m, pyrrolidine), 3.19 (1H, ddd, pyrrolidine), 3.36 (1H, ddd, NHCH₂), 3.46 (3H, s, OMe), 3.53 (1H, ddd, pyrrolidine), 3.64 (1H, ddd, CONHCH₂), 3.86 (1H, ddd, CONHCH₂), 3.92 - 4.10 (4H, m, pyrrolidine and CONHCH₂CH and NHCH₂), 6.53 (1H, t, pyrimidine), 6.99 - 7.05 (2H, br dd, C₆H₄), 7.26 (1H, dd, C₆H₄), 7.34 (1H, br s, C₆H₄), 7.45 - 7.50 (2H, m, C₆H₅), 7.52 - 7.58 (1H, m, C₆H₅), 7.83 - 7.87 (2H, m, C₆H₅), 8.32 (2H, d, pyrimidine)

### Example 57: (2S)-Benzenesulfonylamino-3-[3-{(3S)-methoxy-(2S)-(pyrimidin-2-ylaminomethyl)pyrrolidin-1-yl}benzoylamino]propionic acid

Methylene chloride (4.0 ml) was added to the compound (215 mg, 0.35 mmol) prepared in Example 56 to prepare a solution, and 3.0 ml of trifluoroacetic acid was added to the solution at room temperature. A reaction was allowed to proceed for 8 hr, and the reaction mixture was concentrated under the reduced pressure to give the title compound (213 mg, 67% (as tritrifluoroacetate)).
Physicochemical properties of the compound prepared in Example 57 (as tritrifluoroacetate)
(1) Color and form: Light yellow solid
(2) Molecular formula: C₂₆H₃₀N₆O₆S
(3) Mass spectrum (TSPMS): m/z 555 (N+H)⁺
(4) Specific rotation: [α]_{D}²⁵ -2.8° (c 0.65, CH₃OH)
(5) ¹H NMR spectrum (400 MHz, CD₃OD) δ (ppm): 2.13 (1H, dddd, pyrrolidine), 2.40 (1H, dddd, pyrrolidine), 3.19 (1H, ddd, pyrrolidine), 3.48 (1H, dd, NHCH₂), 3.48 (3H, s, OMe), 3.54 (1H, dd, pyrrolidine), 3.59 (1H, ddd, CONHCH₂), 3.75 (1H, dd, NHCH₂), 3.94 (1H, ddd, CONHCH₂), 4.16 (1H, ddd, pyrrolidine), 4.19 - 4.25 (2H, m, CONHCH₂CH and pyrrolidine), 6.77 (1H, t, pyrimidine), 6.91 (1H, dd, C₆H₄), 6.99 (1H, d, C₆H₄), 7.22 (1H, dd, C₆H₄), 7.27 (1H, dd, C₆H₄), 7.42 - 7.48 (2H, m, C₆H₅), 7.49 - 7.55 (1H, m, C₆H₅), 7.81 - 7.85 (2H, m, C₆H₅), 8.44 (2H, d, pyrimidine)

### Example 58 : (2S)-Benzenesulfonylamino-3-[3-{(3S)-methoxy-(2S)-(1,4,5,6-tetrahydropyrimidin-2-ylaminomethyl)pyrrolidin-1-yl}benzoylamino]propionic acid

1,4-Dioxane (10 ml) and 5.0 ml of water were added in that order to 100 mg of the compound prepared in Example 57 to prepare a solution, and 26 mg of 10% palladium-carbon was added to the solution. A reaction was allowed to proceed in a hydrogen atmosphere at room temperature for 5 hr. The insolubles were filtered and were washed with 90 ml of a solvent having the same composition as the mixed solvent used in the reaction. The filtrate and the washings were combined followed by concentration under the reduced pressure. The residue was purified by column chromatography on silica gel (development system: methylene chloride : methanol : concentrated aqueous ammonia = 100 : 10 : 1) and was then purified by Sephadex LH-20 (methanol) to give the title compound (66 mg, 100%).
Physicochemical properties of the compound prepared in Example 57
(1) Color and form: Colorless solid
(2) Molecular formula: C₂₆H₃₄N₆O₆S
(3) Mass spectrum (TSPMS): m/z 559 (M+H)⁺
(4) ¹H NMR spectrum (400 MHz, CD₃OD) δ (ppm): 1.56 (2H, ddd, tetrahydropyrimidine), 2.06 (1H, dddd, pyrrolidine), 2.37 (1H, dddd, pyrrolidine), 3.00 - 3.10 (4H, m, tetrahydropyrimidine), 3.20 (1H, ddd, pyrrolidine), 3.26 (1H, dd, NHCH₂), 3.45 - 3.58 (3H, m, pyrrolidine and CONHCH₂ and NHCH₂), 3.47 (3H, s, OMe), 3.78 (1H, dd, CONHCH₂CH), 3.84 (1H, dd, CONHCH₂), 4.08 (1H, ddd, pyrrolidine), 4.22 (1H, ddd, pyrrolidine), 6.74 (1H, dd, C₆H₄), 7.06 (1H, d, C₆H₄), 7.12 (1H, dd, C₆H₄), 7.27 (1H, dd, C₆H₄), 7.50 - 7.57 (2H, m, C₆H₅), 7.57 - 7.63 (1H, m, C₆H₅), 7.86 - 7.93 (2H, m, C₆H₅)

### Pharmacological Test Example 1: αᵥβ₃ Binding assay

Integrin αᵥβ₃ antagonistic activity was first measured in a vitronectin-vitronectin receptor binding assay system in accordance with the method of Kouns et al. (W. C. Kouns, D. Kirchhofer, P. Hadvary, A. Edenhofer, T. Weller, G. Pfenninger, H. R. Baumgartner, L. K. Jennings and B. Steiner, Blood, 80, 2539-2547 (1992)). Specifically, a vitronectin receptor (protein content: 118 mg/ml) purified from the human placenta in accordance with the method of Pytela et al. (R. Pytela, M. D. Pierschbacher, S. Argraves, S. Suzuki, and E. Ruoslahti, Method in Enzymology, 144, 475-489 (1987)) was diluted 50 times with TBS (20 mM Tris-HCl, 150 mM NaCl, 1 mM CaCl₂, 1 mM MgCl₂, pH 7.4), and distributed and coated on wells (50 µl/well) of a plate (Maxisorp, Nunc, 96 well Immuno Plate). The plate was then allowed to stand at 4°C for one day, washed twice with TBS (200 µl/well), and then subjected to blocking with TBS (150 µl/well) containing 1% bovine serum albumin (SIGMA) at 4°C overnight. After washing twice with TBS (200 µl/well), 50 µl of vitronectin (CALBIOCHEM) adjusted to 0.2 mg/ml by the addition of TBS (TBS-Tween) containing 0.01% Tween-20 was mixed with 50 µl of each test compound adjusted to each concentration in wells, and a reaction was allowed to proceed at room temperature for 4 hr. After the completion of the reaction, the wells were washed five times with TBS-Tween. A solution prepared by diluting anti-vitronectin rabbit antiserum (CHEMICON) 500 times with TBS-Tween was added as a primary antibody in an amount of 50 µl/well, and a reaction was allowed to proceed at room temperature for 1.5 hr. After washing five times with 200 µl/well of TBS-Tween, a peroxidase (POD)-labeled anti-rabbit IgG antibody solution (CAPPEL) diluted 500 times with TBS-Tween was added as a secondary antibody in an amount of 50 µl/well, and a reaction was allowed to proceed at room temperature for 1.5 hr. After washing five times with TBS-Tween (200 µl/well), ABTS (2,2'-azino-bis(3-ethylbenzthiazoline-6-sulfonic acid), SIGMA) was adjusted to 1 mg/ml by the addition of a ten-fold diluted POD-buffer (ZYMED), and was added in an amount of 50 µl/well, and a reaction was allowed to proceed for 5 to 10 min. A 0.1 M citric acid buffer (pH 4.3) containing 0.05% NaN₃ was added in an amount of 50 µl/well to stop the reaction, followed by the measurement of the absorbance at 415 nm with a microplate reader (MTP 32, Corona Electric) (reference: 675 nm). The total binding was defined as the absorbance after a reaction using 50 µl of TBS-Tween instead of the test compound, and the non-specific binding (100% inhibition) was defined as the absorbance after a reaction using 50 µl of TBS-Tween containing 2 x 10⁻³ M RGDS. The inhibition was calculated by the following equation:$\text{Inhibition (%) = 100-} \frac{\text{(absorbance in the presenee of test compound - non-specific binding)}}{\text{(total binding - non-specific binding)}} \text{× 100}$

IC₅₀ was determined from a primary regression line of the logarithm of each concentration of the test compound and the logarithm of (100 - inhibition)/inhibition.

The integrin αᵥβ₃ antagonistic activity was 1.3 nM for the compound prepared in Example 3, and was 2.0 nM for the compound prepared in Example 9.

These compounds according to the present invention, as compared with corresponding p-substituted derivatives, the balance between the αᵥβ₃ antagonistic activity and the α_{IIb}β₃ antagonistic activity was improved by several tens of folds. At the same time, the water solubility was improved by not less than two folds. Therefore, regarding the administration of drugs, for example, through intraveneous injection in acute phase or intraveneous drip in acute phase and instillation, the compounds of the present invention are expected to have excellent clinical applications.

### Pharmacological Test Example 2: GP IIb/IIIa antagonistic activity and human platelet aggregation inhibitory activity

GP IIb/IIIa antagonistic activity was measured for the compounds according to the present invention. The measurement of the GP IIb/IIIa antagonistic activity was carried out according to the method described in Pharmacological Test 2 in WO 94/21599. As a result, both the compounds prepared in Examples 3 and 6 had significant GP IIb/IIIa antagonistic activity. In particular, the IC₅₀ value of the compound prepared in Example 3 was 2.0 nM.

### Pharmacological Test Example 3: Inhibitory activity against adhesion of human vascular smooth muscle cells to vitronectin

The adhesion of human vascular smooth muscle cells to immobilized human vitronectin was measured in accordance with the method of Liaw et al. (Liaw L, Almeida M, Hart CE, Schwartz SM, and Giachelli CM, Circulation Research, 74 (2), 214-224 (1994)).

A Dulbecco's phosphate buffer (Dulbecco's PBS(-), Nissui Pharmaceutical Co., Ltd.) solution of human plasma-derived vitronectin (CALBIOCHEM) adjusted to a concentration of 4 µg/ml was first added to wells (50 µl/well) of a microplate (Maxisorp, Nunc), and a reaction for immobilization was allowed to proceed at 4°C overnight. After washing twice with 150 µl of Dulbecco's phosphate buffer, a Dulbecco's phosphate buffer containing 10 mg/ml of bovine serum albumin (SIGMA) was added, followed by blocking at 37°C for one hr. The assay plate was then washed twice with 150 µl of Dulbecco's phosphate buffer.

Separately, human vascular smooth muscle cells cultivated at 37°C under 5% carbon dioxide in a medium for vascular smooth muscle cells (Clonetics) were separated using a Dulbecco's phosphate buffer containing trypsin-EDTA (GIBCO BRL), washed with Dulbecco's phosphate buffer, and then suspended in a Dulbecco's modified Eagle's basal medium (Nissui Pharmaceutical Co., Ltd.) containing 0.1% bovine serum albumin to a concentration of 5 x 10⁵/ml.

Next, 50 µl of a Dulbecco's modified Eagle's basal medium containing 0.1% of bovine serum albumin with a medicament added thereto was added to the wells of the human vitronectin-coated assay microplate, followed by pre-cultivation under 5% carbon dioxide at 37°C for 10 min. Thereafter, 50 µl of the medium with human vascular smooth muscle cells suspended therein was added thereto, and the plate was thoroughly stirred. A reaction was allowed to proceed under 5% carbon dioxide at 37°C for 90 min. The reaction solution containing non-adherent cells were removed, followed by washing three times with Dulbecco's phosphate buffer. For the adhered cells, 100 µl of a Dulbecco's phosphate buffer containing 4% paraformaldehyde (Wako Pure Chemical Industries, Ltd.) was added, and immobilization was allowed to proceed at room temperature for 10 min. Next, 100 µl of a Dulbecco's phosphate buffer containing 0.5% Toluidine Blue (Croma) and 4% paraformaldehyde was added, and staining was allowed to proceed at room temperature for 5 min, followed by thorough washing with distilled water. The inside of the wells was then air-dried, and 1% aqueous sodium dodecylsulfate solution was added to perform cytolysis. The absorbance of the microplate thus obtained was measured at 595 nm. The total binding was defined as the absorbance of the well not containing the test compound, and the non-specific binding (100% inhibition) was defined as the absorbance of the well which does not contain vitronectin and has been subjected to blocking with bovine serum albumin. The inhibition was calculated by the following equation. IC₅₀ was determined from a primary regression line of the logarithm of each concentration of the test compound and the logarithm of (100 - inhibition)/inhibition.$\text{Inhibition (%) = 100-} \frac{\text{(absorbance in the presence of test compound - non-specific binding)}}{\text{(total binding - non-specific binding)}} \text{× 100}$

As a result, the compound of Example 27 had potent cell adhesion inhibitory activity, and, for this compound, the IC₅₀ value on the inhibitory activity against the adhesion of human vascular smooth muscle cells to vitronectin was 96 nM.

The structures of the compounds described in Examples 1 to 58 can be summarized as follows.

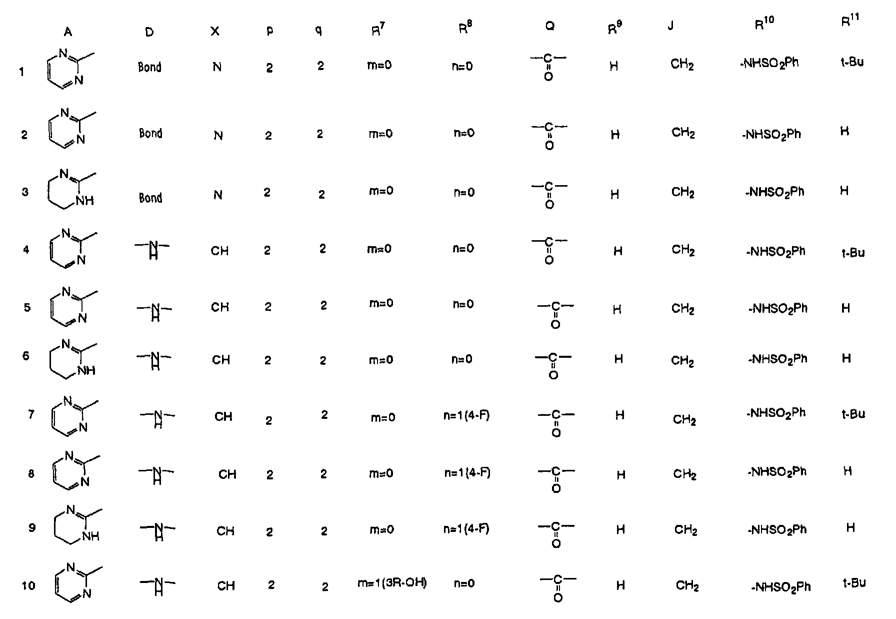

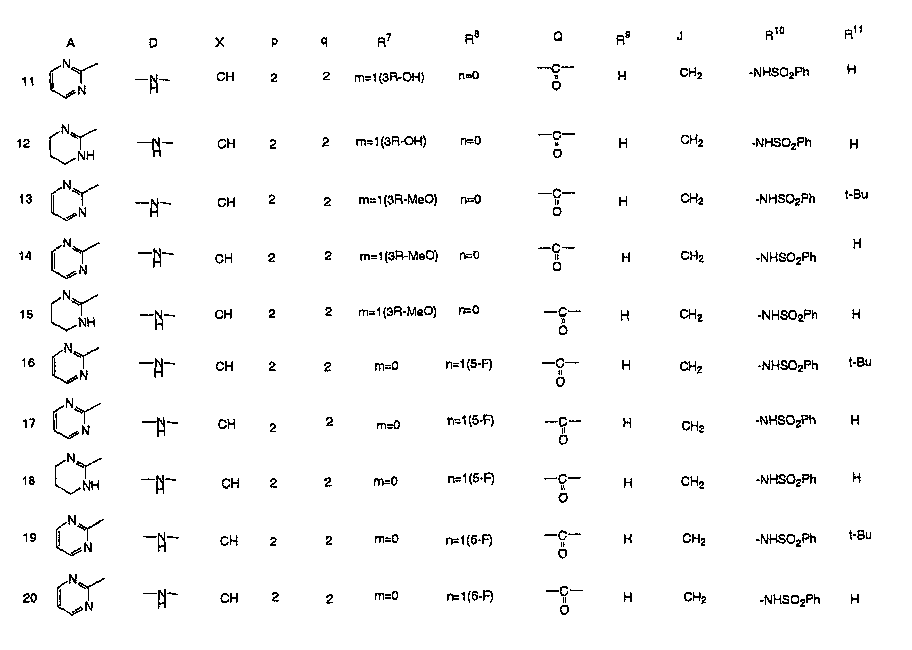

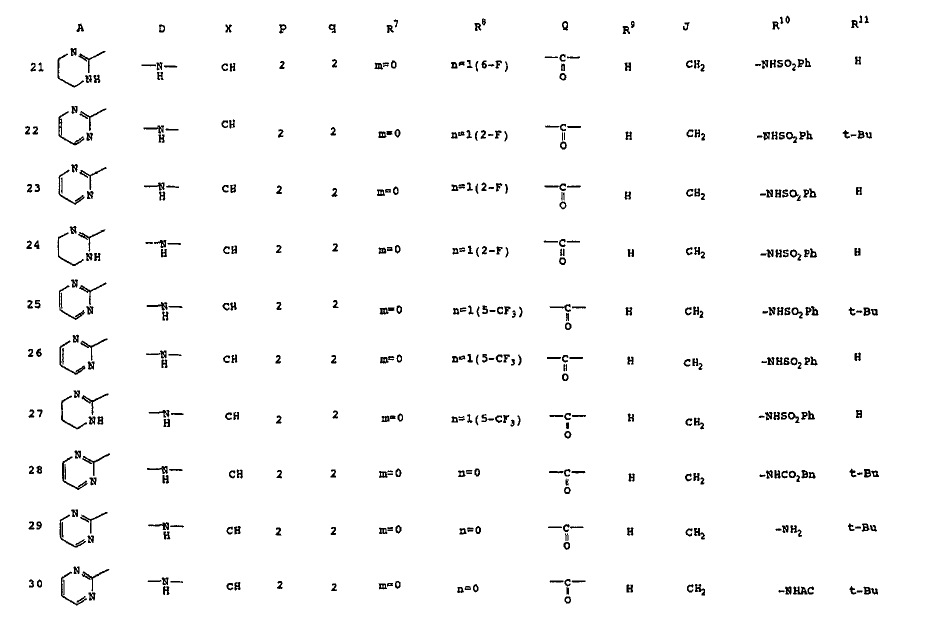

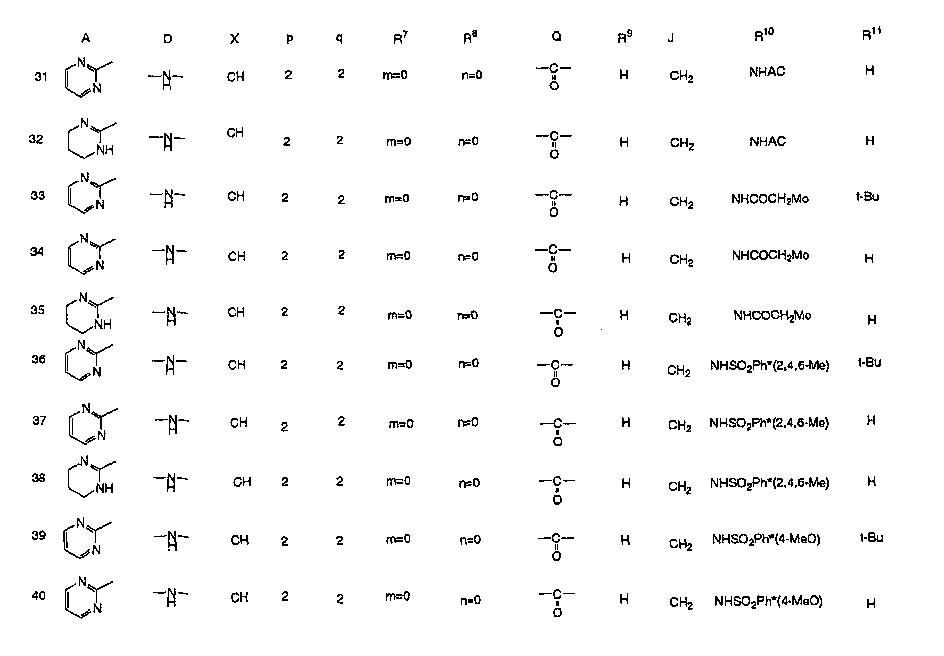

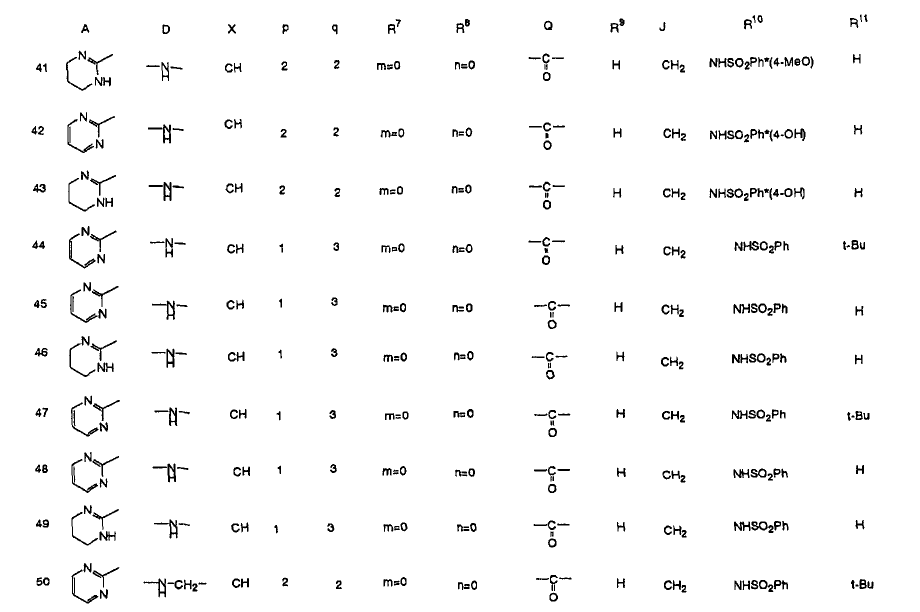

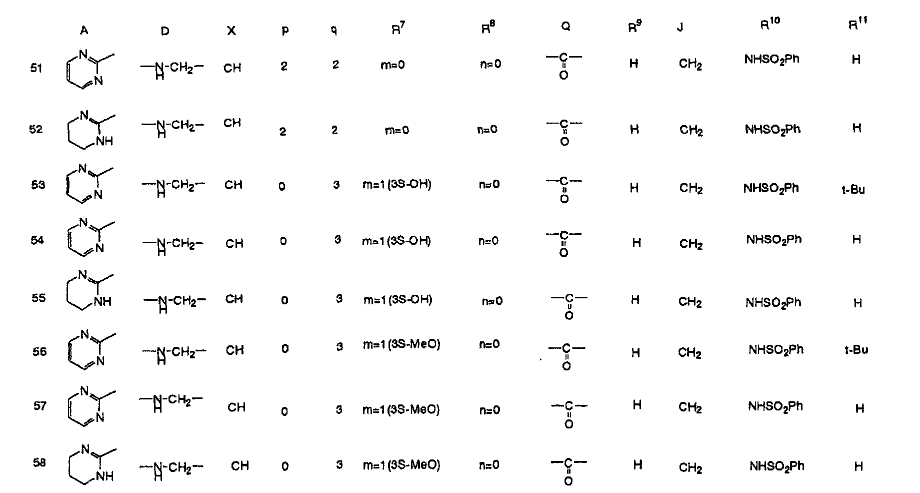

Me: methyl, Bu: butyl, Ac: acetyl, MeO: methoxy, Ph: phenyl,
Ph^{*} : substituted phenyl, Bn: benzyl, and Mo: morpholin-4-yl

## Claims

1. A compound represented by formula (I) or a pharmaceutically acceptable salt or solvate thereof: 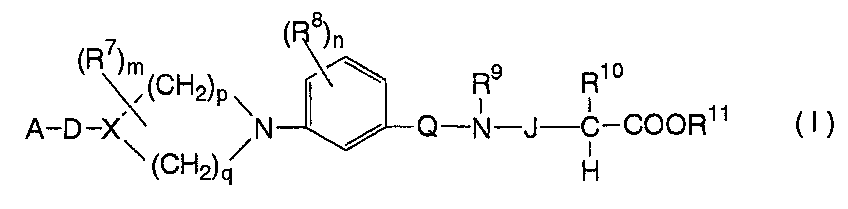 wherein
A represents a saturated or unsaturated five- to seven-membered heterocyclic group containing two nitrogen atoms, which is optionally condensed with another saturated or unsaturated five- to seven-membered carbocyclic ring or heterocyclic ring to form a bicyclic group, wherein the heterocyclic group and the bicyclic group are optionally substituted by C₁₋₆ alkyl optionally substituted by C₁₋₆ alkyl, C₁₋₆ alkoxy, C₁₋₆ alkoxycarbonyl, aralkyl, amino, or hydroxyl; a halogen atom; or amino optionally substituted by C₁₋₆ alkyl, C₁₋₆ alkoxy, C₁₋₆ alkoxycarbonyl, or aralkyl,
or a group represented by formula 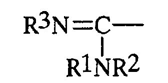 wherein R¹, R², and R³, which may be the same or different, represent a hydrogen atom, C₁₋₆ alkyl, C₂₋₆ alkenyl, or aralkyl, wherein the C₁₋₆ alkyl, C₂₋₆ alkenyl, and aralkyl groups are optionally substituted by C₁₋₆ alkyl, C₁₋₆ alkoxy, C₁₋₆ alkoxycarbonyl, aralkyl, amino, or hydroxyl;
D represents a bond; >NR⁴ wherein R⁴ represents a hydrogen atom or C₁₋₆ alkyl and this C₁₋₆ alkyl group is optionally substituted by C₁₋₆ alkyl, C₁₋₆ alkoxy, C₁₋₆ alkoxycarbonyl, aralkyl, amino, or hydroxyl; >CR⁵R⁶ wherein R⁵ and R⁶ each independently represent a hydrogen atom or C₁₋₆ alkyl and this C₁₋₆ alkyl group is optionally substituted by C₁₋₆ alkyl, C₁₋₆ alkoxy, C₁₋₆ alkoxycarbonyl, aralkyl, amino, or hydroxyl; -O-; -S-; or -NR⁴-CR⁵R⁶-wherein R⁴, R⁵, and R⁶ are as defined above;
X represents CH or N;
R⁷ represents C₁₋₆ alkyl, C₁₋₆ alkoxy, a halogen atom, amino, nitro, cyano, hydroxyl, thiol, or an oxygen atom, wherein the C₁₋₆ alkyl and C₁₋₆ alkoxy groups represented by R⁷ are optionally substituted by C₁₋₆ alkyl, C₁₋₆ alkoxy, C₁₋₆ alkoxycarbonyl, aralkyl, amino, hydroxyl, or a halogen atom, the amino group represented by R⁷ is optionally substituted by one or two C₁₋₆ alkyl groups, and the thiol group represented by R⁷ is optionally substituted by C₁₋₄ alkyl or phenyl;
R⁸ represents C₁₋₆ alkyl, C₁₋₆ alkoxy, a halogen atom, amino, nitro, cyano, hydroxyl, or thiol, wherein the C₁₋₆ alkyl and C₁₋₆ alkoxy groups represented by R⁸ are optionally substituted by C₁₋₆ alkyl, C₁₋₆ alkoxy, C₁₋₆ alkoxycarbonyl, aralkyl, amino, hydroxyl, or a halogen atom, the amino group represented by R⁸ is optionally substituted by one or two C₁₋₆ alkyl groups, and the thiol group represented by R⁸ is optionally substituted by C₁₋₄ alkyl or phenyl;
Q represents >C=O, >CHR¹³, or >CHOR¹³ wherein R¹³ represents a hydrogen atom or C₁₋₆ alkyl;
R⁹ represents a hydrogen atom, C₁₋₆ alkyl, C₂₋₆ alkenyl, or aralkyl and the C₁₋₆ alkyl, C₂₋₆ alkenyl, and aralkyl groups are optionally substituted by C₁₋₆ alkyl, C₁₋₆ alkoxy, C₁₋₆ alkoxycarbonyl, aralkyl, amino, or hydroxyl;
J represents a bond or an alkylene chain having 1 to 3 carbon atoms, wherein one or more hydrogen atoms on the alkylene chain are optionally substituted by the same or different substituent selected from C₁₋₆ alkyl, C₂₋₆ alkenyl, C₂₋₆ alkynyl, aralkyl, hydroxyl, or amino, the C₁₋₆ alkyl, C₂₋₆ alkenyl, C₂₋₆ alkynyl, and aralkyl groups are optionally substituted by a halogen atom, C₁₋₆ alkoxy, amino, or hydroxyl, and the hydroxyl and amino groups are optionally substituted by carboxyl; sulfonyl; C₁₋₆ alkyl; C₁₋₆ alkylcarbonyl; C₁₋₆ alkoxycarbonyl; C₁₋₆ alkylsulfonyl; -C(=O)-O-(CH₂)u-R¹⁴ wherein u is an integer of 0 to 4, R¹⁴ represents a saturated or unsaturated five- to seven-membered carbocyclic or heterocyclic group, and the carbocyclic group and the heterocyclic group are optionally substituted by C₁₋₆ alkyl, C₁₋₆ alkoxy, phenyl optionally condensed with the carbocyclic group or the heterocyclic group, carboxyl, hydroxyl, nitro, amino, C₁₋₆ alkylamino, or a halogen atom; -C(=O)-R¹⁴ wherein R¹⁴ is as defined above; or -S(=O)₂-(CH₂)v-R¹⁴ wherein v is an integer of 0 to 4 and R¹⁴ is as defined above;
R¹⁰ represents a hydrogen atom, hydroxyl, C₁₋₆ alkyl, C₂₋₆ alkenyl, C₂₋₆ alkynyl, aralkyl, or amino, the C₁₋₆ alkyl, C₂₋₆ alkenyl, C₂₋₆ alkynyl, and aralkyl groups are optionally substituted by a halogen atom, C₁₋₆ alkoxy, amino, or hydroxyl and the hydroxyl and amino groups are optionally substituted by carboxyl; sulfonyl; C₁₋₆ alkyl; C₁₋₆ alkylcarbonyl; C₁₋₆ alkoxycarbonyl; C₁₋₆ alkylsulfonyl; -C(=O)-O-(CH₂)u-R¹⁴ wherein u is an integer of 0 to 4 and R¹⁴ represents a saturated or unsaturated five- to seven-membered carbocyclic or heterocyclic group, and the carbocyclic group and the heterocyclic group are optionally substituted by C₁₋₆ alkyl, C₁₋₆ alkoxy, phenyl optionally condensed with the carbocyclic group or the heterocyclic group, carboxyl, hydroxyl, nitro, amino, C₁₋₆ alkylamino, or a halogen atom; -C(=O)-R¹⁴ wherein R¹⁴ is as defined above; or -S(=O)₂-(CH₂)v-R¹⁴ wherein v is an integer of 0 to 4 and R¹⁴ is as defined above;
R¹¹ represents a hydrogen atom, aralkyl, or C₁₋₆ alkyl and this C₁₋₆ alkyl group is optionally substituted by C₁₋₆ alkyl, C₁₋₆ alkoxy, C₁₋₆ alkoxycarbonyl, aralkyl, amino, or hydroxyl;
m is an integer of 0 to 5;
n is an integer of 0 to 4;
p is an integer of 0 to 3; and
q is an integer of 0 to 3.

2. The compound according to claim 1, wherein X represents N.

3. The compound according to claim 1, wherein X represents CH.

4. The compound according to any one of claims 1 to 3, wherein p is 0 to 2 and q is 2 or 3.

5. The compound according to any one of claims 1 to 4, wherein A represents a group of formula 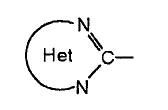 wherein
Het represents a saturated or unsaturated five- to seven-membered heterocyclic group containing two nitrogen atoms, which is optionally condensed with another saturated or unsaturated five- to seven-membered carbocyclic ring or heterocyclic ring to form a bicyclic group, wherein the heterocyclic group and the bicyclic group are optionally substituted by C₁₋₆ alkyl optionally substituted by C₁₋₆ alkyl, C₁₋₆ alkoxy, C₁₋₆ alkoxycarbonyl, aralkyl, amino, or hydroxyl; a halogen atom; or amino optionally substituted by C₁₋₆ alkyl, C₁₋₆ alkoxy, C₁₋₆ alkoxycarbonyl, or aralkyl.

6. The compound according to any one of claims 1 to 4, wherein A represents a group of formula 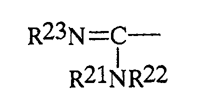 wherein
R²¹, R²², and R²³, which may be the same or different, represent a hydrogen atom, C₁₋₆ alkyl, C₂₋₆ alkenyl, or aralkyl and C₁₋₆ alkyl, C₂₋₆ alkenyl, and aralkyl groups are optionally substituted by C₁₋₆ alkyl, C₁₋₆ alkoxy, C₁₋₆ alkoxycarbonyl, aralkyl, amino, or hydroxyl, or
R²¹ and R²³ may together form
group -(CH₂)₄-,
group -(CH₂)₃-,
group -CHR²⁴CH₂CH₂- wherein R²⁴ represents C₁₋₆ alkyl, a halogen atom, or amino, and this amino group is optionally substituted by C₁₋₆ alkyl, C₁₋₆ alkoxy, C₁₋₆ alkoxycarbonyl, or aralkyl,
group -CH₂CHR²⁴CH₂- wherein R²⁴ is as defined above,
group -CH₂CH₂-,
group -CHR²⁴CH₂- wherein R²⁴ is as defined above,
group -CR²⁵=CR²⁶- wherein R²⁵ and R²⁶, which may be the same or different, represent a hydrogen atom or C₁₋₆ alkyl, or R²⁵ and R²⁶ may together form -CH=CH-CH=CH-, -CR²⁴=CH-CH=CH- wherein R²⁴ is as defined above, -CH=CR²⁴-CH=CH- wherein R²⁴ is as defined above, -N=CH-CH=CH-, or -CH=N-CH=CH-, or
R²¹ and R²³ may together form
=CH-CH=CH-,
-CHR²⁴CH₂CR₂- wherein R²⁴ is as defined above,
-CH₂CHR²⁴CH₂- wherein R²⁴ is as defined above,
=CH-CH=N-, or
=CH-N=CH-, and
R²² may represent a single bond between R²¹ and the nitrogen atom attached to R²¹.

7. The compound according to any one of claims 1 to 6, wherein D represents a bond, >NH, or -NH-CH₂-.

8. The compound according to any one of claims 1 to 7, wherein Q represents >C=O or >CH₂.

9. The compound according to any one of claims 1 to 8, wherein m and n are each an integer of 0 to 2.

10. The compound according to claim 1, wherein A represents a group of formula 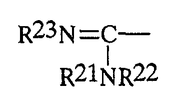 wherein
R²¹, R²², and R²³ are as defined in claim 6;
D represents a bond, >NH, or -NH-CH₂-;
X represents CH or N;
Q represents >C=O or >CH₂;
R⁹ represents a hydrogen atom, C₁₋₆ alkyl or aralkyl and the C₁₋₆ alkyl and aralkyl groups are optionally substituted by a halogen atom, C₁₋₆ alkoxy, amino, or hydroxyl;
J represents a methylene chain;
R¹⁰ represents a hydrogen atom, hydroxyl, or amino, the hydroxyl group is optionally substituted by C₁₋₆ alkyl and the amino group is optionally substituted by C₁₋₆ alkyl; C₁₋₆ alkylcarbonyl; C₁₋₆ alkoxycarbonyl; C₁₋₆ alkylsulfonyl; benzoyl or benzyloxycarbonyl wherein the phenyl portion of benzoyl and benzyloxycarbonyl is optionally substituted by C₁₋₆ alkyl, C₁₋₆ alkoxy, carboxyl, hydroxyl, nitro, amino, or a halogen atom; -C(=O)-O-(CH₂)u-R¹⁴ wherein u is an integer of 0 to 4 and R¹⁴ represents phenyloptionally substituted by C₁₋₆ alkyl, C₁₋₆ alkoxy, carboxyl, hydroxyl, nitro, amino, or a halogen atom, or a five- or six-membered heterocyclic group containing one or two hetero-atoms; or -S(=O)₂-(CH₂)v-R¹⁴ wherein v is an integer of 0 to 4 and R¹⁴ represents phenyl optionally substituted by C₁₋₆ alkyl, C₁₋₆ alkoxy, carboxyl, hydroxyl, nitro, amino, or a halogen atom, or a five- or six-membered heterocyclic group containing one or two hetero-atoms;
R¹¹ represents a hydrogen atom or C₁₋₆ alkyl;
m and n are each an integer of 0 to 2;
p is 0 to 2; and
q is 2 or 3.

11. The compound according to claim 1, which is selected from:
1. t-butyl (2S)-benzenesulfonylamino-3-[3-{4-(pyrimidin-2-yl)piperazin-1-yl}benzoylamino]propionate;
2. (2S)-benzenesulfonylamino-3-[3-{4-(pyrimidin-2-yl)piperazin-1-yl}benzoylamino]propionic acid;
3. (2S)-benzenesulfonylamino-3-[3-{4-(1,4,5,6-tetrahydropyrimidin-2-yl)piperazin-1-yl}benzoylamino]-propionic acid;
4. t-butyl (2S)-benzenesulfonylamino-3-[3-{4-(pyrimidin-2-ylamino)piperidin-1-yl}benzoylamino]-propionate;
5. (2S)-benzenesulfonylamino-3-[3-{4-(pyrimidin-2-ylamino)piperidin-1-yl}benzoylamino]propionic acid;
6. (2S)-benzenesulfonylamino-3-[3-{4-(1,4,5,6-tetrahydropyrimidin-2-ylamino)piperidin-1-yl}benzoylamino]propionic acid;
7. t-butyl (2S)-benzenesulfonylamino-3-[4-fluoro-3-{4-(pyrimidin-2-ylamino)piperidin-1-yl}benzoylamino]-propionate;
8. (2S)-benzenesulfonylamino-3-[4-fluoro-3-{4-(pyrimidin-2-ylamino)piperidin-1-yl}benzoylamino]-propionic acid;
9. (2S)-benzenesulfonylamino-3-[4-fluoro-3-{4-(1,4,5,6-tetrahydropyrimidin-2-ylamino)piperidin-1-yl}benzoylamino]propionic acid;
10. t-butyl (2S)-benzenesulfonylamino-3-[3-{(3R)-hydroxy-(4R)-(pyrimidin-2-ylamino)piperidin-1-yl}benzoylamino]propionate;
11. (2S)-benzenesulfonylamino-3-[3-{(3R)-hydroxy(4R)-(pyrimidin-2-ylamino)piperidin-1-yl}benzoylamino]propionic acid;
12. (2S)-benzenesulfonylamino-3-[3-{(3R)-hydroxy(4R)-(1,4,5,6-tetrahydropyrimidin-2-ylamino)piperidin-1-yl}benzoylamino]propionic acid;
13. t-butyl (2S)-benzenesulfonylamino-3-[{(3R)-methoxy-(4R)-(pyrimidin-2-ylamino)}piperidin-1-yl}benzoylamino]propionate;
14. (2S)-benzenesulfonylamino-3-[{(3R)-methoxy-(4R)-(pyrimidin-2-ylamino)piperidin-1-yl}benzoylamino]-propionic acid;
15. (2S)-benzenesulfonylamino-3-[3-{(3R)-methoxy-(4R)-(1,4,5,6-tetrahydropyrimidin-2-ylamino)piperidin-1-yl}benzoylamino]propionic acid;
16. t-butyl (2S)-benzenesulfonylamino-3-[5-fluoro-3-{4-(pyrimidin-2-ylamino)piperidin-1-yl}benzoylamino) propionate;
17. (2S)-benzenesulfonylamino-3-[5-fluoro-3-{4-(pyrimidin-2-ylamino)piperidin-1-yl}benzoylamino]-propionic acid;
18. (2S)-benzenesulfonylamino-3-[5-fluoro-3-{4-(1,4,5,6-tetrahydropyrimidin-2-ylamino)piperidin-1-yl}benzoylamino]propionic acid;
19. t-butyl (2S)-benzenesulfonylamino-3-[6-fluoro-3-{4-(pyrimidin-2-ylamino)piperidin-1-yl)benzoylamino]-propionate;
20. (2S)-benzenesulfonylamino-3-[6-fluoro-3-{4-(pyrimidin-2-ylamino)piperidin-1-yl}benzoylamino]-propionic acid;
21. (2S)-benzenesulfonylamino-3-[6-fluoro-3-{4-(1,4,5,6-tetrahydropyrimidin-2-ylamino)piperidin-1-yl}-benzoylamino]propionic acid;
22. t-butyl (2S)-benzenesulfonylamino-3-[2-fluoro-3-{4-(pyrimidin-2-ylamino)piperidin-1-yl}benzoylamino]-propionate;
23. (2S)-benzenesulfonylamino-3-[2-fluoro-3-{4-(pyrimidin-2-ylamino)piperidin-1-yl}benzoylamino]-propionic acid;
24. (2S)-benzenesulfonylamino-3-[2-fluoro-3-{4-(1,4,5,6-tetrahydropyrimidin-2-ylamino)piperidin-1-yl}-benzoylamino]propionic acid;
25. t-butyl (2S)-benzenesulfonylamino-3-[3-{4-(pyrimidin-2-ylamino)piperidin-1-yl}-5-(trifluoromethyl)benzoylamino]propionate;
26. (2S)-benzenesulfonylamino-3-[3-{4-(pyrimidin-2-ylamino)piperidin-1-yl}-5-(trifluoromethyl)benzoylamino]propionic acid;
27. (2S)-benzenesulfonylamino-3-[3-{4-(1,4,5,6-tetrahydropyrimidin-2-ylamino)piperidin-1-yl}-5-(trifluoromethyl)benzoylamino]propionic acid;
28. t-butyl (2S)-(benzyloxycarbonyl)amino-3-[3-{4-(pyrimidin-2-ylamino)piperidin-1-yl}benzoylamino]-propionate;
29. t-butyl (2S)-amino-3-[3-{4-(pyrimidin-2-ylamino)piperidin-1-yl}benzoylamino]propionate;
30. t-butyl (2S)-acetamido-3-[3-{4-(pyrimidin-2-ylamino)piperidin-1-yl}benzoylamino]propionate;
31. (2S)-acetamido-3-[3-{4-(pyrimidin-2-ylamino)-piperidin-1-yl}benzoylamino]propionic acid;
32. (2S)-acetamido-3-[3-{4-(1,4,5,6-tetrahydropyrimidin-2-ylamino)piperidin-1-yl}benzoylamino]-propionic acid;
33. t-butyl (2S)-{2-(morpholin-4-yl-acetyl)amino}-3-[3-{4-(pyrimidin-2-ylamino)piperidin-1-yl}benzoylamino]propionate;
34. (2S)-{2-(morpholin-4-yl-acetyl)amino}-3-[3-{4-(pyrimidin-2-ylamino)piperidin-1-yl}benzoylamino]-propionic acid;
35. (2S)-{2-(morpholin-4-yl-acetyl)amino}-3-[3-{4-(1,4,5,6-tetrahydropyrimidin-2-ylamino)piperidin-1-yl}benzoylamino]propionic acid;
36. t-butyl 3-[3-{4-(pyrimidin-2-ylamino)piperidin-1-yl}benzoylamino]-(2S)-{(2,4,6-trimethylbenzenesulfonyl)amino}propionate;
37. 3-[3-{4-(pyrimidin-2-ylamino)piperidin-1-yl}benzoylamino]-(2S)-{(2,4,6-trimethylbenzenesulfonyl)-amino}propionic acid;
38. 3-[3-{4-(1,4,5,6-tetrahydropyrimidin-2-yl-amino)piperidin-1-yl}benzoylamino]-(2S)-{(2,4,6-trimethylbenzenesulfonyl)amino}propionic acid;
39. t-butyl (2S)-{(4-methoxybenzenesulfonyl)amino}-3-[3-{4-(pyrimidin-2-ylamino)piperidin-1-yl}benzoylamino]propionate;
40. (2S)-{(4-methoxybenzenesulfonyl)amino}-3-[3-{4-(pyrimidin-2-ylamino)piperidin-1-yl}benzoylamino]-propionic acid;
41. (2S)-{(4-methoxybenzenesulfonyl)amino}-3-[3-{4-(1,4,5,6-tetrahydropyrimidin-2-ylamino)piperidin-1-yl}benzoylamino]propionic acid;
42. (2S)-{(4-hydroxybenzenesulfonyl)amino}-3-[3-{4-(pyrimidin-2-ylamino)piperidin-1-yl}benzoylamino]-propionic acid;
43. (2S)-{(4-hydroxybenzenesulfonyl)amino}-3-[3-{4-(1,4,5,6-tetrahydropyrimidin-2-ylamino)piperidin-1-yl}benzoylamino]propionic acid;
44. t-butyl (2S)-benzenesulfonylamino-3-[3-{(3S)-(pyrimidin-2-ylamino)piperidin-1-yl}benzoylamino]-propionate;
45. (2S)-benzenesulfonylamino-3-[3-{(3S)-(pyrimidin-2-ylamino)piperidin-1-yl}benzoylamino]-propionic acid;
46. (2S)-benzenesulfonylamino-3-[3-{(3S)-(1,4,5,6-tetrahydropyrimidin-2-ylamino)piperidin-1-yl}benzoylamino]propionic acid;
47. t-butyl (2S)-benzenesulfonylamino-3-[3-{(3R)-(pyrimidin-2-ylamino)piperidin-1-yl}benzoylamino]-propionate;
48. (2S)-benzenesulfonylamino-3-[3-{(3R)-(pyrimidin-2-ylamino)piperidin-1-yl}benzoylamino]-propionic acid;
49. (2S)-benzenesulfonylamino-3-[3-{(3R)-(1,4,5,6-tetrahydropyrimidin-2-ylamino)piperidin-1-yl}benzoylamino]propionic acid;
50. t-butyl (2S)-benzenesulfonylamino-3-[3-{4-(pyrimidin-2-ylaminomethyl)piperidin-1-yl}benzoylamino]-propionate;
51. (2S)-benzenesulfonylamino-3-[3-{4-(pyrimidin-2-ylaminomethyl)piperidin-1-yl}benzoylamino]propionic acid;
52. (2S)-benzenesulfonylamino-3-[3-{4-(1,4,5,6-tetrahydropyrimidin-2-ylaminomethyl)piperidin-1-yl}benzoylamino]propionic acid;
53. t-butyl (2S)-benzenesulfonylamino-3-[3-{(3S)-hydroxy-(2S)-(pyrimidin-2-ylaminomethyl)pyrrolidin-1-yl}benzoylamino]propionate;
54. (2S)-benzenesulfonylamino-3-[3-{(3S)-hydroxy(2S)-(pyrimidin-2-ylaminomethyl)pyrrolidin-1-yl}benzoylamino]propionic acid;
55. (2S)-benzenesulfonylamino-3-[3-{(3S)-hydroxy(2S)-(1,4,5,6-tetrahydropyrimidin-2-ylaminomethyl)-pyrrolidin-1-yl}benzoylamino]propionic acid;
56. t-butyl (2S)-benzenesulfonylamino-3-[3-{(3S)-methoxy-(2S)-(pyrimidin-2-ylaminomethyl)pyrrolidin-1-yl}benzoylamino]propionate;
57. (2S)-benzenesulfonylamino-3-[3-{(3S)-methoxy(2S)-(pyrimidin-2-ylaminomethyl)pyrrolidin-1-yl}benzoylamino]propionic acid; and
58. (2S)-benzenesulfonylamino-3-[3-{(3S)-methoxy-(2S)-(1,4,5,6-tetrahydropyrimidin-2-ylaminomethyl)-pyrrolidin-1-yl}benzoylamino]propionic acid.

12. A pharmaceutical composition comprising as active ingredient the compound according to any one of claims 1 to 11 or a pharmaceutically acceptable salt or solvate thereof.

13. The pharmaceutical composition according to claim 12, for use in the treatment of integrin αᵥβ₃-mediated diseases.

14. The pharmaceutical composition according to claim 12, for use in the treatment of diseases where the inhibition of cell adhesion is therapeutically effective.

15. The pharmaceutical composition according to claim 12, for use in the treatment of diseases where GP IIb/IIIa antagonistic activity and/or platelet aggregation inhibitory activity are therapeutically effective.

16. The pharmaceutical composition according to claim 12, for use in the treatment of a disease selected from the group consisting of cardiovascular diseases, angiogenesis-related diseases, cerebrovascular diseases, cancers and metastasis thereof, immunological diseases, and osteopathy.

17. The pharmaceutical composition according to claim 16, wherein the cardiovascular disease is selected from acute myocardial infarction, neointima formation hypertrophy, restenosis after PTCA/stent operation, unstable angina, acute coronary syndrome, angina pectoris after PTCA/stent operation, and arterial sclerosis, particularly atherosclerosis, the angiogenesis-related disease is selected from diabetic retinopathy, diabetic vascular complication, and vascular grafting, the cerebrovascular disease is cerebral infarction, the cancer and metastasis thereof are solid tumors and metastasis thereof, the immunological disease is arthritis, particularly rheumatic arthritis, and the osteopathy is selected from osteoporosis, hypercalcemia, periodontitis, hyperparathyroidism, periarticular sore, and Paget's diseases.

18. The pharmaceutical composition according to claim 12, for use in the treatment of platelet thrombosis or thromboembolism, the improvement of peripheral circulating blood stream, the inhibition of blood clotting during extracorporeal circulation, or the treatment of thrombotic thrombocytopenic purpura or hemolytic uremic syndrome.

19. The pharmaceutical composition according to claim 12, for use as an agent for inhibiting platelet aggregation.

20. Use of the compound according to any one of claims 1 to 11 or a pharmaceutically acceptable salt or solvate thereof, for the manufacture of a medicament for use in the treatment of a disease selected from the group consisting of integrin αᵥβ₃-mediated diseases, diseases where the inhibition of cell adhesion is therapeutically effective, and diseases where GP IIb/IIIa antagonistic activity and/or platelet aggregation inhibitory activity are therapeutically effective.

21. A method for treating a disease selected from the group consisting of integrin αᵥβ₃-mediated diseases, diseases where the inhibition of cell adhesion is therapeutically effective, and diseases where GP IIb/IIIa antagonistic activity and/or platelet aggregation inhibitory activity are therapeutically effective, which comprises the step of administering an effective amount of the compound according to any one of claims 1 to 11 or a pharmaceutically acceptable salt or solvate thereof, together with a pharmaceutically acceptable carrier, to a mammal including a human.

22. A process for producing a compound represented by formula (XX) 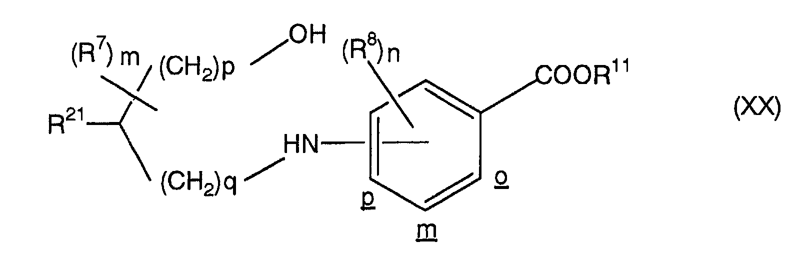 wherein R²¹ represents hydroxyl, azide, or optionally protected amino; R⁷, R⁸, R¹¹, m, n, p, and q are as defined in claim 1, provided that q is not 0 (zero) and the nitrogen atom is attached to the ortho-, meta-, or para-position of the phenyl group, which comprises the step of reacting a compound represented by formula (XIX) 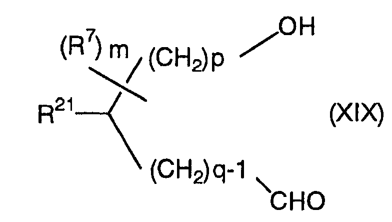 wherein R²¹ is as defined in the definition of formula (XX); and R⁷, m, p, and q are as defined in claim 1, provided that q is not 0 (zero),
with a compound represented by formula (XV) 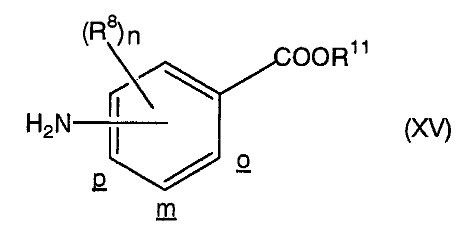 wherein R⁸, R¹¹, and n are as defined in claim 1; and the nitrogen atom is attached to the ortho-, meta-, or para-position of the phenyl group.

23. The process according to claim 22, wherein, in formulae (XIX) and (XX), p and q are 2, R⁷ and R²¹ represent hydroxyl, and m is 1.

24. The process according to claim 22, wherein the compound represented by formula (XIX) is selected from the group consisting of pentoses, hexoses, and heptoses and derivatives thereof.

25. The process according to claim 22, wherein the compound represented by formula (XIX) is 2-deoxy-D-ribose.

26. A process for producing a compound represented by formula (XXII) 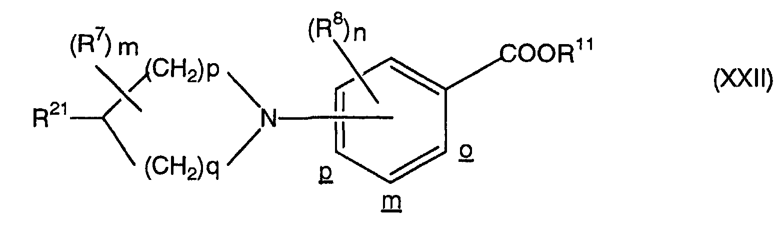 wherein R⁷, R⁸, R¹¹, m, n, p, and q are as defined in claim 1, provided that q is not 0 (zero); R²¹ is as defined in claim 22; and the nitrogen atom is attached to the ortho-, meta-, or para-position of the phenyl group, which comprises the step of cyclizing a compound represented by formula (XXI) 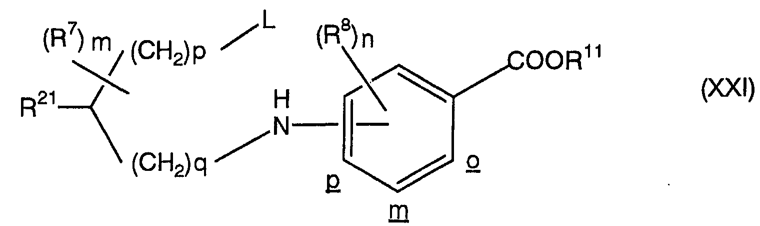 wherein R⁷, R⁸, R¹¹, m, n, p, and q are as defined in claim 1, provided that q is not 0 (zero); R²¹ is as defined in claim 22; L represents a leaving group; and the nitrogen atom is attached to the ortho-, meta-, or para-position of the phenyl group by an intramolecular ring-closing reaction.

27. The process according to claim 26, which further comprises, before the intramolecular ring-closing reaction, the step of converting a primary hydroxyl group in the compound represented by formula (XX) according to claim 22 to a leaving group L to produce the compound represented by formula (XXI).

28. The process according to claim 26 or 27, wherein, in formulae (XX), (XXI), and (XXII), p and q are 2, R⁷ and R²¹ represent hydroxyl, and m is 1.

29. The process according to any one of claims 26 to 28, wherein the compounds represented by formulae (XX), (XXI), and (XXII) are respectively compounds represented by formulae (XX'), (XXI'), and (XXII') 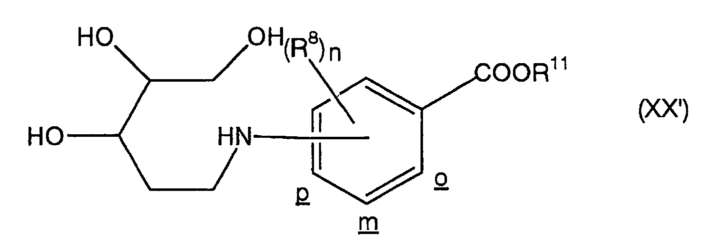 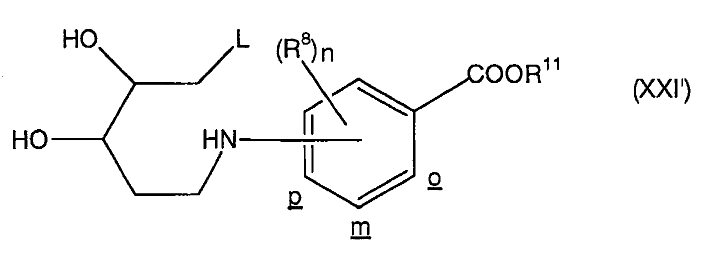 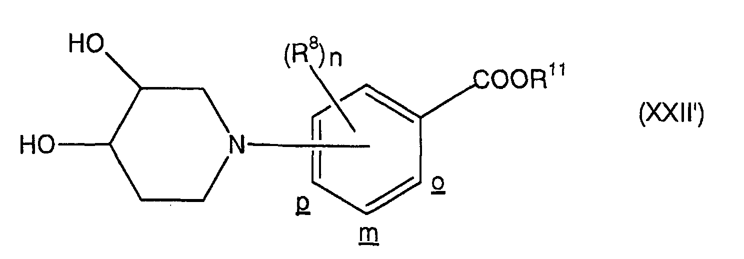 wherein R⁸, R¹¹, and n are as defined in claim 1; and the nitrogen atom is attached to the ortho-, meta-, or para-position of the phenyl group.

30. A compound represented by formula (XXIII): 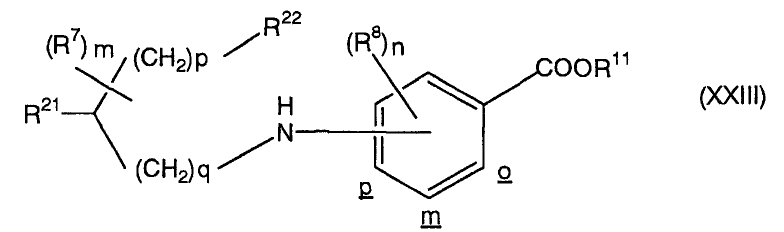 wherein R⁷, R⁸, R¹¹, m, n, p, and q are as defined in claim 1, provided that q is not 0 (zero); R²¹ is as defined in claim 22; R²² represents hydroxyl or a leaving group; and the nitrogen atom is attached to the ortho-, meta-, or para-position of the phenyl group.

31. The compound according to claim 30, wherein p and q are 2, R⁷ and R²¹ represent hydroxyl, and m is 1.

32. A compound represented by formula (XXII): 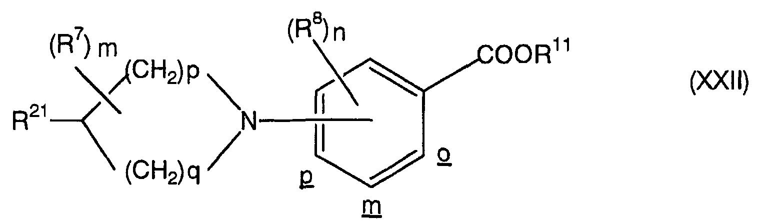 wherein R⁷, R⁸, R¹¹, m, n, p, and q are as defined in claim 1, provided that q is not 0 (zero); R²¹ is as defined in claim 22; and the nitrogen atom is attached to the ortho-, meta-, or para-position of the phenyl group.

33. The compound according to claim 32, wherein p and q are 2, R⁷ and R²¹ represent hydroxyl, and m is 1.
